(19) Europäisches Patentamt
European Patent Office
Office européen des brevets

(11) EP 4 745 130 A1

(12) **EUROPEAN PATENT APPLICATION**
published in accordance with Art. 153(4) EPC

(43) Date of publication:
**20.05.2026 Bulletin 2026/21**

(21) Application number: **24842309.7**

(22) Date of filing: **12.07.2024**

(51) International Patent Classification (IPC):
***C07D 401/12*** *(2006.01)* ***C07D 413/12*** *(2006.01)*
***A61K 31/4439*** *(2006.01)* ***A61K 31/5377*** *(2006.01)*
***A61K 31/137*** *(2006.01)* ***A61P 35/00*** *(2006.01)*

(52) Cooperative Patent Classification (CPC):
**A61K 31/137; A61K 31/4439; A61K 31/5377; A61P 17/00; A61P 17/06; A61P 35/00; A61P 35/02; C07D 401/12; C07D 413/12; C07D 491/048**

(86) International application number:
**PCT/CN2024/105220**

(87) International publication number:
**WO 2025/016316 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.07.2023 CN 202310874007**

(71) Applicant: **Hubei Bio-Pharmaceutical Industrial Technological Institute Inc.**
**Wuhan, Hubei 430075 (CN)**

(72) Inventors:
- **ZHANG, Xuejun**
  **Wuhan, Hubei 430075 (CN)**
- **ZANG, Yang**
  **Wuhan, Hubei 430075 (CN)**
- **YANG, Hui**
  **Wuhan, Hubei 430075 (CN)**
- **WEI, Wenjun**
  **Wuhan, Hubei 430075 (CN)**
- **SONG, Zejin**
  **Wuhan, Hubei 430075 (CN)**
- **ZHANG, Xin**
  **Wuhan, Hubei 430075 (CN)**

(74) Representative: **Henderson, Helen Lee**
**Withers & Rogers LLP**
**2 London Bridge**
**London SE1 9RA (GB)**

# (54) CRYSTAL FORMS OF TUBULIN-SRC DUAL-TARGETING INHIBITOR, PREPARATION METHOD THEREFOR AND USE THEREOF

(57) The present invention provides a crystal forms of a tubulin-SRC dual-targeting inhibitor, a preparation method therefor and a use thereof. Specifically disclosed are 2 free crystal forms of the compound shown in Formula I and 13 crystal forms of 10 salts, a preparation method therefor and a use thereof. The compound and the salt forms and crystal forms thereof can be used as tubulin and Src kinase dual-targeting inhibitors, or as tubulin inhibotors or Src kinase inhibitors alone. The compound, and the salt forms and crystal forms thereof, can also significantly inhibit the polymerization of tubulin monomers and inhibit cell proliferation, have good pharmacokinetic properties for topical administration to the skin, have good druggability, can be used to prepare skin topical preparations, and have the advantages of fast metabolism and low side effects.

EP 4 745 130 A1

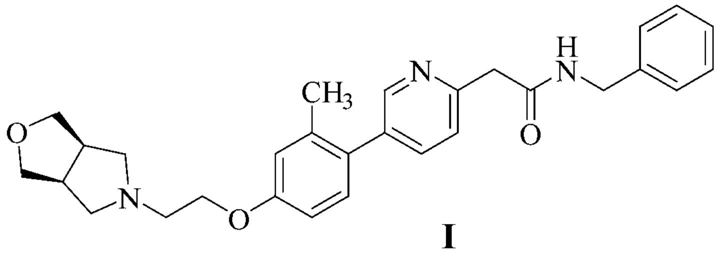

**I**

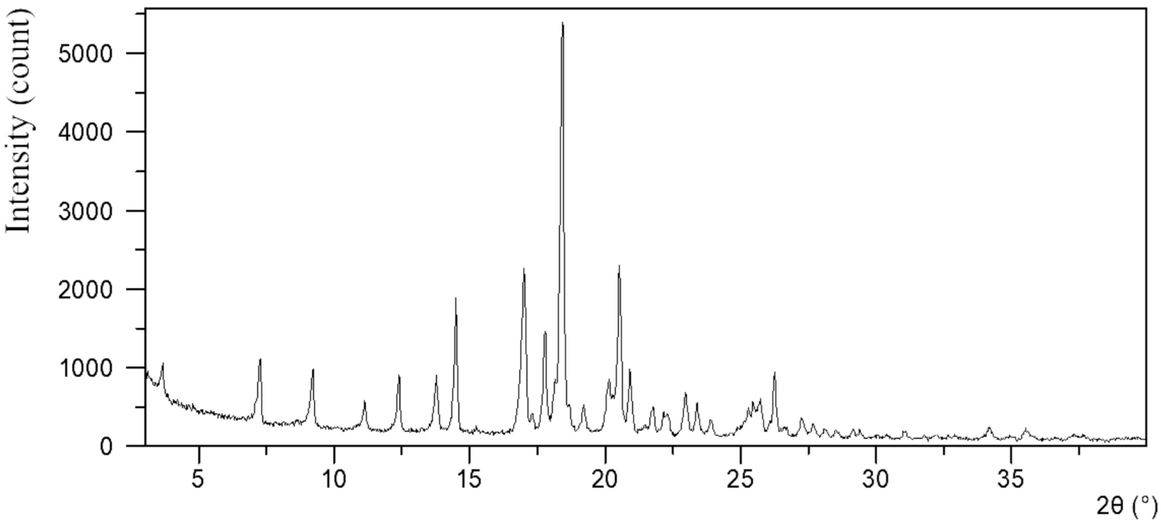
Intensity (count)
5000
4000
3000
2000
1000
0
5
10
15
20
25
30
35
2θ (°)

FIG. 1

## Description

The present application claims the right of the priority of Chinese patent application 2023108740075 filed on July 14, 2023. The content of the above Chinese patent application is incorporated herein by reference in its entirety.

TECHNICAL FIELD

The present disclosure relates to a crystal form of a tubulin-SRC dual-target inhibitor, a preparation method therefor, and a use thereof.

BACKGROUND

Microtubules are an important component of the cytoskeleton in eukaryotic cells and play an important role in a variety of cellular functions such as maintaining cell morphology, signal transmission, organelle transport, cell movement, cell division, and mitosis.

Microtubules are composed of two types of tubulin subunits, namely $\alpha$-tubulin and $\beta$-tubulin. $\alpha$-tubulin and $\beta$-tubulin form tubulin heterodimers, which are the basic units of microtubule assembly. Microtubule-targeting agents (MTAs) can destroy the dynamic stability and structure of microtubules, interfere with the formation of mitotic spindles, induce cell cycle arrest in the G2/M phase, and promote cell apoptosis.

Microtubules are involved in many important cellular processes and have become one of the most important drug targets for the treatment of hyperproliferative diseases. Several microtubule-targeting agents approved by FDA of the United States, such as vinblastine and taxane compounds, are widely used to treat multiple solid tumors and hematological malignancies, but the drug resistance and dose-limiting toxicity of microtubule-targeting drugs limit their clinical efficacy. Compared with single-target drugs, dual-target inhibitors overcome drug resistance, can improve therapeutic effects, and have become a research hotspot, such as: tubulin-SRC dual-target inhibitors, tubulin-receptor tyrosine kinase (RTK) dual-target inhibitors, tubulin-histone deacetylases (HDAC) dual-target inhibitors.

Actinic keratosis (AK) is a skin disease related to long-term exposure to ultraviolet light. AK is the second most common disease among dermatologists in the United States, characterized by uncontrolled proliferation of mutant keratinocytes, which is considered as a precancerous lesion. If not treated in time, 20% of cases may develop into cutaneous squamous cell carcinoma (SCC). At present, Tirbanibulin, a tubulin/SRC dual-target inhibitor, has a remarkable clinical effect on local treatment of AK (NCT03285477), which has been approved for marketing by FDA. This indicates that it may be a promising direction to develop a new tubulin/SRC dual-target inhibitor with better effect for local treatment of AK.

The Chinese invention patent application with application number 202211139978.7 describes compound I-2 (compound of Formula I in the present disclosure) with tubulin-SRC dual-target inhibitory effect:

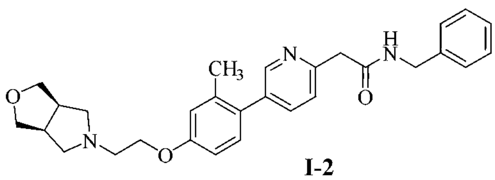

I-2

,

On the basis of the compound exhibiting favorable biological activity, it is necessary to develop a suitable salt form and a solid form thereof to achieve improved druggability or other characteristics.

SUMMARY

The present disclosure aims to provide a crystal form of a tubulin-SRC dual-target inhibitor, a preparation method therefor, and a use thereof. The dual-target inhibitor has the crystal form or salt form as described in the present disclosure, which exhibits advantages such as good stability. The crystal form or salt form can serve as a dual-target inhibitor of tubulin and SRC kinase, or as a tubulin inhibitor or Src kinase inhibitor alone.

The present disclosure provides a free form crystal form A of a compound of Formula I,

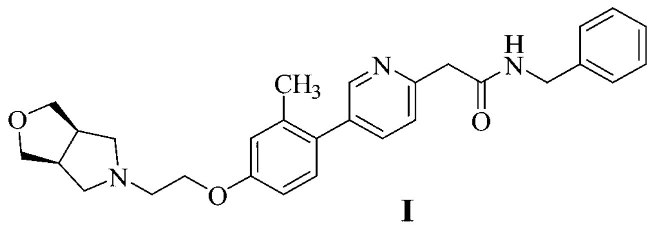

I

wherein the free form crystal form A has an X-ray powder diffraction pattern, measured using Cu-Kα radiation and expressed in terms of 20 angles, comprising diffraction peaks at 14.49±0.2°, 17.02±0.2°, 18.42±0.2°, and 20.53±0.2°.

In one embodiment, the free form crystal form A has an X-ray powder diffraction pattern, measured using Cu-Kα radiation and expressed in terms of 20 angles, further comprising diffraction peaks at one or more of the following positions: 7.25±0.2°, 9.20±0.2°, 12.39±0.2°, and 13.77±0.2°.

In one embodiment, the free form crystal form A has an X-ray powder diffraction pattern, measured using Cu-Kα radiation and expressed in terms of 20 angles, comprising diffraction peaks at 3.66±0.2°, 7.25±0.2°, 9.20±0.2°, 11.13 ±0.2°, 12.39±0.2°, 13.77±0.2°, and 14.49±0.2°.

In one embodiment, the free form crystal form A has an X-ray powder diffraction pattern, measured using Cu-Kα radiation and expressed in terms of 20 angles, comprising diffraction peaks at 7.25±0.2°, 9.20±0.2°, 12.39±0.2°, 13.77 ±0.2°, 14.49±0.2°, 17.02±0.2°, 17.78±0.2°, 18.42±0.2°, and 20.53±0.2°.

In one embodiment, the free form crystal form A has an X-ray powder diffraction pattern, measured using Cu-Kα radiation and expressed in terms of 20 angles, comprising diffraction peaks at 3.66±0.2°, 7.25±0.2°, 9.20±0.2°, 11.13 ±0.2°, 12.39±0.2°, 13.77±0.2°, 14.49±0.2°, 17.02±0.2°, 17.78±0.2°, 18.42±0.2°, and 20.53±0.2°.

In one embodiment, the free form crystal form A has an X-ray powder diffraction pattern, measured using Cu-Kα radiation and expressed in terms of 20 angles, comprising diffraction peaks at 3.66±0.2°, 7.25±0.2°, 9.20±0.2°, 11.13 ±0.2°, 12.39±0.2°, 13.77±0.2°, 14.49±0.2°, 17.02±0.2°, 17.78±0.2°, 18.42±0.2°, 20.53±0.2°, and 20.92±0.2°.

In one embodiment, the free form crystal form A is an anhydrous crystal form.

In one embodiment, the free form crystal form A has an X-ray powder diffraction pattern, measured using Cu-Kα radiation and expressed in terms of 2θ angles, comprising diffraction peak positions and relative intensities substantially as shown in Table 1:

Table 1

| Position [°2θ] | Relative intensity [%] | Position [°2θ] | Relative intensity [%] |
|---|---|---|---|
| 3.6586 | 7.14 | 22.9597 | 10.42 |
| 7.2523 | 15.19 | 23.3865 | 7.72 |
| 9.2043 | 13.87 | 23.9035 | 3.75 |
| 11.1262 | 7.27 | 25.7372 | 8.71 |
| 12.3913 | 13.21 | 26.2579 | 15.26 |
| 13.7658 | 13.84 | 26.6575 | 2.24 |
| 14.4926 | 31.62 | 27.2477 | 4.46 |
| 17.0216 | 38.40 | 27.6853 | 3.21 |
| 17.7818 | 24.47 | 28.1122 | 2.01 |
| 18.4187 | 100.00 | 28.5449 | 1.68 |
| 19.2078 | 6.70 | 29.1385 | 1.69 |
| 20.1286 | 12.32 | 31.0525 | 1.80 |
| 20.5296 | 39.57 | 34.1923 | 2.83 |
| 20.9229 | 15.31 | 35.5215 | 2.32 |
| 21.7756 | 6.40 | 37.2727 | 1.21 |
| 22.2614 | 4.58 | | |

In one embodiment, the free form crystal form A has an X-ray powder diffraction pattern, measured using Cu-Kα

radiation and expressed in terms of 2θ angles, substantially as shown in FIG. 1.

In one embodiment, the free form crystal form A has a differential scanning calorimetry curve showing an onset of an endothermic peak at 94.7±3°C.

In one embodiment, the free form crystal form A has a differential scanning calorimetry curve showing an endothermic peak at 96.6±3°C.

In one embodiment, the free form crystal form A has a thermogravimetric analysis curve showing a weight loss of approximately 0.98% at 150°C.

In one embodiment, the free form crystal form A has a differential scanning calorimetry curve substantially as shown in FIG. 3.

In one embodiment, the free form crystal form A has a thermogravimetric analysis curve substantially as shown in FIG. 2.

The present disclosure further provides a free form crystal form B of a compound of Formula I,

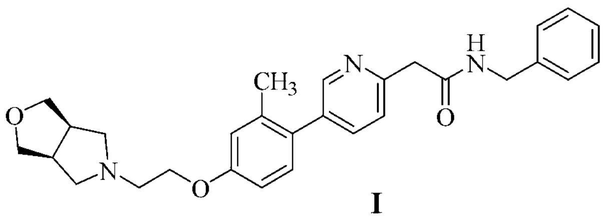

I

wherein the free form crystal form B has an X-ray powder diffraction pattern, measured using Cu-Kα radiation and expressed in terms of 2θ angles, comprising diffraction peaks at 16.55±0.2°, 17.77±0.2°, 20.36±0.2°, 21.24±0.2°, and 22.66±0.2°.

In one embodiment, the free form crystal form B has an X-ray powder diffraction pattern, measured using Cu-Kα radiation and expressed in terms of 2θ angles, further comprising diffraction peaks at one or more of the following positions: 8.89±0.2°, 10.99±0.2°, 13.81±0.2°, 16.10±0.2°, and 19.65±0.2°.

In one embodiment, the free form crystal form B has an X-ray powder diffraction pattern, measured using Cu-Kα radiation and expressed in terms of 2θ angles, comprising diffraction peaks at 8.89±0.2°, 10.99±0.2°, 13.81±0.2°, 16.10 ±0.2°, 16.55±0.2°, 17.77±0.2°, 19.65±0.2°, 20.36±0.2°, 21.24±0.2°, and 22.66±0.2°.

In one embodiment, the free form crystal form B has an X-ray powder diffraction pattern, measured using Cu-Kα radiation and expressed in terms of 2θ angles, comprising diffraction peaks at 8.89±0.2°, 10.99±0.2°, 13.81±0.2°, 14.97 ±0.2°, 16.10±0.2°, 16.55±0.2°, 17.77±0.2°, 19.65±0.2°, 20.36±0.2°, 21.24±0.2°, 22.66±0.2°, 23.92±0.2°, 24.80 ±0.2°, 25.41±0.2°, and 26.65±0.2°.

In one embodiment, the free form crystal form B is an anhydrous crystal form.

In one embodiment, the free form crystal form B has an X-ray powder diffraction pattern, measured using Cu-Kα radiation and expressed in terms of 2θ angles, comprising diffraction peak positions and relative intensities substantially as shown in Table 2:

Table 2

| Position [°2θ] | Relative intensity [%] | Position [°2θ] | Relative intensity [%] |
|---|---|---|---|
| 7.4873 | 3.10 | 22.6576 | 61.87 |
| 8.8864 | 9.12 | 23.9201 | 7.02 |
| 10.9912 | 18.04 | 24.7970 | 12.05 |
| 13.8121 | 14.37 | 25.4143 | 15.92 |
| 14.9693 | 6.34 | 26.6488 | 16.69 |
| 16.1011 | 36.37 | 27.7637 | 6.02 |
| 16.5486 | 39.23 | 28.3501 | 3.63 |
| 16.9933 | 5.23 | 29.5259 | 2.44 |
| 17.7740 | 100.00 | 30.7717 | 5.46 |
| 18.6972 | 5.30 | 31.9792 | 2.29 |
| 19.6520 | 31.67 | 33.3167 | 3.42 |
| 20.3614 | 65.65 | 37.7157 | 3.02 |

(continued)

| Position [°2θ] | Relative intensity [%] | Position [°2θ] | Relative intensity [%] |
|---|---|---|---|
| 21.2371 | 58.26 | 39.0657 | 1.38 |
| 22.0673 | 19.39 | | |

In one embodiment, the free form crystal form B has an X-ray powder diffraction pattern, measured using Cu-Kα radiation and expressed in terms of 2θ angles, substantially as shown in FIG. 4.

In one embodiment, the free form crystal form B has a differential scanning calorimetry curve showing an onset of an endothermic peak at 77.2±3°C.

In one embodiment, the free form crystal form B has a differential scanning calorimetry curve showing an endothermic peak at 84.5±3°C.

In one embodiment, the free form crystal form B has a differential scanning calorimetry curve showing an endothermic peak at 92.4±3°C.

In one embodiment, the free form crystal form B has a thermogravimetric analysis curve showing a weight loss of approximately 0.48% at 150°C.

In one embodiment, the free form crystal form B has a differential scanning calorimetry curve substantially as shown in FIG. 6.

In one embodiment, the free form crystal form B has a thermogravimetric analysis curve substantially as shown in FIG. 5.

The present disclosure further provides a preparation method for the free form crystal form A of the compound of Formula I: dissolving the compound of Formula I in a solvent and evaporating at room temperature to obtain the free form crystal form A; the solvent is an organic solvent or a mixed solvent, wherein the organic solvent includes, but is not limited to: $CHCl_3$, THF, acetone, toluene, cyclopentyl methyl ether, 2-methyltetrahydrofuran, isopropanol, IPAc, and 2-butanone; the mixed solvent is a mixed solvent of an organic solvent and water, and the mixed solvent includes, but is not limited to: a mixed solvent of MeOH and $H_2O$, and a mixed solvent of acetonitrile and $H_2O$.

The present disclosure further provides a preparation method for the free form crystal form B of the compound of Formula I: concentrating a solution of the compound of Formula I under reduced pressure and drying at 40-60°C to obtain the free form crystal form B; the solution is an alcohol solution or a halogenated hydrocarbon solution.

Preferably, the compound of Formula I is dissolved in MeOH or dichloromethane, followed by rotary evaporation under reduced pressure, and the resulting gel-like sample is transferred for vacuum drying at 50°C to obtain the free form crystal form B.

The present disclosure further provides a phosphate salt of the compound of Formula I.

In one embodiment, the phosphate salt of the compound of Formula I is crystal form A of the phosphate salt of the compound of Formula I, wherein the crystal form A of the phosphate salt has an X-ray powder diffraction pattern, measured using Cu-Kα radiation and expressed in terms of 2θ angles, comprising diffraction peaks at 6.74±0.2°, 13.47±0.2°, 15.69 ±0.2°, 16.86±0.2°, and 18.09±0.2°.

In one embodiment, the crystal form A of the phosphate salt has an X-ray powder diffraction pattern, measured using Cu-Kα radiation and expressed in terms of 2θ angles, comprising diffraction peaks at 6.74±0.2°, 13.47±0.2°, 15.69 ±0.2°, 18.09±0.2°, and 20.26±0.2°.

In one embodiment, the crystal form A of the phosphate salt has an X-ray powder diffraction pattern, measured using Cu-Kα radiation and expressed in terms of 2θ angles, further comprising diffraction peaks at one or more of the following positions: 11.87±0.2°, 12.56±0.2°, 13.75±0.2°, 18.60±0.2°, 20.26±0.2°, and 25.26±0.2°.

In one embodiment, the crystal form A of the phosphate salt has an X-ray powder diffraction pattern, measured using Cu-Kα radiation and expressed in terms of 2θ angles, comprising diffraction peaks at 6.74±0.2°, 11.87±0.2°, 12.56 ±0.2°, 13.47±0.2°, 13.75±0.2°, 15.69±0.2°, 16.86±0.2°, 18.09±0.2°, and 20.26±0.2°.

In one embodiment, the crystal form A of the phosphate salt has an X-ray powder diffraction pattern, measured using Cu-Kα radiation and expressed in terms of 2θ angles, comprising diffraction peaks at 3.40±0.2°, 6.74±0.2°, 7.47 ±0.2°, 11.87±0.2°, 12.56±0.2°, 13.47±0.2°, 13.75±0.2°, 14.94±0.2°, 15.69±0.2°, 16.86±0.2°, 18.09±0.2°, 18.61 ±0.2°, 20.26±0.2°, and 25.26±0.2°.

In one embodiment, the crystal form A of the phosphate salt has an X-ray powder diffraction pattern, measured using Cu-Kα radiation and expressed in terms of 2θ angles, comprising diffraction peak positions and relative intensities substantially as shown in Table 3:

Table 3

| Position [°2θ] | Relative intensity [%] | Position [°2θ] | Relative intensity [%] |
|---|---|---|---|
| 3.3971 | 3.92 | 20.7346 | 11.38 |
| 6.7420 | 64.07 | 21.0112 | 17.83 |
| 7.4735 | 11.63 | 22.1650 | 19.60 |
| 7.8430 | 6.09 | 22.4611 | 18.76 |
| 11.8665 | 6.89 | 22.6861 | 21.05 |
| 12.5613 | 11.06 | 23.3038 | 14.49 |
| 13.4735 | 48.13 | 23.6997 | 10.47 |
| 13.7472 | 23.38 | 24.2381 | 14.86 |
| 14.9424 | 15.60 | 24.6170 | 6.42 |
| 15.6909 | 48.98 | 25.2605 | 18.00 |
| 16.5665 | 19.30 | 26.0498 | 4.76 |
| 16.8571 | 100.00 | 27.1236 | 3.12 |
| 17.2241 | 19.19 | 27.6743 | 7.67 |
| 18.0887 | 33.50 | 28.2245 | 2.87 |
| 18.6053 | 18.77 | 29.6701 | 5.48 |
| 19.2940 | 2.78 | 30.6420 | 4.94 |
| 20.2570 | 23.97 | | |

In one embodiment, the crystal form A of the phosphate salt has an X-ray powder diffraction pattern, measured using Cu-Kα radiation and expressed in terms of 2θ angles, substantially as shown in FIG. 7.

In one embodiment, in the crystal form A of the phosphate salt, the molar ratio of the compound of Formula I to phosphoric acid is 1:1.

In one embodiment, the crystal form A of the phosphate salt has a differential scanning calorimetry curve showing an onset of an endothermic peak at 172.8±3°C.

In one embodiment, the crystal form A of the phosphate salt has a differential scanning calorimetry curve showing an endothermic peak at 174.5±3°C.

In one embodiment, the crystal form A of the phosphate salt has a thermogravimetric analysis curve showing a weight loss of approximately 4.40% at 150°C.

In one embodiment, the crystal form A of the phosphate salt has a differential scanning calorimetry curve substantially as shown in FIG. 9.

In one embodiment, the crystal form A of the phosphate salt has a thermogravimetric analysis curve substantially as shown in FIG. 8.

The present disclosure further provides a preparation method for the crystal form A of the phosphate salt of the compound of Formula I: mixing the compound of Formula I, phosphoric acid, and a solvent, and slurrying at room temperature to obtain the crystal form A of the phosphate salt of the compound of Formula I;

the solvent is an ester solvent, an ether solvent, a mixed solvent of an alcohol solvent and water, or a mixed solvent of a ketone solvent and a saturated alkane solvent.

In one embodiment, the molar ratio of the phosphoric acid to the compound of Formula I is 1.0-1.7; preferably 1.2.

In one embodiment, the molar ratio of the phosphoric acid to the compound of Formula I is (1.0-1.7):1; preferably 1.2:1.

In one embodiment, the molar volume ratio of the compound of Formula I to the solvent is 0.11-0.21 mol/L; preferably 0.16 mol/L.

In one embodiment, in the mixed solvent of the alcohol solvent and water, the volume ratio of the alcohol solvent to water is (14-24):1, preferably 19:1.

In one embodiment, in the mixed solvent of the ketone solvent and the saturated alkane solvent, the volume ratio of the ketone solvent to the saturated alkane solvent is (0.5-2):1.

In one embodiment, the solvent is a mixed solvent of IPA/$H_2O$ (19:1, v/v), a mixed solvent of acetone/n-heptane (1:1, v/v), isopropyl acetate, or methyl *tert*-butyl ether.

In one embodiment, the phosphate salt of the compound of Formula I is crystal form B of the phosphate salt of the compound of Formula I, wherein the crystal form B of the phosphate salt has an X-ray powder diffraction pattern, measured using Cu-Kα radiation and expressed in terms of 20 angles, comprising diffraction peaks at 6.68± 0.2°, 13.37±0.2°, 14.16 ±0.2°, 15.30±0.2°, 16.74±0.2°, and 20.12±0.2°.

In one embodiment, the crystal form B of the phosphate salt is an anhydrous crystal form.

In one embodiment, in the crystal form B of the phosphate salt, the molar ratio of the compound of Formula I to phosphoric acid is 1:1.

In one embodiment, the crystal form B of the phosphate salt has an X-ray powder diffraction pattern, measured using Cu-Kα radiation and expressed in terms of 20 angles, further comprising diffraction peaks at one or more of the following positions: 3.35±0.2°, 14.53±0.2°, 21.26±0.2°, 22.53±0.2°, 24.42±0.2°, and 25.24±0.2°.

In one embodiment, the crystal form B of the phosphate salt has an X-ray powder diffraction pattern, measured using Cu-Kα radiation and expressed in terms of 20 angles, further comprising diffraction peaks at one or more of the following positions: 3.35±0.2°, 14.53±0.2°, 21.27±0.2°, 22.53±0.2°, 24.43±0.2°, and 25.24±0.2°.

In one embodiment, the crystal form B of the phosphate salt has an X-ray powder diffraction pattern, measured using Cu-Kα radiation and expressed in terms of 2θ angles, comprising diffraction peaks at 6.68±0.2°, 13.37±0.2°, 14.16 ±0.2°, 14.53±0.2°, 15.30±0.2°, 16.74±0.2°, 20.12±0.2°, and 21.26±0.2°.

In one embodiment, the crystal form B of the phosphate salt has an X-ray powder diffraction pattern, measured using Cu-Kα radiation and expressed in terms of 20 angles, comprising diffraction peaks at 3.35±0.2°, 6.68±0.2°, 13.37 ±0.2°, 14.16±0.2°, 14.53±0.2°, 15.30±0.2°, 16.74±0.2°, 20.12±0.2°, 21.26±0.2°, 22.53±0.2°, 24.42±0.2°, and 25.24 ±0.2°.

In one embodiment, the crystal form B of the phosphate salt has an X-ray powder diffraction pattern, measured using Cu-Kα radiation and expressed in terms of 20 angles, comprising diffraction peak positions and relative intensities substantially as shown in Table 4:

Table 4

| Position [°2θ] | Relative intensity [%] | Position [°2θ] | Relative intensity [%] |
|---|---|---|---|
| 3.3488 | 100.00 | 21.4946 | 10.39 |
| 6.6843 | 21.93 | 21.7492 | 10.41 |
| 7.6346 | 4.49 | 22.5344 | 11.77 |
| 10.0305 | 3.43 | 23.0287 | 5.88 |
| 12.1675 | 7.90 | 23.2345 | 5.21 |
| 13.3734 | 45.72 | 23.9394 | 3.17 |
| 13.7627 | 11.36 | 24.4279 | 12.17 |
| 14.1560 | 9.48 | 25.2438 | 15.76 |
| 14.5261 | 7.21 | 25.6966 | 5.56 |
| 15.0409 | 11.28 | 27.0403 | 1.05 |
| 15.3041 | 34.26 | 27.8899 | 2.69 |
| 16.7414 | 68.50 | 28.7762 | 1.94 |
| 17.2869 | 7.43 | 29.6849 | 3.25 |
| 17.4813 | 8.70 | 32.1272 | 1.17 |
| 17.7801 | 12.25 | 32.7569 | 1.84 |
| 18.1056 | 17.31 | 33.5155 | 1.11 |
| 18.8004 | 4.56 | 34.5572 | 1.79 |
| 19.6890 | 4.68 | 35.9222 | 0.83 |
| 20.1204 | 23.28 | 36.8640 | 1.30 |
| 21.2684 | 20.03 | | |

In one embodiment, the crystal form B of the phosphate salt has an X-ray powder diffraction pattern, measured using Cu-Kα radiation and expressed in terms of 20 angles, substantially as shown in FIG. 11.

The present disclosure further provides a preparation method for the crystal form B of the phosphate salt of the compound of Formula I: heating the crystal form A of the phosphate salt to 150±10°C to obtain the crystal form B of the phosphate salt.

In one embodiment, the crystal form A of the phosphate salt is heated under a nitrogen atmosphere.

In one embodiment, the temperature for the heating is 145-155°C, preferably 150°C.

The present disclosure further provides a fumarate salt of the compound of Formula I.

In one embodiment, the fumarate salt of the compound of Formula I is crystal form A of the fumarate salt of the compound of Formula I, wherein the crystal form A of the fumarate salt has an X-ray powder diffraction pattern, measured using Cu-Kα radiation and expressed in terms of 20 angles, comprising diffraction peaks at 13.51±0.2°, 15.41±0.2°, 17.57±0.2°, 19.48±0.2°, and 20.11±0.2°.

In one embodiment, the crystal form A of the fumarate salt has an X-ray powder diffraction pattern, measured using Cu-Kα radiation and expressed in terms of 20 angles, further comprising diffraction peaks at one or more of the following positions: 9.03±0.2°, 9.78±0.2°, 16.89±0.2°, 22.04±0.2°, 23.71±0.2°, 24.24±0.2°, and 25.72±0.2°.

In one embodiment, the crystal form A of the fumarate salt has an X-ray powder diffraction pattern, measured using Cu-Kα radiation and expressed in terms of 20 angles, comprising diffraction peaks at 9.03±0.2°, 9.78±0.2°, 13.51 ±0.2°, 15.41±0.2°, 16.89±0.2°, 17.57±0.2°, 19.48±0.2°, 20.11±0.2°, and 22.04±0.2°.

In one embodiment, the crystal form A of the fumarate salt has an X-ray powder diffraction pattern, measured using Cu-Kα radiation and expressed in terms of 2θ angles, comprising diffraction peaks at 9.03±0.2°, 9.78±0.2°, 13.51 ±0.2°, 15.41±0.2°, 16.89±0.2°, 17.57±0.2°, 19.48±0.2°, 20.11±0.2°, 22.04±0.2°, 23.71±0.2°, 24.24±0.2°, and 25.72 ±0.2°.

In one embodiment, the crystal form A of the fumarate salt is an anhydrous crystal form.

In one embodiment, in the crystal form A of the fumarate salt, the molar ratio of the compound of Formula I to fumaric acid is 1:1.

In one embodiment, the crystal form A of the fumarate salt has an X-ray powder diffraction pattern, measured using Cu-Kα radiation and expressed in terms of 20 angles, comprising diffraction peak positions and relative intensities substantially as shown in Table 5:

Table 5

| Position [°2θ] | Relative intensity [%] | Position [°2θ] | Relative intensity [%] |
|---|---|---|---|
| 9.0262 | 10.80 | 24.2390 | 26.28 |
| 9.7784 | 29.03 | 25.7165 | 23.45 |
| 13.5143 | 53.68 | 27.1984 | 7.74 |
| 15.4104 | 56.59 | 28.5027 | 2.01 |
| 16.8922 | 29.24 | 29.5519 | 5.77 |
| 17.5651 | 99.14 | 30.2545 | 6.02 |
| 18.3356 | 27.28 | 30.6842 | 8.40 |
| 18.6617 | 21.63 | 31.3364 | 4.48 |
| 19.4757 | 100.00 | 32.3634 | 2.45 |
| 20.1090 | 52.33 | 33.8625 | 1.35 |
| 21.1154 | 12.41 | 35.4107 | 3.44 |
| 22.0379 | 34.40 | 36.5349 | 3.18 |
| 23.0333 | 7.97 | 38.9309 | 1.92 |
| 23.7137 | 46.33 | | |

In one embodiment, the crystal form A of the fumarate salt has an X-ray powder diffraction pattern, measured using Cu-Kα radiation and expressed in terms of 20 angles, substantially as shown in FIG. 12.

In one embodiment, the crystal form A of the fumarate salt has a differential scanning calorimetry curve showing an onset of an endothermic peak at 128.2±3°C.

In one embodiment, the crystal form A of the fumarate salt has a differential scanning calorimetry curve showing an endothermic peak at 130.3±3°C.

In one embodiment, the crystal form A of the fumarate salt has a thermogravimetric analysis curve showing a

weight loss of approximately 1.17% at 150°C.

In one embodiment, the crystal form A of the fumarate salt has a differential scanning calorimetry curve substantially as shown in FIG. 14.

In one embodiment, the crystal form A of the fumarate salt has a thermogravimetric analysis curve substantially as shown in FIG. 13.

The present disclosure further provides a preparation method for the crystal form A of the fumarate salt of the compound of Formula I: mixing the compound of Formula I, fumaric acid, and a solvent, and slurrying at room temperature to obtain the crystal form A of the fumarate salt; the solvent is an ester solvent, an ether solvent, a mixed solvent of an alcohol solvent and water, or a mixed solvent of a ketone solvent and a saturated alkane solvent.

In one embodiment, the molar ratio of the fumaric acid to the compound of Formula I is 1.0-1.5; preferably 1.0.

In one embodiment, the molar ratio of the fumaric acid to the compound of Formula I is (1.0-1.5):1; preferably 1.0:1;

In one embodiment, the molar volume ratio of the compound of Formula I to the solvent is 0.06-0.16 mol/L; preferably 0.11 mol/L.

In one embodiment, in the mixed solvent of the alcohol solvent and water, the volume ratio of the alcohol solvent to water is (14-24):1, preferably 19:1.

In one embodiment, in the mixed solvent of the ketone solvent and the saturated alkane solvent, the volume ratio of the ketone solvent to the saturated alkane solvent is (0.5-2):1.

In one embodiment, the solvent is a mixed solvent of IPA/$H_2O$ (19:1, v/v), a mixed solvent of acetone/*n*-heptane (1:1, v/v), isopropyl acetate, or methyl *tert*-butyl ether.

The present disclosure further provides a *p*-toluenesulfonate salt of the compound of Formula I.

In one embodiment, the *p*-toluenesulfonate salt of the compound of Formula I is crystal form A of the *p*-toluenesulfonate salt of the compound of Formula I, wherein the crystal form A of the *p*-toluenesulfonate salt has an X-ray powder diffraction pattern, measured using Cu-Kα radiation and expressed in terms of 2θ angles, comprising diffraction peaks at 5.41± 0.2°, 8.10±0.2°, 14.74±0.2°, 17.13±0.2°, 18.10±0.2°, and 19.98±0.2°.

In one embodiment, the crystal form A of the *p*-toluenesulfonate salt has an X-ray powder diffraction pattern, measured using Cu-Kα radiation and expressed in terms of 2θ angles, further comprising diffraction peaks at one or more of the following positions: 10.81±0.2°, 18.83±0.2°, 21.83±0.2°, and 24.37±0.2°.

In one embodiment, the crystal form A of the p-toluenesulfonate salt has an X-ray powder diffraction pattern, measured using Cu-Kα radiation and expressed in terms of 2θ angles, comprising diffraction peaks at 5.41±0.2°, 7.74 ±0.2°, 8.10±0.2°, 10.81±0.2°, 14.74±0.2°, 17.13±0.2°, 18.10±0.2°, 18.83±0.2°, and 19.98±0.2°.

In one embodiment, the crystal form A of the p-toluenesulfonate salt has an X-ray powder diffraction pattern, measured using Cu-Kα radiation and expressed in terms of 2θ angles, comprising diffraction peaks at 5.41±0.2°, 7.74 ±0.2°, 8.10±0.2°, 10.81±0.2°, 14.74±0.2°, 17.13±0.2°, 18.10±0.2°, 18.83±0.2°, 19.98±0.2°, 21.01±0.2°, 21.83 ±0.2°, 22.85±0.2°, 23.41±0.2°, 24.37±0.2°, 26.18±0.2°, and 28.58±0.2°.

In one embodiment, in the crystal form A of the *p*-toluenesulfonate salt, the molar ratio of the compound of Formula I to *p*-toluenesulfonic acid is 1:1.

In one embodiment, the crystal form A of the *p*-toluenesulfonate salt has an X-ray powder diffraction pattern, measured using Cu-Kα radiation and expressed in terms of 2θ angles, comprising diffraction peak positions and relative intensities substantially as shown in Table 6:

Table 6

| Position [°2θ] | Relative intensity [%] | Position [°2θ] | Relative intensity [%] |
|---|---|---|---|
| 5.4061 | 50.32 | 19.9778 | 61.40 |
| 7.7375 | 20.44 | 21.0061 | 12.71 |
| 8.1037 | 40.15 | 21.8329 | 62.62 |
| 10.8067 | 22.25 | 22.8534 | 30.96 |
| 13.1118 | 2.94 | 23.4116 | 26.46 |
| 14.7415 | 61.45 | 24.3701 | 39.55 |
| 15.2264 | 7.77 | 26.1763 | 17.86 |
| 16.2076 | 5.59 | 27.2507 | 6.34 |
| 17.1253 | 54.33 | 28.5791 | 19.45 |
| 18.1038 | 100.00 | 30.4321 | 3.98 |

(continued)

| Position [°2θ] | Relative intensity [%] | Position [°2θ] | Relative intensity [%] |
|---|---|---|---|
| 18.3589 | 38.45 | 31.2458 | 6.35 |
| 18.8288 | 80.63 | | |

In one embodiment, the crystal form A of the *p*-toluenesulfonate salt has an X-ray powder diffraction pattern, measured using Cu-Kα radiation and expressed in terms of 2θ angles, substantially as shown in FIG. 16.

In one embodiment, the crystal form A of the *p*-toluenesulfonate salt has a differential scanning calorimetry curve showing an onset of an endothermic peak at 149.9±3°C.

In one embodiment, the crystal form A of the *p*-toluenesulfonate salt has a differential scanning calorimetry curve showing an endothermic peak at 152.1±3°C.

In one embodiment, the crystal form A of the *p*-toluenesulfonate salt has a thermogravimetric analysis curve showing a weight loss of approximately 0.94% at 150°C.

In one embodiment, the crystal form A of the *p*-toluenesulfonate salt has a differential scanning calorimetry curve substantially as shown in FIG. 18.

In one embodiment, the crystal form A of the *p*-toluenesulfonate salt has a thermogravimetric analysis curve substantially as shown in FIG. 17.

The present disclosure further provides a preparation method for the crystal form A of the *p*-toluenesulfonate salt of the compound of Formula I: mixing the compound of Formula I, *p*-toluenesulfonic acid, and a solvent, and slurrying at room temperature to obtain the crystal form A of the *p*-toluenesulfonate salt;
the solvent is an ester solvent or an ether solvent.

In one embodiment, the molar ratio of the p-toluenesulfonic acid to the compound of Formula I is 1.0-1.5; preferably 1.0.

In one embodiment, the molar ratio of the p-toluenesulfonic acid to the compound of Formula I is (1.0-1.5):1; preferably 1.0:1.

In one embodiment, the molar volume ratio of the compound of Formula I to the solvent is 0.06-0.16 mol/L; preferably 0.11 mol/L.

In one embodiment, the solvent is isopropyl acetate or methyl *tert*-butyl ether.

The present disclosure further provides a benzenesulfonate salt of the compound of Formula I.

In one embodiment, the benzenesulfonate salt of the compound of Formula I is crystal form A of the benzenesulfonate salt of the compound of Formula I, wherein the crystal form A of the benzenesulfonate salt has an X-ray powder diffraction pattern, measured using Cu-Kα radiation and expressed in terms of 2θ angles, comprising diffraction peaks at 4.29±0.2°, 8.49±0.2°, 10.02±0.2°, 13.81±0.2°, and 15.26±0.2°.

In one embodiment, the crystal form A of the benzenesulfonate salt has an X-ray powder diffraction pattern, measured using Cu-Kα radiation and expressed in terms of 2θ angles, further comprising diffraction peaks at one or more of the following positions: 10.68±0.2°, 16.80±0.2°, 18.80±0.2°, 19.73±0.2°, 20.53±0.2°, and 20.99±0.2°.

In one embodiment, the crystal form A of the benzenesulfonate salt has an X-ray powder diffraction pattern, measured using Cu-Kα radiation and expressed in terms of 2θ angles, comprising diffraction peaks at 4.29±0.2°, 8.49 ±0.2°, 10.02±0.2°, 10.68±0.2°, 13.81±0.2°, 15.26±0.2°, 16.80±0.2°, 18.80±0.2°, 19.73±0.2°, 20.53±0.2°, and 20.99 ±0.2°.

In one embodiment, the crystal form A of the benzenesulfonate salt has an X-ray powder diffraction pattern, measured using Cu-Kα radiation and expressed in terms of 2θ angles, comprising diffraction peaks at 4.29±0.2°, 5.75 ±0.2°, 8.49±0.2°, 10.02±0.2°, 10.68±0.2°, 11.18±0.2°, 11.48±0.2°, 12.70±0.2°, 13.81±0.2°, 15.26±0.2°, 16.80 ±0.2°, 18.80±0.2°, 19.73±0.2°, 20.53±0.2°, 20.99±0.2°, 21.95±0.2°, and 23.90±0.2°.

In one embodiment, in the crystal form A of the benzenesulfonate salt, the molar ratio of the compound of Formula I to benzenesulfonic acid is 1:1.

In one embodiment, the crystal form A of the benzenesulfonate salt has an X-ray powder diffraction pattern, measured using Cu-Kα radiation and expressed in terms of 2θ angles, comprising diffraction peak positions and relative intensities substantially as shown in Table 7:

Table 7

| Position [°2θ] | Relative intensity [%] | Position [°2θ] | Relative intensity [%] |
|---|---|---|---|
| 4.2927 | 90.89 | 20.2214 | 43.49 |
| 5.7545 | 6.24 | 20.5266 | 100.00 |

(continued)

| Position [°2θ] | Relative intensity [%] | Position [°2θ] | Relative intensity [%] |
|---|---|---|---|
| 8.4936 | 76.94 | 20.9947 | 73.78 |
| 10.0153 | 39.96 | 21.2345 | 28.85 |
| 10.6755 | 22.03 | 21.4984 | 24.65 |
| 11.1754 | 8.55 | 21.9486 | 45.89 |
| 11.4806 | 9.68 | 22.3819 | 23.59 |
| 12.7029 | 7.82 | 22.6234 | 14.90 |
| 13.8134 | 41.83 | 23.4571 | 10.81 |
| 14.4981 | 11.11 | 23.8950 | 42.58 |
| 15.2579 | 54.96 | 25.5219 | 30.50 |
| 16.8048 | 46.57 | 25.7195 | 28.44 |
| 16.9835 | 26.02 | 27.6008 | 3.86 |
| 17.8361 | 20.18 | 28.7734 | 9.90 |
| 18.4629 | 22.00 | 29.2011 | 12.35 |
| 18.7971 | 56.58 | 30.3950 | 5.38 |
| 19.1714 | 26.12 | 31.7722 | 4.14 |
| 19.7264 | 74.71 | 33.1659 | 6.58 |

In one embodiment, the crystal form A of the benzenesulfonate salt has an X-ray powder diffraction pattern, measured using Cu-Kα radiation and expressed in terms of 2θ angles, substantially as shown in FIG. 19.

In one embodiment, the crystal form A of the benzenesulfonate salt has a differential scanning calorimetry curve showing an onset of an endothermic peak at 144.5±3°C.

In one embodiment, the crystal form A of the benzenesulfonate salt has a differential scanning calorimetry curve showing an endothermic peak at 146.4±3°C.

In one embodiment, the crystal form A of the benzenesulfonate salt has a thermogravimetric analysis curve showing a weight loss of approximately 1.28% at 140°C.

In one embodiment, the crystal form A of the benzenesulfonate salt has a differential scanning calorimetry curve substantially as shown in FIG. 21.

In one embodiment, the crystal form A of the benzenesulfonate salt has a thermogravimetric analysis curve substantially as shown in FIG. 20.

The present disclosure further provides a preparation method for the crystal form A of the benzenesulfonate salt of the compound of Formula I: mixing the compound of Formula I, benzenesulfonic acid, and a solvent, and slurrying at room temperature to obtain the crystal form A of the benzenesulfonate salt;
the solvent is an ester solvent, or a mixed solvent of an alcohol solvent and water.

In one embodiment, the molar ratio of the benzenesulfonic acid to the compound of Formula I is 1.0-1.5; preferably 1.0.

In one embodiment, the molar ratio of the benzenesulfonic acid to the compound of Formula I is (1.0-1.5); preferably 1.0:1.

In one embodiment, the molar volume ratio of the compound of Formula I to the solvent is 0.06-0.16 mol/L; preferably 0.11 mol/L.

In one embodiment, in the mixed solvent of the alcohol solvent and water, the volume ratio of the alcohol solvent to water is (14-24):1, preferably 19:1.

In one embodiment, the solvent is a mixed solvent of IPA/$H_2O$ (19:1, v/v) or isopropyl acetate.

The present disclosure further provides an oxalate salt of the compound of Formula I.

In one embodiment, the oxalate salt of the compound of Formula I is crystal form A of the oxalate salt of the compound of Formula I, wherein the crystal form A of the oxalate salt comprises the compound of Formula I and oxalic acid; wherein the crystal form A of the oxalate salt has an X-ray powder diffraction pattern, measured using Cu-Kα radiation and expressed in terms of 2θ angles, comprising diffraction peaks at 7.32±0.2°, 8.47±0.2°, 14.62±0.2°, 17.00±0.2°, 18.58 ±0.2°, and 19.44±0.2°.

In one embodiment, the crystal form A of the oxalate salt has an X-ray powder diffraction pattern, measured using Cu-Kα radiation and expressed in terms of 20 angles, further comprising diffraction peaks at one or more of the following positions: 12.93±0.2°, 13.47±0.2°, 15.23±0.2°, 22.38±0.2°, and 26.00±0.2°.

In one embodiment, the crystal form A of the oxalate salt has an X-ray powder diffraction pattern, measured using Cu-Kα radiation and expressed in terms of 20 angles, comprising diffraction peaks at 7.32±0.2°, 8.47±0.2°, 12.93±0.2°, 13.47±0.2°, 14.62±0.2°, 15.23±0.2°, 17.00±0.2°, 18.58±0.2°, 19.44±0.2°, 22.38±0.2°, and 26.00±0.2°.

In one embodiment, the crystal form A of the oxalate salt has an X-ray powder diffraction pattern, measured using Cu-Kα radiation and expressed in terms of 20 angles, comprising diffraction peaks at 4.64±0.2°, 7.32±0.2°, 8.47±0.2°, 12.93±0.2°, 13.47±0.2°, 14.62±0.2°, 15.23±0.2°, 17.00±0.2°, 18.05±0.2°, 18.58±0.2°, 19.44±0.2°, 20.57±0.2°, 21.49±0.2°, 22.38±0.2°, 23.26±0.2°, 23.59±0.2°, and 26.00±0.2°.

In one embodiment, the crystal form A of the oxalate salt has an X-ray powder diffraction pattern, measured using Cu-Kα radiation and expressed in terms of 20 angles, comprising diffraction peak positions and relative intensities substantially as shown in Table 8:

Table 8

| Position [°2θ] | Relative intensity [%] | Position [°2θ] | Relative intensity [%] |
|---|---|---|---|
| 4.6409 | 9.67 | 20.5689 | 32.57 |
| 7.3243 | 34.07 | 21.0873 | 24.03 |
| 8.4725 | 100.00 | 21.4883 | 39.50 |
| 12.9334 | 19.51 | 22.3807 | 86.67 |
| 13.4699 | 12.40 | 23.2608 | 31.24 |
| 14.6183 | 86.76 | 23.5935 | 30.33 |
| 15.2278 | 7.68 | 24.0272 | 21.48 |
| 16.9988 | 89.93 | 25.9955 | 32.08 |
| 18.0487 | 48.94 | 27.5449 | 4.60 |
| 18.5824 | 84.16 | 28.6854 | 3.47 |
| 18.8319 | 39.40 | 30.1750 | 12.59 |
| 19.4386 | 62.43 | 30.7971 | 5.66 |

In one embodiment, the crystal form A of the oxalate salt has an X-ray powder diffraction pattern, measured using Cu-Kα radiation and expressed in terms of 20 angles, substantially as shown in FIG. 38.

In one embodiment, the crystal form A of the oxalate salt has a differential scanning calorimetry curve showing an onset of an endothermic peak at 151.3±3°C.

In one embodiment, the crystal form A of the oxalate salt has a differential scanning calorimetry curve showing an endothermic peak at 153.6±3°C.

In one embodiment, the crystal form A of the oxalate salt has a differential scanning calorimetry curve showing an endothermic peak at 162.8±3°C.

In one embodiment, the crystal form A of the oxalate salt has a differential scanning calorimetry curve showing an endothermic peak at 218.8±3°C.

In one embodiment, the crystal form A of the oxalate salt has a thermogravimetric analysis curve showing a weight loss of approximately 4.10% at 150°C.

In one embodiment, the crystal form A of the oxalate salt has a differential scanning calorimetry curve substantially as shown in FIG. 40.

In one embodiment, the crystal form A of the oxalate salt has a thermogravimetric analysis curve substantially as shown in FIG. 39.

In one embodiment, the oxalate salt of the compound of Formula I is crystal form B of the oxalate salt of the compound of Formula I, wherein in the crystal form B of the oxalate salt, the molar ratio of the compound of Formula I to oxalic acid is 1:1;

wherein the crystal form B of the oxalate salt has an X-ray powder diffraction pattern, measured using Cu-Kα radiation and expressed in terms of 2θ angles, comprising diffraction peaks at 7.63±0.2°, 8.32±0.2°, 15.25±0.2°, 16.29±0.2°, 22.05 ±0.2°, and 23.84±0.2°.

In one embodiment, the crystal form B of the oxalate salt has an X-ray powder diffraction pattern, measured using

Cu-Kα radiation and expressed in terms of 2θ angles, further comprising diffraction peaks at one or more of the following positions: 12.47±0.2°, 17.66±0.2°, 18.26±0.2°, 18.77±0.2°, and 19.16±0.2°.

In one embodiment, the crystal form B of the oxalate salt has an X-ray powder diffraction pattern, measured using Cu-Kα radiation and expressed in terms of 20 angles, comprising diffraction peaks at 7.63±0.2°, 8.32±0.2°, 12.47±0.2°, 15.25±0.2°, 16.29±0.2°, 17.66±0.2°, 18.26±0.2°, 18.77±0.2°, 19.16±0.2°, 22.05±0.2°, and 23.84±0.2°.

In one embodiment, the crystal form B of the oxalate salt has an X-ray powder diffraction pattern, measured using Cu-Kα radiation and expressed in terms of 20 angles, comprising diffraction peaks at 7.63±0.2°, 8.32±0.2°, 12.47±0.2°, 15.25±0.2°, 16.29±0.2°, 17.66±0.2°, 18.26±0.2°, 18.77±0.2°, 19.16±0.2°, 19.92±0.2°, 20.83±0.2°, 21.28±0.2°, 22.05±0.2°, 23.84±0.2°, 25.05±0.2°, 26.40±0.2°, 27.41±0.2°, and 29.25±0.2°.

In one embodiment, in the crystal form B of the oxalate salt, the molar ratio of the compound of Formula I to oxalic acid is 1:1.

In one embodiment, the crystal form B of the oxalate salt has an X-ray powder diffraction pattern, measured using Cu-Kα radiation and expressed in terms of 20 angles, comprising diffraction peak positions and relative intensities substantially as shown in Table 9:

Table 9

| Position [°2θ] | Relative intensity [%] | Position [°2θ] | Relative intensity [%] |
|---|---|---|---|
| 7.6303 | 39.67 | 20.8286 | 39.95 |
| 8.3152 | 56.34 | 21.2807 | 28.72 |
| 12.4726 | 31.39 | 22.0493 | 70.88 |
| 15.2505 | 70.07 | 23.8443 | 93.78 |
| 16.2944 | 100.00 | 24.5635 | 18.67 |
| 17.6634 | 71.80 | 25.0476 | 23.37 |
| 18.2646 | 82.23 | 26.4010 | 16.45 |
| 18.4892 | 59.67 | 27.4132 | 20.10 |
| 18.7712 | 90.80 | 28.3635 | 5.97 |
| 19.1571 | 67.01 | 29.2511 | 13.78 |
| 19.9191 | 16.34 | | |

In one embodiment, the crystal form B of the oxalate salt has an X-ray powder diffraction pattern, measured using Cu-Kα radiation and expressed in terms of 20 angles, substantially as shown in FIG. 22.

In one embodiment, the crystal form B of the oxalate salt has a differential scanning calorimetry curve showing an onset of an endothermic peak at 155.5±3°C.

In one embodiment, the crystal form B of the oxalate salt has a differential scanning calorimetry curve showing an endothermic peak at 158.2±3°C.

In one embodiment, the crystal form B of the oxalate salt has a differential scanning calorimetry curve showing an endothermic peak at 218.7±3°C.

In one embodiment, the crystal form B of the oxalate salt has a thermogravimetric analysis curve showing a weight loss of approximately 1.21% at 150°C.

In one embodiment, the crystal form B of the oxalate salt has a differential scanning calorimetry curve substantially as shown in FIG. 24.

In one embodiment, the crystal form B of the oxalate salt has a thermogravimetric analysis curve substantially as shown in FIG. 23.

The present disclosure further provides a preparation method for the crystal form A of the oxalate salt of the compound of Formula I: mixing the compound of Formula I, oxalic acid, and a solvent, and slurrying at room temperature to obtain the crystal form A of the oxalate salt;

the solvent is a mixed solvent of an alcoholic solvent and water.

In one embodiment, the molar ratio of the oxalic acid to the compound of Formula I is 1.0-1.5; preferably 1.0.

In one embodiment, the molar ratio of the oxalic acid to the compound of Formula I is (1.0-1.5):1; preferably 1.0:1.

In one embodiment, in the mixed solvent of the alcohol solvent and water, the volume ratio of the alcohol solvent to water is (14-24): 1, preferably 19:1.

In one embodiment, the solvent is a mixed solvent of IPA/$H_2O$ (19:1, v/v).

The present disclosure further provides a preparation method for the crystal form B of the oxalate salt of the compound of Formula I: mixing the compound of Formula I, oxalic acid, and a solvent, and slurrying at room temperature to obtain the crystal form B of the oxalate salt;
the solvent is an ester solvent, an ether solvent, or a mixed solvent of a ketone solvent and a saturated alkane solvent.
In one embodiment, the molar ratio of the oxalic acid to the compound of Formula I is 1.0-1.5; preferably 1.0.
In one embodiment, the molar ratio of the oxalic acid to the compound of Formula I is (1.0-1.5):1; preferably 1.0:1.
In one embodiment, the molar volume ratio of the compound of Formula I to the solvent is 0.02-0.08 mol/L; preferably 0.05 mol/L.
In one embodiment, in the mixed solvent of the ketone solvent and the saturated alkane solvent, the volume ratio of the ketone solvent to the saturated alkane solvent is (0.5-2):1.
In one embodiment, the solvent is a mixed solvent of acetone/n-heptane (1:1, v/v), isopropyl acetate, or methyl *tert*-butyl ether.
The present disclosure further provides a maleate salt of the compound of Formula I.
In one embodiment, the maleate salt of the compound of Formula I is crystal form A of the maleate salt of the compound of Formula I, wherein the crystal form A of the maleate salt has an X-ray powder diffraction pattern, measured using Cu-Kα radiation and expressed in terms of 2θ angles, comprising diffraction peaks at 13.54±0.2°, 16.92±0.2°, 17.64±0.2°, 19.61±0.2°, and 23.77±0.2°.
In one embodiment, the crystal form A of the maleate salt has an X-ray powder diffraction pattern, measured using Cu-Kα radiation and expressed in terms of 2θ angles, further comprising diffraction peaks at one or more of the following positions: 3.44±0.2°, 9.64±0.2°, 18.08±0.20, 20.82±0.2°, and 27.20±0.2°.
In one embodiment, the crystal form A of the maleate salt has an X-ray powder diffraction pattern, measured using Cu-Kα radiation and expressed in terms of 2θ angles, comprising diffraction peaks at 3.44±0.2°, 9.64±0.2°, 13.54 ±0.2°, 16.92±0.2°, 17.64±0.2°, 18.08±0.2°, 19.61±0.2°, 20.82±0.2°, 23.77±0.2°, and 27.20±0.2°.
In one embodiment, the crystal form A of the maleate salt has an X-ray powder diffraction pattern, measured using Cu-Kα radiation and expressed in terms of 2θ angles, comprising diffraction peaks at 3.44±0.2°, 9.64±0.2°, 13.54 ±0.2°, 16.92±0.2°, 17.64±0.2°, 18.08±0.2°, 19.61±0.2°, 20.82±0.2°, 22.36±0.2°, 22.71±0.2°, 23.77±0.2°, 27.20 ±0.2°, 28.90±0.2°, and 30.67±0.2°.
In one embodiment, in the crystal form A of the maleate salt, the molar ratio of the compound of Formula I to maleic acid is 1:1.
In one embodiment, the crystal form A of the maleate salt has an X-ray powder diffraction pattern, measured using Cu-Kα radiation and expressed in terms of 2θ angles, comprising diffraction peak positions and relative intensities substantially as shown in Table 10:

Table 10

| Position [°2θ] | Relative intensity [%] | Position [°2θ] | Relative intensity [%] |
|---|---|---|---|
| 3.4417 | 16.32 | 21.6390 | 7.94 |
| 9.6432 | 27.14 | 22.3570 | 27.06 |
| 10.1448 | 5.95 | 22.7123 | 28.31 |
| 13.5352 | 100.00 | 23.7674 | 95.50 |
| 15.7013 | 7.54 | 24.4456 | 6.76 |
| 16.9220 | 49.89 | 24.8503 | 8.90 |
| 17.6360 | 60.94 | 25.5895 | 12.79 |
| 18.0813 | 36.58 | 26.7126 | 5.99 |
| 18.5713 | 13.66 | 27.2013 | 26.88 |
| 18.9912 | 11.46 | 27.7331 | 8.29 |
| 19.6099 | 51.55 | 28.8950 | 13.37 |
| 20.8195 | 31.63 | 30.6662 | 21.93 |

In one embodiment, the crystal form A of the maleate salt has an X-ray powder diffraction pattern, measured using Cu-Kα radiation and expressed in terms of 2θ angles, substantially as shown in FIG. 25.
In one embodiment, the crystal form A of the maleate salt has a differential scanning calorimetry curve showing an onset of an endothermic peak at 123.4±3°C.

In one embodiment, the crystal form A of the maleate salt has a differential scanning calorimetry curve showing an endothermic peak at 125.2±3°C.

In one embodiment, the crystal form A of the maleate salt has a thermogravimetric analysis curve showing a weight loss of approximately 2.56% at 120°C.

In one embodiment, the crystal form A of the maleate salt has a differential scanning calorimetry curve substantially as shown in FIG. 27.

In one embodiment, the crystal form A of the maleate salt has a thermogravimetric analysis curve substantially as shown in FIG. 26.

The present disclosure further provides a preparation method for the crystal form A of the maleate salt of the compound of Formula I: mixing the compound of Formula I, maleic acid, and a solvent, and slurrying at room temperature to obtain the crystal form A of the maleate salt;

the solvent is an ester solvent, an ether solvent, or a mixed solvent of a ketone solvent and a saturated alkane solvent.

In one embodiment, the molar ratio of the maleic acid to the compound of Formula I is 1.0-1.5; preferably 1.0.

In one embodiment, the molar ratio of the maleic acid to the compound of Formula I is (1.0-1.5):1; preferably 1.0:1.

In one embodiment, in the mixed solvent of the ketone solvent and the saturated alkane solvent, the volume ratio of the ketone solvent to the saturated alkane solvent is (0.5-2):1.

In one embodiment, the solvent is a mixed solvent of acetone/n-heptane (1:1, v/v), isopropyl acetate, or methyl *tert*-butyl ether.

The present disclosure further provides a malate salt of the compound of Formula I.

In one embodiment, the malate salt of the compound of Formula I is crystal form A of the malate salt of the compound of Formula I, wherein the crystal form A of the malate salt has an X-ray powder diffraction pattern, measured using Cu-Kα radiation and expressed in terms of 2θ angles, comprising diffraction peaks at 8.95±0.2°, 9.89±0.2°, 13.21 ±0.2°, 16.53±0.2°, and 18.93±0.2°.

In one embodiment, the crystal form A of the malate salt has an X-ray powder diffraction pattern, measured using Cu-Kα radiation and expressed in terms of 2θ angles, comprising diffraction peaks at 8.95±0.2°, 9.89±0.2°, 13.21±0.2°, 16.17±0.2°, 16.53±0.2°, 18.93±0.2°, and 24.77±0.2°.

In one embodiment, the crystal form A of the malate salt has an X-ray powder diffraction pattern, measured using Cu-Kα radiation and expressed in terms of 2θ angles, comprising diffraction peaks at 6.63±0.2°, 8.95±0.2°, 9.89±0.2°, 13.21±0.2°, 16.17±0.2°, 16.53±0.2°, 18.93±0.2°, 21.62±0.2°, 23.20±0.2°, 23.80±0.2°, and 24.77±0.2°.

In one embodiment, in the crystal form A of the malate salt, the molar ratio of the compound of Formula I to malic acid is 1:1.

In one embodiment, the crystal form A of the malate salt has an X-ray powder diffraction pattern, measured using Cu-Kα radiation and expressed in terms of 2θ angles, comprising diffraction peak positions and relative intensities substantially as shown in Table 11:

Table 11

| Position [°2θ] | Relative intensity [%] | Position [°2θ] | Relative intensity [%] |
|---|---|---|---|
| 6.6262 | 7.38 | 19.7373 | 21.21 |
| 8.9534 | 20.94 | 21.6214 | 25.16 |
| 9.8936 | 38.20 | 23.1988 | 17.64 |
| 13.2054 | 50.38 | 23.8001 | 22.22 |
| 16.1763 | 29.75 | 24.7654 | 32.77 |
| 16.5259 | 50.45 | 27.2491 | 13.36 |
| 17.8951 | 33.19 | 31.4661 | 5.26 |
| 18.9290 | 100.00 | | |

In one embodiment, the crystal form A of the malate salt has an X-ray powder diffraction pattern, measured using Cu-Kα radiation and expressed in terms of 2θ angles, substantially as shown in FIG. 28.

In one embodiment, the crystal form A of the malate salt has a differential scanning calorimetry curve showing an onset of an endothermic peak at 86.3±3°C.

In one embodiment, the crystal form A of the malate salt has a differential scanning calorimetry curve showing an endothermic peak at 96.3±3°C.

In one embodiment, the crystal form A of the malate salt has a thermogravimetric analysis curve showing a weight loss of approximately 2.26% at 150°C.

In one embodiment, the crystal form A of the malate salt has a differential scanning calorimetry curve substantially as shown in FIG. 30.

In one embodiment, the crystal form A of the malate salt has a thermogravimetric analysis curve substantially as shown in FIG. 29.

The present disclosure further provides a preparation method for the crystal form A of the malate salt of the compound of Formula I: mixing the compound of Formula I, malic acid, and a solvent, and slurrying at room temperature to obtain the crystal form A of the malate salt;

the solvent is a mixed solvent of a ketone solvent and a saturated alkane solvent.

In one embodiment, the molar ratio of the malic acid to the compound of Formula I is 1.0-1.5; preferably 1.0.

In one embodiment, the molar ratio of the malic acid to the compound of Formula I is (1.0-1.5):1; preferably 1.0:1.

In one embodiment, in the mixed solvent of the ketone solvent and the saturated alkane solvent, the volume ratio of the ketone solvent to the saturated alkane solvent is (0.5-2):1, preferably 1:1.

In one embodiment, the solvent is a mixed solvent of acetone/n-heptane (1:1, v/v).

The present disclosure further provides a succinate salt of the compound of Formula I.

In one embodiment, the succinate salt of the compound of Formula I is crystal form A of the succinate salt of the compound of Formula I, wherein the crystal form A of the succinate salt has an X-ray powder diffraction pattern, measured using Cu-K$\alpha$ radiation and expressed in terms of 2θ angles, comprising diffraction peaks at 6.94± 0.2°, 9.20±0.2°, 13.87 ±0.2°, 16.29±0.2°, 17.94±0.2°, and 20.21±0.2°.

In one embodiment, the crystal form A of the succinate salt has an X-ray powder diffraction pattern, measured using Cu-K$\alpha$ radiation and expressed in terms of 2θ angles, further comprising diffraction peaks at one or more of the following positions: 14.70±0.2°, 17.40±0.2°, 19.48±0.2°, 20.96±0.2°, 22.18±0.2°, 23.04±0.2°, 23.92±0.2°, and 26.18 ±0.2°.

In one embodiment, the crystal form A of the succinate salt has an X-ray powder diffraction pattern, measured using Cu-K$\alpha$ radiation and expressed in terms of 2θ angles, comprising diffraction peaks at 6.94±0.2°, 9.20±0.2°, 13.87 ±0.2°, 16.29±0.2°, 17.94±0.2°, 20.21±0.2°, 20.96±0.2°, 22.18±0.2°, and 26.18±0.2°.

In one embodiment, the crystal form A of the succinate salt has an X-ray powder diffraction pattern, measured using Cu-K$\alpha$ radiation and expressed in terms of 2θ angles, comprising diffraction peaks at 6.94±0.2°, 9.20±0.2°, 13.87 ±0.2°, 14.70±0.2°, 16.29±0.2°, 17.40±0.2°, 17.94±0.2°, 19.48±0.2°, 20.21±0.2°, 20.96±0.2°, 22.18±0.2°, 23.04 ±0.2°, 23.92±0.2°, and 26.18±0.2°.

In one embodiment, in the crystal form A of the succinate salt, the molar ratio of the compound of Formula I to succinic acid is 1:1.

In one embodiment, the crystal form A of the succinate salt has an X-ray powder diffraction pattern, measured using Cu-K$\alpha$ radiation and expressed in terms of 2θ angles, comprising diffraction peak positions and relative intensities substantially as shown in Table 12:

Table 12

| Position [°2θ] | Relative intensity [%] | Position [°2θ] | Relative intensity [%] |
|---|---|---|---|
| 6.9349 | 100.00 | 19.4760 | 19.47 |
| 9.1969 | 38.52 | 20.2096 | 35.96 |
| 11.7777 | 6.58 | 20.9648 | 45.17 |
| 13.8697 | 56.93 | 22.1780 | 31.28 |
| 14.6969 | 18.62 | 23.0453 | 19.19 |
| 16.2949 | 72.71 | 23.9225 | 28.52 |
| 17.3959 | 14.28 | 26.1847 | 32.80 |
| 17.9354 | 39.73 | 28.0540 | 5.21 |
| 18.3860 | 12.73 | 34.8694 | 3.06 |

In one embodiment, in the crystal form A of the succinate salt, the molar ratio of the compound of Formula I to succinic acid is 1:1.2, 1:1.1, or 1:1.

In one embodiment, the crystal form A of the succinate salt has an X-ray powder diffraction pattern, measured using Cu-K$\alpha$ radiation and expressed in terms of 2θ angles, substantially as shown in FIG. 31.

In one embodiment, the crystal form A of the succinate salt has a differential scanning calorimetry curve showing an onset of an endothermic peak at 75.9±3°C.

In one embodiment, the crystal form A of the succinate salt has a differential scanning calorimetry curve showing an endothermic peak at 85.4±3°C.

In one embodiment, the crystal form A of the succinate salt has a thermogravimetric analysis curve showing a weight loss of approximately 4.49% at 150°C.

In one embodiment, the crystal form A of the succinate salt has a differential scanning calorimetry curve substantially as shown in FIG. 33.

In one embodiment, the crystal form A of the succinate salt has a thermogravimetric analysis curve substantially as shown in FIG. 32.

The present disclosure further provides a preparation method for the crystal form A of the succinate salt of the compound of Formula I: mixing the compound of Formula I, succinic acid, and a solvent, and slurrying at room temperature to obtain the crystal form A of the succinate salt;
the solvent is an ether solvent.

In one embodiment, the molar ratio of the succinic acid to the compound of Formula I is 1.0-1.5; preferably 1.0.

In one embodiment, the molar ratio of the succinic acid to the compound of Formula I is (1.0-1.5):1; preferably 1.0:1.

In one embodiment, the solvent is methyl *tert*-butyl ether.

The present disclosure further provides a methanesulfonate salt of the compound of Formula I.

In one embodiment, the methanesulfonate salt of the compound of Formula I is crystal form A of the methanesulfonate salt of the compound of Formula I, wherein the crystal form A of the methanesulfonate salt has an X-ray powder diffraction pattern, measured using Cu-Kα radiation and expressed in terms of 20 angles, comprising diffraction peaks at 5.03±0.2°, 17.03±0.2°, 18.11±0.2°, 20.60±0.2°, and 23.40±0.2°.

In one embodiment, the crystal form A of the methanesulfonate salt has an X-ray powder diffraction pattern, measured using Cu-Kα radiation and expressed in terms of 20 angles, comprising diffraction peak positions and relative intensities substantially as shown in Table 13:

Table 13

| Position [°2θ] | Relative intensity [%] | Position [°2θ] | Relative intensity [%] |
|---|---|---|---|
| 5.0258 | 56.54 | 20.5965 | 100.00 |
| 17.0335 | 38.98 | 23.3964 | 71.59 |
| 18.1100 | 61.40 | | |

In one embodiment, the crystal form A of the methanesulfonate salt has an X-ray powder diffraction pattern, measured using Cu-Kα radiation and expressed in terms of 20 angles, substantially as shown in FIG. 34.

In one embodiment, the methanesulfonate salt of the compound of Formula I is crystal form B of the methanesulfonate salt of the compound of Formula I, wherein the crystal form B of the methanesulfonate salt has an X-ray powder diffraction pattern, measured using Cu-Kα radiation and expressed in terms of 20 angles, comprising diffraction peaks at 18.89±0.2°, 20.43±0.2°, 21.07±0.2°, 23.44±0.2°, and 24.46±0.2°.

In one embodiment, the crystal form B of the methanesulfonate salt has an X-ray powder diffraction pattern, measured using Cu-Kα radiation and expressed in terms of 20 angles, further comprising diffraction peaks at one or more of the following positions: 9.76±0.2°, 11.32±0.2°, 15.46±0.2°, and 17.26±0.2°.

In one embodiment, the crystal form B of the methanesulfonate salt has an X-ray powder diffraction pattern, measured using Cu-Kα radiation and expressed in terms of 2θ angles, further comprising diffraction peaks at one or more of the following positions: 9.83±0.2°, 11.32±0.2°, 15.46±0.2°, and 17.26±0.2°.

In one embodiment, the crystal form B of the methanesulfonate salt has an X-ray powder diffraction pattern, measured using Cu-Kα radiation and expressed in terms of 20 angles, comprising diffraction peaks at 9.76±0.2°, 11.32 ±0.2°, 15.46±0.2°, 17.26±0.2°, 18.89±0.2°, 20.43±0.2°, 21.07±0.2°, 23.44±0.2°, and 24.46±0.2°.

In one embodiment, the crystal form B of the methanesulfonate salt has an X-ray powder diffraction pattern, measured using Cu-Kα radiation and expressed in terms of 20 angles, comprising diffraction peaks at 9.83±0.2°, 11.32 ±0.2°, 15.46±0.2°, 17.26±0.2°, 18.89±0.2°, 20.43±0.2°, 21.07±0.2°, 23.44±0.2°, and 24.46±0.2°.

In one embodiment, the crystal form B of the methanesulfonate salt has an X-ray powder diffraction pattern, measured using Cu-Kα radiation and expressed in terms of 20 angles, comprising diffraction peaks at 9.76±0.2°, 11.32 ±0.2°, 15.46±0.2°, 16.98±0.2°, 17.26±0.2°, 17.63±0.2°, 18.89±0.2°, 19.68±0.2°, 20.43±0.2°, 21.07±0.2°, 21.58 ±0.2°, 22.09±0.2°, 22.69±0.2°, 23.44±0.2°, and 24.46±0.2°.

In one embodiment, the crystal form B of the methanesulfonate salt has an X-ray powder diffraction pattern, measured using Cu-Kα radiation and expressed in terms of 20 angles, comprising diffraction peaks at 9.83±0.2°, 11.32 ±0.2°, 15.46±0.2°, 16.98±0.2°, 17.26±0.2°, 17.63±0.2°, 18.89±0.2°, 19.68±0.2°, 20.43±0.2°, 21.07±0.2°, 21.58±0.2°, 22.09±0.2°, 22.69±0.2°, 23.44±0.2°, and 24.46±0.2°.

In one embodiment, in the crystal form B of the methanesulfonate salt, the molar ratio of the compound of Formula I to methanesulfonic acid is 1:2.

In one embodiment, the crystal form B of the methanesulfonate salt has an X-ray powder diffraction pattern, measured using Cu-Kα radiation and expressed in terms of 2θ angles, comprising diffraction peak positions and relative intensities substantially as shown in Table 14:

Table 14

| Position [°2θ] | Relative intensity [%] | Position [°2θ] | Relative intensity [%] |
|---|---|---|---|
| 9.8298 | 9.76 | 22.6933 | 23.52 |
| 11.3186 | 15.66 | 23.4414 | 73.53 |
| 11.8183 | 5.19 | 23.9398 | 15.74 |
| 12.6882 | 3.11 | 24.4552 | 60.92 |
| 13.7455 | 5.81 | 25.3114 | 10.00 |
| 14.3775 | 6.16 | 25.6224 | 15.39 |
| 15.4572 | 22.47 | 25.8897 | 14.19 |
| 16.5409 | 6.51 | 27.0121 | 4.85 |
| 16.9751 | 29.83 | 27.4334 | 6.22 |
| 17.2607 | 52.29 | 27.9938 | 6.98 |
| 17.6280 | 39.70 | 28.5662 | 8.75 |
| 18.4451 | 35.15 | 29.3314 | 8.75 |
| 18.8911 | 100.00 | 29.7831 | 9.39 |
| 19.6841 | 30.75 | 30.3249 | 5.40 |
| 20.4301 | 65.78 | 31.3892 | 5.83 |
| 21.0733 | 65.86 | 32.6597 | 5.59 |
| 21.5827 | 20.17 | 35.2268 | 6.84 |
| 22.0878 | 29.37 | 37.3650 | 3.62 |

In one embodiment, in the crystal form B of the methanesulfonate salt, the molar ratio of the compound of Formula I to methanesulfonic acid is 1:2.1 or 1:2.

In one embodiment, the crystal form B of the methanesulfonate salt has an X-ray powder diffraction pattern, measured using Cu-Kα radiation and expressed in terms of 2θ angles, substantially as shown in FIG. 35.

In one embodiment, the crystal form B of the methanesulfonate salt has a differential scanning calorimetry curve showing an onset of an endothermic peak at 97.3±3°C.

In one embodiment, the crystal form B of the methanesulfonate salt has a differential scanning calorimetry curve showing an endothermic peak at 109.4±3°C.

In one embodiment, the crystal form B of the methanesulfonate salt has a thermogravimetric analysis curve showing a weight loss of approximately 0.91% at 80°C and a weight loss of approximately 2.40% at 80-130°C.

In one embodiment, the crystal form B of the methanesulfonate salt has a differential scanning calorimetry curve substantially as shown in FIG. 37.

In one embodiment, the crystal form B of the methanesulfonate salt has a thermogravimetric analysis curve substantially as shown in FIG. 36.

The present disclosure further provides a preparation method for the crystal form A of the methanesulfonate salt of the compound of Formula I: mixing the compound of Formula I, methanesulfonic acid, and a solvent, and slurrying at room temperature to obtain the crystal form A of the methanesulfonate salt;

the solvent is a mixed solvent of a ketone solvent and a saturated alkane solvent.

In one embodiment, the molar ratio of the methanesulfonic acid to the compound of Formula I is (1.0-1.2):1; preferably 1:1.

In one embodiment, in the mixed solvent of the ketone solvent and the saturated alkane solvent, the volume ratio of the ketone solvent to the saturated alkane solvent is (0.5-2):1, preferably 1:1.

In one embodiment, the solvent is a mixed solvent of acetone/n-heptane (1:1, v/v).

The present disclosure further provides a preparation method for the crystal form B of the methanesulfonate salt of the compound of Formula I: mixing the compound of Formula I, methanesulfonic acid, and a solvent, and slurrying at room temperature to obtain the crystal form B of the methanesulfonate salt;

the solvent is an ester solvent, an ether solvent, or a mixed solvent of a ketone solvent and a saturated alkane solvent.

In one embodiment, the molar ratio of the methanesulfonic acid to the compound of Formula I is (1.5-2.5):1; preferably 2:1.

In one embodiment, in the mixed solvent of the ketone solvent and the saturated alkane solvent, the volume ratio of the ketone solvent to the saturated alkane solvent is (0.5-2):1.

In one embodiment, the solvent is a mixed solvent of acetone/n-heptane (1:1, v/v), isopropyl acetate, or methyl *tert-butyl* ether.

The present disclosure further provides a 2-hydroxyethanesulfonate salt of the compound of Formula I.

In one embodiment, the 2-hydroxyethanesulfonate salt of the compound of Formula I is crystal form A of the 2-hydroxyethanesulfonate salt of the compound of Formula I, wherein the crystal form A of the 2-hydroxyethanesulfonate salt has an X-ray powder diffraction pattern, measured using Cu-Kα radiation and expressed in terms of 20 angles, comprising diffraction peaks at 4.85± 0.2°, 9.85±0.2°, 10.98±0.2°, 18.09±0.2°, 19.35±0.2°, and 20.33±0.2°.

In one embodiment, the crystal form A of the 2-hydroxyethanesulfonate salt has an X-ray powder diffraction pattern, measured using Cu-Kα radiation and expressed in terms of 20 angles, further comprising diffraction peaks at one or more of the following positions: 15.54±0.2°, 15.94±0.2°, 21.83±0.2°, 22.33±0.2°, and 22.87±0.2°.

In one embodiment, the crystal form A of the 2-hydroxyethanesulfonate salt has an X-ray powder diffraction pattern, measured using Cu-Kα radiation and expressed in terms of 20 angles, comprising diffraction peaks at 4.85±0.2°, 9.85±0.2°, 10.98±0.2°, 15.54±0.2°, 15.94±0.2°, 18.09±0.2°, 19.35±0.2°, 20.33±0.2°, 21.83±0.2°, 22.33±0.2°, and 22.87±0.2°.

In one embodiment, the crystal form A of the 2-hydroxyethanesulfonate salt has an X-ray powder diffraction pattern, measured using Cu-Kα radiation and expressed in terms of 20 angles, comprising diffraction peaks at 4.85±0.2°, 9.85±0.2°, 10.98±0.2°, 12.83±0.2°, 15.54±0.2°, 15.94+0.2°, 16.66±0.2°, 18.09±0.2°, 19.35±0.2°, 20.33±0.2°, 21.83 ±0.2°, 22.33±0.2°, and 22.87±0.2°.

In one embodiment, in the crystal form A of the 2-hydroxyethanesulfonate salt, the molar ratio of the compound of Formula I to 2-hydroxyethanesulfonic acid is 1:1.

In one embodiment, the crystal form A of the 2-hydroxyethanesulfonate salt has an X-ray powder diffraction pattern, measured using Cu-Kα radiation and expressed in terms of 20 angles, comprising diffraction peak positions and relative intensities substantially as shown in Table 15:

Table 15

| Position [°2θ] | Relative intensity [%] | Position [°2θ] | Relative intensity [%] |
|---|---|---|---|
| 4.8473 | 97.56 | 20.3297 | 56.13 |
| 9.8479 | 49.33 | 21.8277 | 45.05 |
| 10.9792 | 82.75 | 22.3258 | 67.31 |
| 12.8290 | 12.79 | 22.8682 | 63.84 |
| 14.2549 | 4.53 | 24.9854 | 21.98 |
| 15.5419 | 25.71 | 26.2466 | 10.34 |
| 15.9356 | 30.48 | 26.9452 | 8.37 |
| 16.6623 | 12.53 | 28.5116 | 17.43 |
| 18.0907 | 88.25 | 31.1759 | 4.85 |
| 19.3520 | 100.00 | | |

In one embodiment, the crystal form A of the 2-hydroxyethanesulfonate salt has an X-ray powder diffraction pattern, measured using Cu-Kα radiation and expressed in terms of 20 angles, substantially as shown in FIG. 41.

In one embodiment, the crystal form A of the 2-hydroxyethanesulfonate salt has a differential scanning calorimetry curve showing an onset of an endothermic peak at 109.5±3°C.

In one embodiment, the crystal form A of the 2-hydroxyethanesulfonate salt has a differential scanning calorimetry curve showing an endothermic peak at 118.4±3°C.

In one embodiment, the crystal form A of the 2-hydroxyethanesulfonate salt has a thermogravimetric analysis

curve showing a weight loss of approximately 1.28% at 110°C.

In one embodiment, the crystal form A of the 2-hydroxyethanesulfonate salt has a differential scanning calorimetry curve substantially as shown in FIG. 43.

In one embodiment, the crystal form A of the 2-hydroxyethanesulfonate salt has a thermogravimetric analysis curve substantially as shown in FIG. 42.

The present disclosure further provides a preparation method for the crystal form A of the 2-hydroxyethanesulfonate salt of the compound of Formula I: mixing the compound of Formula I, 2-hydroxyethanesulfonic acid, and a solvent, and slurrying at room temperature to obtain the crystal form A of the 2-hydroxyethanesulfonate salt;

the solvent is an ester solvent, or a mixed solvent of a ketone solvent and a saturated alkane solvent.

In one embodiment, the molar ratio of the 2-hydroxyethanesulfonic acid to the compound of Formula I is (1.5-2.5):1; preferably 2:1.

In one embodiment, in the mixed solvent of the ketone solvent and the saturated alkane solvent, the volume ratio of the ketone solvent to the saturated alkane solvent is (0.5-2):1.

In one embodiment, the solvent is a mixed solvent of acetone/n-heptane (1:1, v/v), or isopropyl acetate.

The present disclosure further provides a pharmaceutical composition comprising the crystal form or salt form of the compound of Formula I according to any one of the above embodiments, and at least one pharmaceutical excipient.

The selection of the pharmaceutical excipient varies depending on the route of administration and functional characteristics, and the pharmaceutical excipient may typically be conventional fillers, diluents, binders, wetting agents, disintegrants, lubricants, emulsifiers, suspending agents, and the like in the art.

The present disclosure further provides a use of a substance M, wherein the substance M is the crystal form or salt form of the compound of Formula I according to any one of the above embodiments, or the above pharmaceutical composition,
the use comprises:

1) inhibiting tubulin polymerization and/or Src kinase;
2) preventing and/or treating a disease related to tubulin polymerization and/or Src kinase;
3) preparing a tubulin polymerization and/or Src kinase inhibitor;
4) preparing a medicament or formulation for preventing and/or treating a disease related to tubulin polymerization and/or Src kinase.

Preferably, the use comprises: inhibiting tubulin polymerization; and/or, preventing and/or treating a disease mediated by tubulin polymerization; and/or, preparing a tubulin polymerization inhibitor, and/or a medicament, pharmaceutical composition, or formulation for preventing and/or treating a disease mediated by tubulin polymerization.

Preferably, the use comprises: inhibiting Src kinase; and/or, preventing and/or treating a disease mediated by Src kinase; and/or, preparing a Src kinase inhibitor; and/or, preparing a medicament, pharmaceutical composition, or formulation for preventing and/or treating a disease mediated by Src kinase.

Preferably, the medicament is an external preparation.

Preferably, the medicament is transdermally administered.

Preferably, the disease related to tubulin polymerization and/or Src kinase comprises tumor and skin disease.

The present disclosure further provides a use of a substance M, wherein the substance M is the crystal form or salt form of the compound of Formula I according to any one of the above embodiments, or the above pharmaceutical composition,
the use is a use in the preparation of a medicament for treating or preventing a tumor and/or a skin disease.

Preferably, the tumor comprises: solid tumor, sarcoma, and hematological cancer;
More preferably, the tumor comprises: breast cancer, ovarian cancer, prostate cancer, cervical cancer, testicular cancer, colon cancer, colorectal cancer, liver cancer, non-small cell lung cancer, squamous cell carcinoma (such as cutaneous squamous cell carcinoma), small cell lung cancer, gastric cancer, gastrointestinal stromal tumor, pancreatic cancer, bladder cancer, germ cell tumor, mast cell tumor, mastocytosis, glioblastoma, neuroblastoma, astrocytoma, melanoma, B-cell lymphoma, T-cell lymphoma, slowly progressive lymphoma, Hodgkin lymphoma, non-Hodgkin lymphoma, acute myeloid leukemia, acute lymphoblastic leukemia, chronic myeloid leukemia, chronic lymphocytic leukemia, myeloma, and/or myelodysplastic syndrome.

Preferably, the skin disease comprises: actinic keratosis, psoriasis, atopic dermatitis, psoriasis, vitiligo, roseola, and/or systemic lupus erythematosus.

Preferably, the medicament is an external preparation.

Preferably, the medicament is transdermally administered.

The present disclosure further provides a use of at least one of the crystal forms or salt forms of the compound of Formula I as described above for treating or preventing a tumor and/or a skin disease.

Preferably, the tumor comprises: solid tumor, sarcoma, and hematological cancer;

More preferably, the tumor comprises: breast cancer, ovarian cancer, prostate cancer, cervical cancer, testicular cancer, colon cancer, colorectal cancer, liver cancer, non-small cell lung cancer, squamous cell carcinoma (such as cutaneous squamous cell carcinoma), small cell lung cancer, gastric cancer, gastrointestinal stromal tumor, pancreatic cancer, bladder cancer, germ cell tumor, mast cell tumor, mastocytosis, glioblastoma, neuroblastoma, astrocytoma, melanoma, B-cell lymphoma, T-cell lymphoma, slowly progressive lymphoma, Hodgkin lymphoma, non-Hodgkin lymphoma, acute myeloid leukemia, acute lymphoblastic leukemia, chronic myeloid leukemia, chronic lymphocytic leukemia, myeloma, and/or myelodysplastic syndrome.

Preferably, the skin disease comprises: actinic keratosis, psoriasis, atopic dermatitis, psoriasis, vitiligo, roseola, and/or systemic lupus erythematosus.

The present disclosure further provides a method for inhibiting Src kinase, or preventing and/or treating a disease related to (or mediated by) Src kinase, comprising the step of: administering the above crystal form and/or salt form to a subject in need thereof.

The present disclosure further provides a method for inhibiting tubulin, or preventing and/or treating a disease related to (or mediated by) tubulin, comprising the step of: administering the crystal form and/or salt form according to the present disclosure to a subject in need thereof.

The above preferred conditions can be combined arbitrarily to obtain preferred embodiments of the present disclosure without violating common knowledge in the art.

Terminology

When the terms "approximately" and "about" are used in reference to XRPD peaks, these terms indicate an error in the mentioned X-ray powder diffraction peak positions [°2θ] of: ±0.3, ±0.2, or ±0.1, preferably ±0.2, and more preferably ±0.1.

When referring to XRPD peak positions, the 20 angles may be rounded to retain 2 or 4 significant digits after the decimal point.

When the terms "approximately" and "about" are used in reference to a temperature or temperature range, these terms indicate an error in the mentioned temperature or temperature range of: ±5°C, ±3°C, ±2°C, or ±1°C, preferably ±3°C.

The reagents and raw materials used in the present disclosure are all commercially available.

The positive and progressive effects of the present disclosure are as follows: a compound and salt forms and crystal forms thereof as tubulin-SRC dual-target inhibitors, wherein the compound has the structure of Formula I as described in the present disclosure. The compound and the salt forms and crystal forms thereof can be used as dual-target inhibitors of tubulin and Src kinase, or as tubulin inhibitors or Src kinase inhibitors alone. The compound of the present disclosure can significantly inhibit the polymerization of tubulin monomers and inhibit cell proliferation. It has good pharmacokinetic properties for topical administration to the skin and has good druggability. It can be used for preparing skin external preparations with the advantages of fast metabolism and low side effects.

BRIEF DESCRIPTION OF THE DRAWINGS

FIG. 1: XRPD pattern of free form crystal form A;
FIG. 2: TGA curve of free form crystal form A;
FIG. 3: DSC curve of free form crystal form A;
FIG. 4: XRPD pattern of free form crystal form B;
FIG. 5: TGA curve of free form crystal form B;
FIG. 6: DSC curve of free form crystal form B;
FIG. 7: XRPD pattern of crystal form A of phosphate salt;
FIG. 8: TGA curve of crystal form A of phosphate salt;
FIG. 9: DSC curve of crystal form A of phosphate salt;
FIG. 10: VT-XRPD pattern of crystal form A of phosphate salt;
FIG. 11: XRPD pattern of crystal form B of phosphate salt;
FIG. 12: XRPD pattern of crystal form A of fumarate salt;
FIG. 13: TGA curve of crystal form A of fumarate salt;
FIG. 14: DSC curve of crystal form A of fumarate salt;
FIG. 15: VH-XRPD pattern of crystal form A of fumarate salt;
FIG. 16: XRPD pattern of crystal form A of *p*-toluenesulfonate salt;
FIG. 17: TGA curve of crystal form A of *p*-toluenesulfonate salt;

FIG. 18: DSC curve of crystal form A of *p*-toluenesulfonate salt;
FIG. 19: XRPD pattern of crystal form A of benzenesulfonate salt;
FIG. 20: TGA curve of crystal form A of benzenesulfonate salt;
FIG. 21: DSC curve of crystal form A of benzenesulfonate salt;
FIG. 22: XRPD pattern of crystal form B of oxalate salt;
FIG. 23: TGA curve of crystal form B of oxalate salt;
FIG. 24: DSC curve of crystal form B of oxalate salt;
FIG. 25: XRPD pattern of crystal form A of maleate salt;
FIG. 26: TGA curve of crystal form A of maleate salt;
FIG. 27: DSC curve of crystal form A of maleate salt;
FIG. 28: XRPD pattern of crystal form A of malate salt;
FIG. 29: TGA curve of crystal form A of malate salt;
FIG. 30: DSC curve of crystal form A of malate salt;
FIG. 31: XRPD pattern of crystal form A of succinate salt;
FIG. 32: TGA curve of crystal form A of succinate salt;
FIG. 33: DSC curve of crystal form A of succinate salt;
FIG. 34: XRPD pattern of crystal form A of methanesulfonate salt;
FIG. 35: XRPD pattern of crystal form B of methanesulfonate salt;
FIG. 36: TGA curve of crystal form B of methanesulfonate salt;
FIG. 37: DSC curve of crystal form B of methanesulfonate salt;
FIG. 38: XRPD pattern of crystal form A of oxalate salt;
FIG. 39: TGA curve of crystal form A of oxalate salt;
FIG. 40: DSC curve of crystal form A of oxalate salt;
FIG. 41: XRPD pattern of crystal form A of 2-hydroxyethanesulfonate salt;
FIG. 42: TGA curve of crystal form A of 2-hydroxyethanesulfonate salt;
FIG. 43: DSC curve of crystal form A of 2-hydroxyethanesulfonate salt;
FIG. 44: Dynamic solubility curve in water at 37°C;
FIG. 45: Dynamic solubility curve in SGF at 37°C;
FIG. 46: Dynamic solubility curve in FaSSIF at 37°C;
FIG. 47: Dynamic solubility curve in FeSSIF at 37°C;
FIG. 48: DVS curve of free form crystal form A;
FIG. 49: XRPD overlay of free form crystal form A before and after DVS testing;
FIG. 50: DVS curve of crystal form A of phosphate salt;
FIG. 51: XRPD overlay of crystal form A of phosphate salt before and after DVS testing;
FIG. 52: DVS curve of crystal form A of fumarate salt;
FIG. 53: XRPD overlay of crystal form A of fumarate salt before and after DVS test;
FIG. 54: DVS curve of crystal form A of *p*-toluenesulfonate salt;
FIG. 55: XRPD overlay of crystal form A of *p*-toluenesulfonate salt before and after DVS testing;
FIG. 56: DVS curve of crystal form A of benzenesulfonate salt;
FIG. 57: XRPD overlay of crystal form A of benzenesulfonate salt before and after DVS testing;
FIG. 58: DVS curve of crystal form B of oxalate salt;
FIG. 59: XRPD overlay of crystal form B of oxalate salt before and after DVS testing;
FIG. 60: XRPD overlay of the stability evaluation samples for free form crystal form A;
FIG. 61: XRPD overlay of the stability evaluation samples for crystal form A of phosphate salt;
FIG. 62: XRPD overlay of the stability evaluation samples for crystal form A of the fumarate salt;
FIG. 63: XRPD overlay of the stability evaluation samples for crystal form A of *p*-toluenesulfonate salt;
FIG. 64: XRPD overlay of the stability evaluation samples for crystal form A of benzenesulfonate salt;
FIG. 65: XRPD overlay of the stability evaluation samples for crystal form B of oxalate salt;
FIG. 66: Results of tubulin polymerization inhibition assay for the compound of Formula I;
FIG. 67: Results of p-SRC inhibitory activity assay.

## DETAILED DESCRIPTION OF THE PREFERRED EMBODIMENT

The present disclosure is further described below by the way of examples, but the present disclosure is not thereby limited to the scope of the described examples. The experimental methods for which the specific conditions are not specified in the following examples are selected according to the conventional methods and conditions, or according to the commodity instructions.

Instrumental information and methods

1. X-ray powder diffraction (XRPD)

The XRPD pattern was collected on an X-ray powder diffraction analyzer produced by PANalytacal, and the scanning parameters are shown in Table 16.

Table 16 XRPD test parameters

| Parameter | VT-XRPD/VH-XRPD | XRPD |
|---|---|---|
| Model | Empyrean | Empyrean/X' Pert3 |
| X-ray | Cu, Kα,<br>Kα1 (Å): 1.540598,<br>Kα2 (Å): 1.544426<br>Kα2/Kα1 intensity ratio:0.50 | Cu, Kα,<br>Kα1 (Å): 1.540598,<br>Kα2 (Å): 1.544426<br>Kα2/Kα1 intensity ratio:0.50 |
| X-ray tube setting | 45 kV, 40 mA | 45 kV, 40 mA |
| Divergence slit | 1/8° | 1/8° |
| Scan mode | Continuous | Continuous |
| Scan range (°2θ) | 3-40 | 3-40 |
| Scan time per step (s) | 33.0 | 46.7 |
| Scan step size (°2θ) | 0.0167 | 0.0263 |
| Test time | about 10 minutes and 13 seconds | about 5 minutes |

2. Thermogravimetric analysis (TGA) and differential scanning calorimetry (DSC)

TGA and DSC curves were collected on a TA 5500 thermogravimetric analyzer and a TA 2500 differential scanning calorimeter, respectively, and the test parameters are listed in Table 17.

Table 17 TGA and DSC test parameters

| Parameter | TGA | DSC |
|---|---|---|
| Method | Linear heating | Linear heating |
| Sample tray | Aluminum pan, open | Aluminum pan, with lid/without lid |
| Temperature range | Room temperature-set endpoint temperature | 25°C-set endpoint temperature |
| Purge rate (°C/min) | 10 | 10 |
| Protective gas | Nitrogen | Nitrogen |

3. Dynamic vapor sorption (DVS)

The dynamic vapor sorption (DVS) curve was collected on DVS IntrInsic from SMS (surface measurement systems). The relative humidity at 25°C was corrected using the deliquescence points of LiCl, $Mg(NO_3)_2$, and KCl. DVS test parameters are listed in Table 18.

Table 18 DVS test parameters

| Parameter | Set value |
|---|---|
| Temperature | 25°C |
| Sample weight | 10-20 mg |
| Protective gas and flow rate | $N_2$, 200 mL/min |
| dm/dt | 0.002%/min |

(continued)

| Parameter | Set value |
|---|---|
| Minimum dm/dt equilibration time | 10 min |
| Maximum equilibrium time | 180 min |
| RH range | 0%RH-95%RH |
| RH gradient | 10%RH (0%RH to 90%RH & 90%RH to 0%RH)<br>5%RH (90%RH to 95%RH & 95%RH to 90%RH) |

4. Polarized light microscopy (PLM)

Polarized light microscope photographs were taken at room temperature using a Zeiss Axio Scope. Al microscope.

5. Liquid nuclear magnetic resonance ($^1H$ NMR)

Liquid NMR spectra were collected on a Bruker 400M NMR spectrometer, using DMSO-$d_6$ or $CD_3OD$ as solvents.

6. High-performance liquid chromatography and ion chromatography (HPLC/IC)

The purity test, dynamic solubility test, and stability test in the assay were measured using an Agilent 1260 high-performance liquid chromatograph, and the salt formation molar ratio of ions was tested by ion chromatography, and the analytical conditions are shown in Table 19 and Table 20.

Table 19 High-performance liquid chromatography test conditions

| | | | | |
|---|---|---|---|---|
| Liquid chromatograph | Agilent 1260 detector | | | |
| Chromatographic column | Waters XBridge C18, 4.6 mm/150 mm/3 μm | | | |
| Mobile phase | A: 0.1% $NH_3 \cdot H_2O$ in $H_2O$ | | | |
| | B: Acetonitrile | | | |
| Gradient | Solubility | | Purity | |
| | Time (min) | %B | Time (min) | %B |
| | 0.0 | 10 | 0.0 | 15 |
| | 10.0 | 90 | 25.0 | 90 |
| | 12.0 | 90 | 27.0 | 90 |
| | 12.1 | 10 | 27.1 | 15 |
| | 15.0 | 10 | 30.0 | 15 |
| Run time | 15 min | | 30 min | |
| Flow rate for mobile phase | 1.0 mL/min | | | |
| Injection volume | 5 μL | | | |
| Liquid chromatograph | Agilent 1260 detector | | | |
| Detection wavelength | UV at 258 nm | | | |
| Column temperature | 30°C | | | |
| Injector temperature | RT | | | |
| Diluent | ACN or ACN/$H_2O$ (1:1) | | | |

Table 20 Ion chromatography test conditions

| | |
|---|---|
| Ion chromatograph | ThermoFisher ICS-1100 |
| Chromatographic column | IonPac AS18 Analytical Column, 250 * 4 mm |
| Mobile phase | 25 mM NaOH |
| Injection volume | 25 μL |
| Flow rate | 1.0 mL/min |
| Temperature | 35°C |
| Column temperature | 35°C |
| Current | 80 mA |
| Run time | 20 min |

Name-Abbreviationreference table for solvents

The abbreviations of solvents are listed against the correspondingName in Table 21.

Table 21 Comparison table of Name and Abbreviation of solvents used in the assay

| Abbreviation | Name | Abbreviation | Name |
|---|---|---|---|
| MeOH | Methanol | 1,4-Dioxane | Dioxane |
| EtOH | Ethanol | ACN | Acetonitrile |
| IPA | Isopropanol | DCM | Dichloromethane |
| Acetone | Propan-2-one | $CHCl_3$ | Chloroform |
| MIBK | Methyl isobutyl ketone | Toluene | Methylbenzene |
| EtOAc | Ethyl acetate | n-Heptane | Normal heptane |
| IPAc | Isopropyl acetate | DMSO | Dimethyl sulfoxide |
| MTBE | Methyl *tert*-butyl ether | DMF | *N,N*-Dimethylformamide |
| THF | Tetrahydrofuran | $H_2O$ | Water |
| 2-MeTHF | 2-Methyltetrahydrofuran | n-Pentane | Normal pentane |
| CPME | Cyclopentyl methyl ether | Xylene | Dimethylbenzene |
| Cyclohexane | Cyclohexane | MEK | 2-Butanone |
| Anisole | Phenyl methyl ether | Cumene | Isopropylbenzene |
| *n*-Hexane | Normal hexane | Ether | Diethyl ether |
| Chlorobenzene | Phenyl chloride | NMP | *N*-Methylpyrrolidone |
| *n*-Propanol | 1-Propanol | -- | -- |

**Preparation of Compound of Formula I**

The synthetic route is as follows:

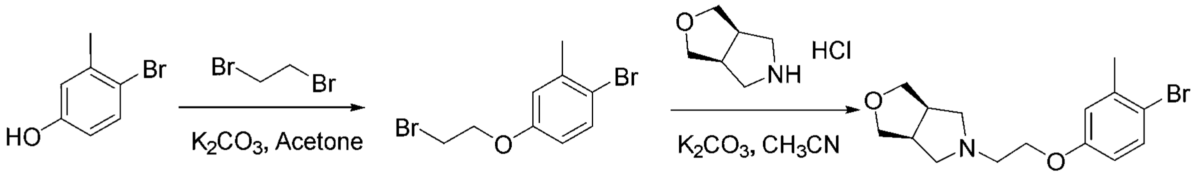


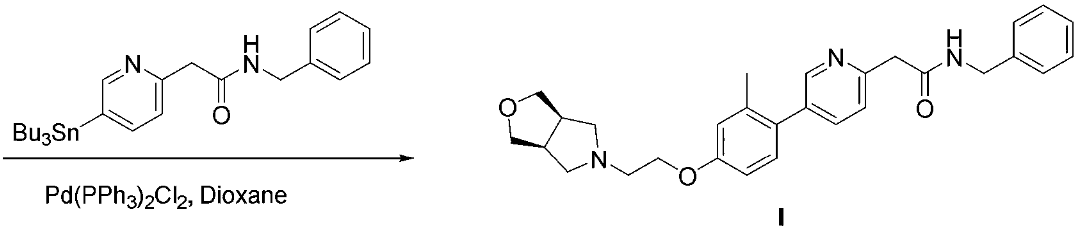


I

Step 1: Synthesis of 1-bromo-4-(2-bromoethoxy)-2-methylbenzene

4-Bromo-3-methylphenol (1 g, 5.37 mmol), 1,2-dibromoethane (5.99 g, 32.2 mmol), and potassium carbonate (4.45 g, 32.2 mmol) were dissolved in acetone (30 mL), and the reaction mixture was heated to 80°C, refluxed, and reacted for 18 hours. The reaction mixture was cooled to room temperature, filtered, the filtrate was concentrated, and the residue was separated by column chromatography (petroleum ether: ethyl acetate (V/V) = 30:1) to obtain 1-bromo-4-(2-bromoethoxy)-2-methylbenzene (1.3 g, colorless oil, yield: 82.8%).

Step 2: (3a*R*,6a*S*)-5-(2-(4-bromo-3-methylphenoxy)ethyl)hexahydro-1*H*-furo[3,4-*c*]pyrrole

1-Bromo-4-(2-bromoethoxy)-2-methylbenzene (1.3 g, 4.45 mmol) was dissolved in dry acetonitrile (30 mL), then (3a*R*,6a*S*)-hexahydro-1*H*-furo[3,4-*c*]pyrrole hydrochloride (730 mg, 4.90 mmol) and potassium carbonate (1.844 g, 13.36 mmol) were added thereto, and the reaction mixture was heated to 80°C and reacted for 20 hours. The reaction mixture was cooled to room temperature, filtered, the filtrate was concentrated, and the residue was purified by silica gel column chromatography (petroleum ether: ethyl acetate (V/V) = 1:1) to obtain (3a*R*,6a*S*)-5-(2-(4-bromo-3-methylphenoxy)ethyl) hexahydro-1*H*-furo[3,4-*c*]pyrrole (1.0 g, yellow oil, yield: 69.1%).

LC-MS, M/Z (ESI): 326.0 $[M+H]^+$.

Step 3: *N*-benzyl-2-(5-(2-methyl-4-(2-((3a*R*,6a*S*)-tetrahydro-1*H*-furo[3,4-*c*]pyrrol-5(3*H*)-yl)ethoxy)phenyl)pyridin-2-yl) acetamide (I)

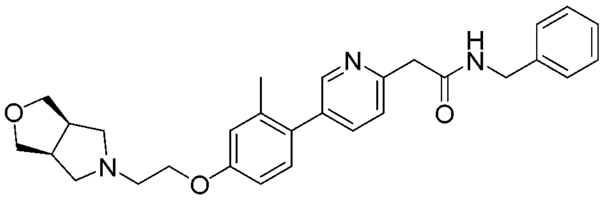

I

(3a*R*,6a*S*)-5-(2-(4-Bromo-3-methylphenoxy)ethyl)hexahydro-1*H*-furo[3,4-*c*]pyrrole (150 mg, 0.460 mmol) was dissolved in dry 1,4-dioxane (5 mL), then *N*-benzyl-2-(5-(tributylstannyl)pyridin-2-yl)acetamide (355 mg, 0.690 mmol) and bis(triphenylphosphine)palladium(II) dichloride (32.3 mg, 0.046 mmol) were added thereto. The reaction system was replaced with argon three times, and heated to 100°C and reacted for 4 hours under an argon atmosphere. The reaction mixture was cooled to room temperature, diluted with water (50 mL), and extracted with dichloromethane (50 mL × 3). The organic phases were combined, dried over anhydrous sodium sulfate, concentrated, and the residue was separated by a silica gel plate (ethyl acetate: methanol (V/V) = 20:1, $NH_3$ $H_2O$) to obtain N-benzyl-2-(5-(2-methyl-4-(2-((3a*R*,6a*S*)-tetrahydro-1*H*-furo[3,4-*c*]pyrrol-5(3*H*)-yl)ethoxy)phenyl)pyridin-2-yl)acetamide (compound of Formula I) (20 mg, yield: 9.22%), which was an amorphous solid of the compound of Formula I.

$^1H$ NMR (400 MHz, DMSO-d6): δ 8.62 (t, 1H), 8.41 (d, 1H), 7.70-6.83 (m, 10H), 4.30 (d, 2H), 4.07 (t, 2H), 3.78-3.62 (m, 4H), 3.39-3.37 (m, 2H), 2.75-2.61 (m, 6H), 2.39-2.37 (m, 2H), 2.21 (s, 3H).

LC-MS, M/Z (ESI): 472.2 $[M+H]^+$.

Example 1 Free form crystal form A

Preparation of free form crystal form A:

The compound of Formula I was stirred and dissolved in a THF solution at room temperature, filtered after dissolution, and slowly evaporated at room temperature to obtain free form crystal form A. The solid was dried under vacuum at room temperature and then characterized by XRPD and TGA/DSC.

The XRPD and TGA/DSC characterization results of the free form crystal form A sample are shown in FIGS. 1-3, and the XRPD diffraction peak data of the free form crystal form A are listed in Table 22. The TGA results show that the sample had a weight loss of 0.98% when heated to 150°C. The DSC results show that the sample had an endothermic peak at 96.6°C, with an onset temperature of 94.7°C. The TGA weight loss of the free form crystal form A is low, the DSC shows only a single melting signal, and the free form crystal form A is an anhydrous crystal form. $^1$H NMR was collected using $CD_3OD$ as the solvent, and the specific data are shown below.

$^1$H NMR (400 MHz, $CD_3OD$): δ 8.40 (1H, d), 7.74 (1H, dd), 7.44 (1H, d), 7.33-7.28 (4H, m), 7.26-7.23 (1H, m), 7.15 (1H, d), 6.91 (1H, d), 6.86 (1H, dd), 4.42 (2H, s), 4.15 (2H, t), 3.82 (2H, s), 3.71-3.64 (4H, m), 3.02 (2H, t), 2.86 (4H, t), 2.33 (2H, dd), 2.25 (3H, s).

Table 22 XRPD diffraction peak data of free form crystal form A

| Position [°2θ] | Relative intensity [%] | Position [°2θ] | Relative intensity [%] |
|---|---|---|---|
| 3.6586 | 7.14 | 22.9597 | 10.42 |
| 7.2523 | 15.19 | 23.3865 | 7.72 |
| 9.2043 | 13.87 | 23.9035 | 3.75 |
| 11.1262 | 7.27 | 25.7372 | 8.71 |
| 12.3913 | 13.21 | 26.2579 | 15.26 |
| 13.7658 | 13.84 | 26.6575 | 2.24 |
| 14.4926 | 31.62 | 27.2477 | 4.46 |
| 17.0216 | 38.40 | 27.6853 | 3.21 |
| 17.7818 | 24.47 | 28.1122 | 2.01 |
| 18.4187 | 100.00 | 28.5449 | 1.68 |
| 19.2078 | 6.70 | 29.1385 | 1.69 |
| 20.1286 | 12.32 | 31.0525 | 1.80 |
| 20.5296 | 39.57 | 34.1923 | 2.83 |
| 20.9229 | 15.31 | 35.5215 | 2.32 |
| 21.7756 | 6.40 | 37.2727 | 1.21 |
| 22.2614 | 4.58 | | |

Example 2 Free form crystal form B

The free form crystal form B sample was obtained by dissolving the compound of Formula I in MeOH, removing the solvent by rotary evaporation under reduced pressure, and then transferring the resulting gel-like sample for vacuum drying at 50°C. The XRPD and TGA/DSC characterization results are shown in FIGS. 4-6, and the XRPD diffraction peak data of the free form crystal form B are shown in Table 23. The TGA results show that the sample had a weight loss of 0.48% when heated to 150°C, and the DSC results show that the sample had two endothermic peaks at 84.5°C and 92.4°C (peak temperatures). $^l$H NMR results are shown below, and it can be observed from the NMR data that no MeOH residue was detected in the crystal form B sample. The TGA weight loss of the free form crystal form B is low, and the free form crystal form B is an anhydrous crystal form. The free form crystal form B was heated to 86°C, cooled to room temperature, and exposed to air, and the sample was converted to the free form crystal form A.

$^1$H NMR (400 MHz, $CD_3OD$): δ 8.66 (1H, t), 8.44 (1H, d), 7.71 (1H, d), 7.41-7.14 (7H, m), 6.92 (1H, s), 6.87 (1H, d),

4.32 (2H, d), 4.09 (2H, t), 3.73 (4H, m), 3.41-3.39 (2H, m), 2.77-2.63 (6H, m), 2.39 (2H, m), 2.23 (3H, s).

Table 23 XRPD diffraction peak data of free form crystal B

| Position [°2θ] | Relative intensity [%] | Position [°2θ] | Relative intensity [%] |
|---|---|---|---|
| 7.4873 | 3.10 | 22.6576 | 61.87 |
| 8.8864 | 9.12 | 23.9201 | 7.02 |
| 10.9912 | 18.04 | 24.7970 | 12.05 |
| 13.8121 | 14.37 | 25.4143 | 15.92 |
| 14.9693 | 6.34 | 26.6488 | 16.69 |
| 16.1011 | 36.37 | 27.7637 | 6.02 |
| 16.5486 | 39.23 | 28.3501 | 3.63 |
| 16.9933 | 5.23 | 29.5259 | 2.44 |
| 17.7740 | 100.00 | 30.7717 | 5.46 |
| 18.6972 | 5.30 | 31.9792 | 2.29 |
| 19.6520 | 31.67 | 33.3167 | 3.42 |
| 20.3614 | 65.65 | 37.7157 | 3.02 |
| 21.2371 | 58.26 | 39.0657 | 1.38 |
| 22.0673 | 19.39 | | |

Example 3 Study on the interconversion relationship between free form crystal forms

To further investigate the interconversion relationship between free form crystal form A and free form crystal form B, suspension competition tests between free form the crystal form A and the free form crystal form B were conducted in CPME and IPAc systems at room temperature and 50°C.

The specific steps were as follows:

1) Saturated solutions of the free form crystal form A sample in different solvent systems were prepared at corresponding temperatures; 2) Equal masses of the free form crystal form A and free form crystal form B samples were added to the saturated solutions to form suspensions; 3) Magnetic stirring was performed at room temperature; 4) After stirring for 2 days, the solids were separated and tested by XRPD. The results are shown in Table 24. The free form crystal form A was obtained in all tests. The experiments indicated that, compared with the free form crystal form B, the free form crystal form A was a thermodynamically more stable crystal form at room temperature and 50°C.

Table 24 Suspension competition test results between free form crystal forms A and B

| Starting sample | Solvent (v:v) | Temperature | Result |
|---|---|---|---|
| Free form crystal form A+B | CPME | RT | Free form crystal form A |
| | | 50°C | Free form crystal form A |
| | IPAc | RT | Free form crystal form A |
| | | 50°C | Free form crystal form A |

Example 4

Crystal form A of phosphate salt

300 mg of the compound of Formula I and 73.5 mg of phosphoric acid were slurried in 4 mL of IPA/$H_2O$ (19:1, v/v) solution at room temperature for 2 days to obtain the crystal form A of the phosphate salt. The XRPD of the crystal form A sample of the phosphate salt is shown in FIG. 7, and the XRPD data are shown in Table 25 below. The TGA/DSC results are shown in FIG. 8 and FIG. 9. The TGA results show that the sample had a weight loss of 4.40% when heated to 150°C; and the DSC results show that the sample had two endothermic peaks at 103.6°C and 174.5°C (peak temperature). $^1$H NMR results are shown below. The results show that the molar ratio of IPA to the compound of Formula I in the sample is 0.04

(about 0.4 wt%). The HPLC/IC results show that the molar ratio of phosphoric acid to the compound of Formula I is 1:1.

The crystal form A of the phosphate salt was identified by VT-XRPD, and the results showed (FIG. 10): after purging the crystal form A of the phosphate salt under $N_2$ for 20 min, the crystal form remained unchanged; after heating to 150°C under a nitrogen atmosphere, cooling to 30°C, and then exposing to air, conversion to free form crystal form B was observed. Combined with the TGA/DSC and $^1$H NMR results of the crystal form A of the phosphate salt, the crystal form A of the phosphate salt was a hydrate, which dehydrated under heating conditions and converted to anhydrous crystal form B of the phosphate salt. The XRPD pattern of the crystal form B of the phosphate salt is shown in FIG. 11, and the XRPD data are shown in Table 26 below.

$^1$H NMR (400 MHz, DMSO-d6): δ 8.67 (1H, t), 8.43 (1H, d), 7.71 (1H, dd), 7.41-7.15 (7H, m), 6.93 (1H, d), 6.87 (1H, dd), 4.31 (2H, d), 4.12 (2H, t), 3.72-3.66 (4H, m), 3.44 (2H, m), 2.86-2.75 (6H, m), 2.23 (3H, s).

Table 25 XRPD diffraction peak data of crystal form A of the phosphate salt

| Position [°2θ] | Relative intensity [%] | Position [°2θ] | Relative intensity [%] |
|---|---|---|---|
| 3.3971 | 3.92 | 20.7346 | 11.38 |
| 6.7420 | 64.07 | 21.0112 | 17.83 |
| 7.4735 | 11.63 | 22.1650 | 19.60 |
| 7.8430 | 6.09 | 22.4611 | 18.76 |
| 11.8665 | 6.89 | 22.6861 | 21.05 |
| 12.5613 | 11.06 | 23.3038 | 14.49 |
| 13.4735 | 48.13 | 23.6997 | 10.47 |
| 13.7472 | 23.38 | 24.2381 | 14.86 |
| 14.9424 | 15.60 | 24.6170 | 6.42 |
| 15.6909 | 48.98 | 25.2605 | 18.00 |
| 16.5665 | 19.30 | 26.0498 | 4.76 |
| 16.8571 | 100.00 | 27.1236 | 3.12 |
| 17.2241 | 19.19 | 27.6743 | 7.67 |
| 18.0887 | 33.50 | 28.2245 | 2.87 |
| 18.6053 | 18.77 | 29.6701 | 5.48 |
| 19.2940 | 2.78 | 30.6420 | 4.94 |
| 20.2570 | 23.97 | | |

.

Table 26 XRPD diffraction peak data of crystal form B of the phosphate salt

| Position [°2θ] | Relative intensity [%] | Position [°2θ] | Relative intensity [%] |
|---|---|---|---|
| 3.3488 | 100.00 | 21.4946 | 10.39 |
| 6.6843 | 21.93 | 21.7492 | 10.41 |
| 7.6346 | 4.49 | 22.5344 | 11.77 |
| 10.0305 | 3.43 | 23.0287 | 5.88 |
| 12.1675 | 7.90 | 23.2345 | 5.21 |
| 13.3734 | 45.72 | 23.9394 | 3.17 |
| 13.7627 | 11.36 | 24.4279 | 12.17 |
| 14.1560 | 9.48 | 25.2438 | 15.76 |
| 14.5261 | 7.21 | 25.6966 | 5.56 |
| 15.0409 | 11.28 | 27.0403 | 1.05 |

(continued)

| Position [°2θ] | Relative intensity [%] | Position [°2θ] | Relative intensity [%] |
|---|---|---|---|
| 15.3041 | 34.26 | 27.8899 | 2.69 |
| 16.7414 | 68.50 | 28.7762 | 1.94 |
| 17.2869 | 7.43 | 29.6849 | 3.25 |
| 17.4813 | 8.70 | 32.1272 | 1.17 |
| 17.7801 | 12.25 | 32.7569 | 1.84 |
| 18.1056 | 17.31 | 33.5155 | 1.11 |
| 18.8004 | 4.56 | 34.5572 | 1.79 |
| 19.6890 | 4.68 | 35.9222 | 0.83 |
| 20.1204 | 23.28 | 36.8640 | 1.30 |
| 21.2684 | 20.03 | | |

Crystal form A of fumarate salt

300 mg of the compound of Formula I and 73.5 mg of fumaric acid were slurried in 6 mL of MTBE solution at room temperature for 2 days to obtain the crystal form A of the fumarate salt. The XRPD of the crystal form A sample of the fumarate salt is shown in FIG. 12. The TGA/DSC results are shown in FIG. 13 and FIG. 14. The TGA results show that the sample had a weight loss of 1.17% when heated to 150°C; and the DSC results show that the sample had an endothermic peak at 130.3°C with an onset temperature of 128.2°C. $^1$H NMR results are shown below. The results show that the molar ratio of fumaric acid to the compound of Formula I in the sample is 1:1, and the molar ratio of residual solvent MTBE to the compound of Formula I is 0.01 (about 0.1 wt%).

The crystal form A of the fumarate salt was studied by variable humidity XRPD (VH-XRPD), and the results (FIG. 15) showed that after the crystal form A of the fumarate salt was placed at 85% RH, a shift in diffraction peaks was observed; after the sample was returned to 30% RH, the sample converted back to the crystal form A of the fumarate salt. Combined with the DVS test results of crystal form A of the fumarate salt (as shown in FIG. 52), the fumarate salt sample shows a significant weight gain under high-humidity conditions (>70% RH), suggesting that the fumarate salt exists in a hydrated state under high humidity. Under low-humidity conditions (*e.g.*, 30% RH), the sample reverts to the anhydrous crystal form A of the fumarate salt.

$^1$H NMR (400 MHz, DMSO-*d6*): δ 8.67 (1H, t), 8.44 (1H, d), 7.71 (1H, dd), 7.41-7.15 (7H, m), 6.93 (1H, s), 6.87 (1H, dd), 6.61 (2H, s), 4.32 (2H, d), 4.11 (2H, t), 3.73-3.68 (4H, m), 3.43 (2H, m), 2.83-2.73 (6H, m), 2.44 (2H, d), 2.23 (3H, s).

Table 27 XRPD diffraction peak data of crystal form A of the fumarate salt

| Position [°2θ] | Relative intensity [%] | Position [°2θ] | Relative intensity [%] |
|---|---|---|---|
| 9.0262 | 10.80 | 24.2390 | 26.28 |
| 9.7784 | 29.03 | 25.7165 | 23.45 |
| 13.5143 | 53.68 | 27.1984 | 7.74 |
| 15.4104 | 56.59 | 28.5027 | 2.01 |
| 16.8922 | 29.24 | 29.5519 | 5.77 |
| 17.5651 | 99.14 | 30.2545 | 6.02 |
| 18.3356 | 27.28 | 30.6842 | 8.40 |
| 18.6617 | 21.63 | 31.3364 | 4.48 |
| 19.4757 | 100.00 | 32.3634 | 2.45 |
| 20.1090 | 52.33 | 33.8625 | 1.35 |
| 21.1154 | 12.41 | 35.4107 | 3.44 |
| 22.0379 | 34.40 | 36.5349 | 3.18 |

(continued)

| Position [°2θ] | Relative intensity [%] | Position [°2θ] | Relative intensity [%] |
|---|---|---|---|
| 23.0333 | 7.97 | 38.9309 | 1.92 |
| 23.7137 | 46.33 | | |

Crystal form A of *p*-toluenesulfonate salt

300 mg of the compound of Formula I and 121.5 mg of p-toluenesulfonic acid monohydrate were slurried in 6 mL of IPAc solution at room temperature for 2 days to obtain the crystal form A of *p*-toluenesulfonate salt. The XRPD of the crystal form A sample of the*p*-toluenesulfonate salt is shown in FIG. 16. The TGA/DSC results are shown in FIG. 17 and FIG. 18. The TGA results show that the sample had a weight loss of 0.94% when heated to 150°C; and the DSC results show that the sample had an endothermic peak at 152.1°C with an onset temperature of 149.9°C. $^{1}$H NMR results are shown below. The results show that the molar ratio of *p*-toluenesulfonic acid to the compound of Formula I in the sample is 1:1, and the molar ratio of residual solvent IPAc to the compound of Formula I is 0.01 (about 0.1 wt%).

$^{1}$H NMR (400 MHz, DMSO-d6): δ 9.79 (1H, s), 8.69 (1H, t), 8.44 (1H, d), 7.73 (1H, dd), 7.47 (2H, d), 7.43 (1H, d), 7.35-7.21 (6H, m), 7.12 (2H, d), 7.01-6.94 (2H, m), 4.36-4.29 (4H, m), 3.93 (1H, s), 3.81-3.45 (8H, m), 3.13-2.80 (3H, m), 2.29 (3H, s), 2.26 (3H, s).

Table 28 XRPD diffraction peak data of crystal form A of the *p*-toluenesulfonate salt

| Position [°2θ] | Relative intensity [%] | Position [°2θ] | Relative intensity [%] |
|---|---|---|---|
| 5.4061 | 50.32 | 19.9778 | 61.40 |
| 7.7375 | 20.44 | 21.0061 | 12.71 |
| 8.1037 | 40.15 | 21.8329 | 62.62 |
| 10.8067 | 22.25 | 22.8534 | 30.96 |
| 13.1118 | 2.94 | 23.4116 | 26.46 |
| 14.7415 | 61.45 | 24.3701 | 39.55 |
| 15.2264 | 7.77 | 26.1763 | 17.86 |
| 16.2076 | 5.59 | 27.2507 | 6.34 |
| 17.1253 | 54.33 | 28.5791 | 19.45 |
| 18.1038 | 100.00 | 30.4321 | 3.98 |
| 18.3589 | 38.45 | 31.2458 | 6.35 |
| 18.8288 | 80.63 | | |

Crystal form A of benzenesulfonate salt

300 mg of the compound of Formula I and 100.5 mg of benzenesulfonic acid were slurried in 6 mL of IPAc solution at room temperature for 2 days to obtain the crystal form A of benzenesulfonate salt. The XRPD pattern of the crystal form A sample of the benzenesulfonate salt is shown in FIG. 19. The TGA/DSC results are shown in FIG. 20 and FIG. 21. The TGA results show that the sample had a weight loss of 1.28% when heated to 140°C; and the DSC results show that the sample had an endothermic peak at 146.4°C with an onset temperature of 144.5°C. $^{1}$H NMR was measured in DMSO-$d_6$, and the results are as follows. The results show that the molar ratio of benzenesulfonic acid to the compound of Formula I in the sample is 1:1, and the molar ratio of residual solvent IPAc to the compound of Formula I is 0.01 (about 0.1 wt%).

$^{1}$H NMR (400 MHz, DMSO-d6): δ 9.79 (1H, s), 8.68 (1H, t), 8.44 (1H, d), 7.73 (1H, dd), 7.61-7.58 (2H, m), 7.43 (1H, d), 7.35-7.21 (9H, m), 7.01-6.94 (2H, m), 4.32 (4H, m), 3.93 (1H, s), 3.80-3.46 (8H, m), 3.13-2.81 (3H, m), 2.25 (3H, s).

Table 29 XRPD diffraction peak data of crystal form A of the benzenesulfonate salt

| Position [°2θ] | Relative intensity [%] | Position [°2θ] | Relative intensity [%] |
|---|---|---|---|
| 4.2927 | 90.89 | 20.2214 | 43.49 |
| 5.7545 | 6.24 | 20.5266 | 100.00 |

(continued)

| Position [°2θ] | Relative intensity [%] | Position [°2θ] | Relative intensity [%] |
|---|---|---|---|
| 8.4936 | 76.94 | 20.9947 | 73.78 |
| 10.0153 | 39.96 | 21.2345 | 28.85 |
| 10.6755 | 22.03 | 21.4984 | 24.65 |
| 11.1754 | 8.55 | 21.9486 | 45.89 |
| 11.4806 | 9.68 | 22.3819 | 23.59 |
| 12.7029 | 7.82 | 22.6234 | 14.90 |
| 13.8134 | 41.83 | 23.4571 | 10.81 |
| 14.4981 | 11.11 | 23.8950 | 42.58 |
| 15.2579 | 54.96 | 25.5219 | 30.50 |
| 16.8048 | 46.57 | 25.7195 | 28.44 |
| 16.9835 | 26.02 | 27.6008 | 3.86 |
| 17.8361 | 20.18 | 28.7734 | 9.90 |
| 18.4629 | 22.00 | 29.2011 | 12.35 |
| 18.7971 | 56.58 | 30.3950 | 5.38 |
| 19.1714 | 26.12 | 31.7722 | 4.14 |
| 19.7264 | 74.71 | 33.1659 | 6.58 |

Crystal form B of oxalate salt

300 mg of the compound of Formula I and 79.5 mg of oxalic acid dihydrate were slurried in 12 mL of acetone/*n*-heptane (1:1, v/v) solution at room temperature for 7 days, and then slurried at 55°C for another 1 day to obtain the crystal form B of the oxalate salt. The XRPD pattern of the crystal form B sample of the oxalate salt is shown in FIG. 22. The TGA/DSC results are shown in FIG. 23 and FIG. 24. The TGA results show that the sample had a weight loss of 1.21% when heated to 150°C; and the DSC results show that the sample had two endothermic peaks at 158.2°C and 218.7°C (peak temperature). $^1H$ NMR results are as follows, and the results show that the molar ratio of residual solvent acetone to the compound of Formula I is 0.01 (about 0.1 wt%). The HPLC/IC results show that the molar ratio of oxalic acid to the compound of Formula I is 1:1.

$^1H$ NMR (400 MHz, DMSO-*d6*): δ 8.68 (1H, t), 8.44 (1H, d), 7.72 (1H, dd), 7.41 (1H, d), 7.35-7.19 (6H, m), 6.98-6.91 (2H, m), 4.32-4.25 (4H, m), 3.74-3.34 (10H, m), 2.94-2.85 (4H, m), 2.25 (3H, s).

Table 30 XRPD diffraction peak data of crystal form B of the oxalate salt

| Position [°2θ] | Relative intensity [%] | Position [°2θ] | Relative intensity [%] |
|---|---|---|---|
| 7.6303 | 39.67 | 20.8286 | 39.95 |
| 8.3152 | 56.34 | 21.2807 | 28.72 |
| 12.4726 | 31.39 | 22.0493 | 70.88 |
| 15.2505 | 70.07 | 23.8443 | 93.78 |
| 16.2944 | 100.00 | 24.5635 | 18.67 |
| 17.6634 | 71.80 | 25.0476 | 23.37 |
| 18.2646 | 82.23 | 26.4010 | 16.45 |
| 18.4892 | 59.67 | 27.4132 | 20.10 |
| 18.7712 | 90.80 | 28.3635 | 5.97 |
| 19.1571 | 67.01 | 29.2511 | 13.78 |
| 19.9191 | 16.34 | | |

Crystal form A of maleate salt

The crystal form A of the maleate salt was obtained by slurrying the compound of Formula I (828906-01-A) with an equimolar amount of maleic acid in an acetone/n-heptane (1:1, v/v) solution for 3 days, followed by isolation of the solid sample and vacuum drying. The XRPD and TGA/DSC results of the crystal form A sample of the maleate salt are listed in FIGS. 25-27. The TGA results show that the sample had a weight loss of 2.56% when heated to 120°C, and the DSC results show that the sample had three endothermic peaks at 54.7°C, 125.2°C, and 200.7°C (peak temperature). $^1$H NMR results are shown below. In the crystal form A of the maleate salt, the molar ratio of maleic acid to the compound of Formula I is 1.0, the molar ratio of residual solvent acetone to the compound of Formula I is 0.03 (about 0.3 wt%), and no n-heptane solvent residue is observed.

$^1$H NMR (400 MHz, DMSO-d6): δ 8.67 (1H, t), 8.44 (1H, d), 7.71 (1H, dd), 7.41-7.20 (7H, m), 6.99-6.93 (2H, m), 6.04 (2H, s), 4.33-4.28 (4H, m), 3.73 (3H, s), 2.98-2.80 (3H, m), 2.25 (3H, s).

Table 31 XRPD diffraction peak data of crystal form A of the maleate salt

| Position [°2θ] | Relative intensity [%] | Position [°2θ] | Relative intensity [%] |
|---|---|---|---|
| 3.4417 | 16.32 | 21.6390 | 7.94 |
| 9.6432 | 27.14 | 22.3570 | 27.06 |
| 10.1448 | 5.95 | 22.7123 | 28.31 |
| 13.5352 | 100.00 | 23.7674 | 95.50 |
| 15.7013 | 7.54 | 24.4456 | 6.76 |
| 16.9220 | 49.89 | 24.8503 | 8.90 |
| 17.6360 | 60.94 | 25.5895 | 12.79 |
| 18.0813 | 36.58 | 26.7126 | 5.99 |
| 18.5713 | 13.66 | 27.2013 | 26.88 |
| 18.9912 | 11.46 | 27.7331 | 8.29 |
| 19.6099 | 51.55 | 28.8950 | 13.37 |
| 20.8195 | 31.63 | 30.6662 | 21.93 |

Crystal form A of malate salt

The crystal form A of the malate salt was obtained by slurrying the compound of Formula I with an equimolar amount of malic acid in an acetone/*n*-heptane (1:1, v/v) solution for 6 days, followed by isolation of the solid sample and vacuum drying. The sample was subjected to powder diffraction, TGA, and DSC experiments. The resulting XRPD is shown in FIG. 28, and the TGA/DSC results are listed in FIGS. 29 and 30. The TGA results show that the sample had a weight loss of 2.26% when heated to 150°C. The DSC results show that the sample had three endothermic peaks at 44.6°C, 96.3°C, and 215.8°C (peak temperature). $^1$H NMR results are shown below. In the crystal form A sample of the malate salt, the molar ratio of malic acid to the compound of Formula I is 1:1, the molar ratio of residual solvent acetone to the compound of Formula I is 0.01 (about 0.1 wt%), and the molar ratio of n-heptane to the compound of Formula I is 0.02 (about 0.3 wt%).

$^1$H NMR (400 MHz, $CD_3OD$): δ 8.40 (1H, d), 7.74 (1H, dd), 7.47 (1H, d), 7.31-7.29 (4H, m), 7.27-7.22 (1H, m), 7.19 (1H, d), 6.97 (1H, d), 6.93 (1H, dd), 4.42 (2H, s), 4.34-4.31 (3H, m), 3.81 (3H, t), 3.67-3.62 (4H, m), 3.44 (2H, t), 3.09 (2H, s), 2.91 (2H, dd), 2.77 (1H, dd), 2.53 (1H, q), 2.26 (3H, s).

Table 32 XRPD diffraction peak data of crystal form A of the malate salt

| Position [°2θ] | Relative intensity [%] | Position [°2θ] | Relative intensity [%] |
|---|---|---|---|
| 6.6262 | 7.38 | 19.7373 | 21.21 |
| 8.9534 | 20.94 | 21.6214 | 25.16 |
| 9.8936 | 38.20 | 23.1988 | 17.64 |
| 13.2054 | 50.38 | 23.8001 | 22.22 |

(continued)

| Position [°2θ] | Relative intensity [%] | Position [°2θ] | Relative intensity [%] |
|---|---|---|---|
| 16.1763 | 29.75 | 24.7654 | 32.77 |
| 16.5259 | 50.45 | 27.2491 | 13.36 |
| 17.8951 | 33.19 | 31.4661 | 5.26 |
| 18.9290 | 100.00 | | |

Crystal form A of succinate salt

The crystal form A of the succinate salt was obtained by slurrying the compound of Formula I with an equimolar amount of succinic acid in an MTBE solution for 6 days, followed by isolation of the solid sample and vacuum drying. The XRPD and TGA/DSC results of the crystal form A of the succinate salt are shown in FIGS. 31-33. The TGA results show that the sample had a weight loss of 4.49% when heated to 150°C. The DSC results show that the sample had two endothermic peaks at 57.3°C and 85.4°C (peak temperatures). $^1$H NMR results are shown below. In the crystal form A of the succinate salt, the molar ratio of succinic acid to the compound of Formula I is 1.2:1, and no residual MTBE solvent is observed.

$^1$H NMR (400 MHz, $CD_3OD$): δ 8.40 (1H, d), 7.74 (1H, dd), 7.47 (1H, d), 7.32-7.31 (4H, m), 7.27-7.22 (1H, m), 7.16 (1H, d), 6.95 (1H, d), 6.91 (1H, dd), 4.43 (2H, s), 4.25 (2H, t), 3.83 (2H, d), 3.76 (2H, dd), 3.69-3.65 (2H, m), 3.41-3.37 (2H, m), 3.21 (2H, t), 3.00 (2H, s), 2.69 (2H, dd), 2.55 (5H, s), 2.26 (3H, s).

Table 33 XRPD diffraction peak data of crystal form A of the succinate salt

| Position [°2θ] | Relative intensity [%] | Position [°2θ] | Relative intensity [%] |
|---|---|---|---|
| 6.9349 | 100.00 | 19.4760 | 19.47 |
| 9.1969 | 38.52 | 20.2096 | 35.96 |
| 11.7777 | 6.58 | 20.9648 | 45.17 |
| 13.8697 | 56.93 | 22.1780 | 31.28 |
| 14.6969 | 18.62 | 23.0453 | 19.19 |
| 16.2949 | 72.71 | 23.9225 | 28.52 |
| 17.3959 | 14.28 | 26.1847 | 32.80 |
| 17.9354 | 39.73 | 28.0540 | 5.21 |
| 18.3860 | 12.73 | 34.8694 | 3.06 |

Crystal form A/B of methanesulfonate salt

The crystal form A of the methanesulfonate salt was obtained by slurrying the compound of Formula I with methanesulfonic acid at a molar ratio of 1:1 in an acetone/*n*-heptane (1:1, v/v) solution for 3 days, followed by isolation of the gel-like solid sample and vacuum drying. The crystal form B of the methanesulfonate salt was obtained by slurrying the compound of Formula I with methanesulfonic acid at a molar ratio of 1:2 in an IPAc solution for 3 days, followed by isolation of the solid sample and vacuum drying. The XRPD patterns of the crystal forms A and B of the methanesulfonate salt are shown in FIG. 34 and FIG. 35, respectively.

The TGA/DSC results of the crystal form B of the methanesulfonate salt are detailed in FIG. 36 and FIG. 37. The TGA results show that the sample had a weight loss of 0.91% when heated to 80°C and a weight loss of 2.40% when heated from 80°C to 130°C; and the DSC results show that the sample had one endothermic peak at 109.4°C (peak temperature). $^1$H NMR results are shown below. In the crystal form B of the methanesulfonate salt, the molar ratio of methanesulfonic acid to the compound of Formula I is 2.1, and the molar ratio of residual solvent IPAc to the compound of Formula I is 0.04 (about 0.7 wt%).

The crystal form B of the methanesulfonate salt: $^1$H NMR (400 MHz, $CD_3OD$): δ 8.80 (1H, s), 8.52 (1H, d), 8.01 (1H, dd), 7.36-7.33 (5H, m), 7.30-7.27 (1H, m), 7.09-7.01 (2H, m), 4.45 (2H, s), 4.41-4.40 (3H, m), 4.17-4.15 (2H, m), 4.05 (2H, t), 3.90 (2H, d), 3.71 (2H, s), 3.65-3.57 (5H, m), 3.16 (2H, s), 2.93 (2H, t), 2.69 (6H, d), 2.35 (3H, s).

Table 34 XRPD diffraction peak data of crystal form A of the methanesulfonate salt

| Position [°2θ] | Relative intensity [%] | Position [°2θ] | Relative intensity [%] |
|---|---|---|---|
| 5.0258 | 56.54 | 20.5965 | 100.00 |
| 17.0335 | 38.98 | 23.3964 | 71.59 |
| 18.1100 | 61.40 | | |

Table 35 XRPD diffraction peak data of crystal form B of the methanesulfonate salt

| Position [°2θ] | Relative intensity [%] | Position [°2θ] | Relative intensity [%] |
|---|---|---|---|
| 9.8298 | 9.76 | 22.6933 | 23.52 |
| 11.3186 | 15.66 | 23.4414 | 73.53 |
| 11.8183 | 5.19 | 23.9398 | 15.74 |
| 12.6882 | 3.11 | 24.4552 | 60.92 |
| 13.7455 | 5.81 | 25.3114 | 10.00 |
| 14.3775 | 6.16 | 25.6224 | 15.39 |
| 15.4572 | 22.47 | 25.8897 | 14.19 |
| 16.5409 | 6.51 | 27.0121 | 4.85 |
| 16.9751 | 29.83 | 27.4334 | 6.22 |
| 17.2607 | 52.29 | 27.9938 | 6.98 |
| 17.6280 | 39.70 | 28.5662 | 8.75 |
| 18.4451 | 35.15 | 29.3314 | 8.75 |
| 18.8911 | 100.00 | 29.7831 | 9.39 |
| 19.6841 | 30.75 | 30.3249 | 5.40 |
| 20.4301 | 65.78 | 31.3892 | 5.83 |
| 21.0733 | 65.86 | 32.6597 | 5.59 |
| 21.5827 | 20.17 | 35.2268 | 6.84 |
| 22.0878 | 29.37 | 37.3650 | 3.62 |

Crystal form A of oxalate salt

The crystal form A of the oxalate salt was obtained by slurrying the compound of Formula I with an equimolar amount of oxalic acid in an IPA/$H_2O$ (19:1, v/v) solution for 3 days, followed by isolation of the solid sample and vacuum drying. The XRPD of the crystal form A of the oxalate salt is shown in FIG. 38.

The TGA/DSC results of the crystal form A of the oxalate salt are listed in FIG. 39 and FIG. 40. The TGA results show that the sample had a weight loss of 4.10% when heated to 150°C. The DSC results show that the sample had three endothermic peaks at 153.6°C, 162.8°C and 218.8°C (peak temperatures). $^1$H NMR results are shown below. In the crystal form A of the oxalate salt, the molar ratio of residual solvent IPA to the compound of Formula I is 0.03 (about 0.3 wt%).

The crystal form A of the oxalate salt: $^1$H NMR (400 MHz, DMSO-d6): δ8.67 (1H, t), 8.44 (1H, d), 7.72 (1H, dd), 7.41 (1H, d), 7.35-7.18 (6H, m), 6.98-6.91 (2H, m), 4.33-4.26 (4H, m), 3.74-3.35 (10H, m), 2.94-2.85 (4H, m), 2.25 (3H, s).

Table 36 XRPD diffraction peak data of crystal form A of the oxalate salt

| Position [°2θ] | Relative intensity [%] | Position [°2θ] | Relative intensity [%] |
|---|---|---|---|
| 4.6409 | 9.67 | 20.5689 | 32.57 |
| 7.3243 | 34.07 | 21.0873 | 24.03 |
| 8.4725 | 100.00 | 21.4883 | 39.50 |
| 12.9334 | 19.51 | 22.3807 | 86.67 |

(continued)

| Position [°2θ] | Relative intensity [%] | Position [°2θ] | Relative intensity [%] |
|---|---|---|---|
| 13.4699 | 12.40 | 23.2608 | 31.24 |
| 14.6183 | 86.76 | 23.5935 | 30.33 |
| 15.2278 | 7.68 | 24.0272 | 21.48 |
| 16.9988 | 89.93 | 25.9955 | 32.08 |
| 18.0487 | 48.94 | 27.5449 | 4.60 |
| 18.5824 | 84.16 | 28.6854 | 3.47 |
| 18.8319 | 39.40 | 30.1750 | 12.59 |
| 19.4386 | 62.43 | 30.7971 | 5.66 |

**Crystal form A of 2-hydroxyethanesulfonate salt**

The crystal form A of the 2-hydroxyethanesulfonate salt was obtained by slurrying the compound of Formula I with an equimolar amount of 2-hydroxyethanesulfonic acid in an acetone/*n*-heptane (1:1, v/v) solution for 3 days, followed by isolation of the solid sample and vacuum drying. The XRPD of the crystal form A sample of the 2-hydroxyethanesulfonate salt is shown in FIG. 41, and the TGA/DSC results are detailed in FIGS. 42 and 43. The TGA results show that the sample had a weight loss of 1.28% when heated to 110°C. The DSC results show that the sample had two endothermic peaks at 57.4°C and 118.4°C (peak temperature). $^1$H NMR results are shown below. In the crystal form A of the 2-hydroxyethanesulfonate salt, the molar ratio of 2-hydroxyethanesulfonic acid to the compound of Formula I is 1.2, the molar ratio of residual solvent acetone to the compound of Formula I is 0.03 (about 0.3 wt%), and the molar ratio of n-heptane to the compound of Formula I is 0.01 (about 0.2 wt%).

$^1$H NMR (400 MHz, DMSO-*d6*): δ 9.78 (1H, s), 8.67 (1H, t), 8.44 (1H, d), 7.73 (1H, dd), 7.43-7.21 (7H, m), 7.00-6.94 (2H, m), 4.37-4.30 (4H, m), 3.93 (1H, s), 3.77-3.45 (10H, m), 3.13-2.80 (3H, m), 2.60 (2H, t), 2.25 (3H, s).

Table 37 XRPD diffraction peak data of crystal form A of the 2-hydroxyethanesulfonate salt

| Position [°2θ] | Relative intensity [%] | Position [°2θ] | Relative intensity [%] |
|---|---|---|---|
| 4.8473 | 97.56 | 20.3297 | 56.13 |
| 9.8479 | 49.33 | 21.8277 | 45.05 |
| 10.9792 | 82.75 | 22.3258 | 67.31 |
| 12.8290 | 12.79 | 22.8682 | 63.84 |
| 14.2549 | 4.53 | 24.9854 | 21.98 |
| 15.5419 | 25.71 | 26.2466 | 10.34 |
| 15.9356 | 30.48 | 26.9452 | 8.37 |
| 16.6623 | 12.53 | 28.5116 | 17.43 |
| 18.0907 | 88.25 | 31.1759 | 4.85 |
| 19.3520 | 100.00 | | |

**Example 5 Performance evaluation experiment**

**Dynamic solubility experiment**

Rotating mixing was carried out at 37°C at a solid feed concentration of 10 mg/mL (calculated as the compound of Formula I). The solubility of each sample in four systems (water, SGF, FaSSIF, and FeSSIF) was determined at different time points (1, 4, and 24 hours). After sampling at each time point, the samples were centrifuged (10000 rpm) and filtered (0.45 μm PTFE filter head), and the HPLC concentration and pH value of the filtrate were measured. The solid samples after centrifugation were tested for XRPD. The solubility test results are summarized in Table 38, and the solubility curves in each system are shown in FIGS. 44-47. Compared with free form crystal form A, different salt form samples exhibited varying degrees of solubility improvement in $H_2O$, SGF, and FaSSIF. Among them, crystal form A of the fumarate salt and

crystal form B of the oxalate salt showed higher solubility in $H_2O$ and FaSSIF compared to other salt forms.

Preparation of biological solvent

Preparation of simulated gastric fluid (SGF)

100 mg of NaCl and 50 mg of Triton X-100 were weighed into a 50 mL volumetric flask, and purified water was added to complete dissolution. 816 μL of 1 M hydrochloric acid was added thereto, and the pH was adjusted to 1.8 with 1 M hydrochloric acid or 1 M NaOH solution. Purified water was added to make up to volume.

Preparation of fasted state simulated intestinal fluid (FaSSIF)

340 mg of anhydrous $NaH_2PO_4$ and 620 mg of NaCl were weighed into a 100 mL volumetric flask. Purified water was added to complete dissolution, followed by addition of 55.44 μL of 50% NaOH solution, and the pH was adjusted to 6.5 using 1M HCl or 1M NaOH solution. Purified water was added to make up to volume. 110 mg of SIF powder was then weighed into a 50 mL volumetric flask, added with the above solution to complete dissolution and make up to volume.

Preparation of fed state simulated intestinal fluid (FeSSIF)

0.82 mL of glacial acetic acid and 1.18 g of NaCl were added to a 100 mL volumetric flask. Purified water was added to complete dissolution, followed by addition of 528 μL of 50% NaOH solution, and the pH was adjusted to 5.0 using 1M HCl or 1M NaOH solution. Purified water was added to make up to volume. 560 mg of SIF powder was then weighed into a 50 mL volumetric flask, added with the above solution to complete dissolution and make up to volume.

Table 38 Summary of dynamic solubility test results at 37°C

| **Starting material** | **Solvent** | **1 hour** | | **4 hours** | | **24 hours** | |
|---|---|---|---|---|---|---|---|
| | | S | pH | S | pH | S | pH |
| Free form crystal form A | $H_2O$ | 0.06 | 8.6 | 0.08 | 8.6 | 0.06 | 8.5 |
| | SGF | 6.71 | 5.7 | 6.77 | 5.8 | 6.61 | 5.9 |
| | FaSSIF | 0.72 | 6.6 | 0.70 | 6.6 | 0.63 | 6.6 |
| | FeSSIF | 3.46 | 5.3 | 3.74 | 5.4 | 3.46 | 5.4 |
| Crystal form A of phosphate salt | $H_2O$ | 9.83 | 5.3 | 9.65 | 5.4 | 9.12 | 5.3 |
| | SGF | 9.14 | 3.4 | 9.67 | 3.4 | 9.07 | 3.4 |
| | FaSSIF | 6.63 | 5.9 | 6.32 | 5.9 | 6.14 | 5.9 |
| | FeSSIF | 5.13 | 4.9 | 4.12 | 4.9 | 3.73 | 4.9 |
| Crystal form A of fumarate salt | $H_2O$ | 9.44 | 4.0 | 9.72 | 4.0 | 9.38 | 4.0 |
| | SGF | 9.65 | 3.4 | 9.82 | 3.4 | 9.17 | 3.4 |
| | FaSSIF | 8.72 | 4.3 | 8.94 | 4.4 | 8.24 | 4.4 |
| | FeSSIF | 3.63 | 4.6 | 3.60 | 4.6 | 3.61 | 4.6 |
| Crystal form A of*p*-toluenesulfonate salt | $H_2O$ | 5.97 | 5.3 | 6.13 | 5.4 | 5.75 | 5.2 |
| | SGF | 9.41 | 3.3 | 9.46 | 3.3 | 8.97 | 3.3 |
| | FaSSIF | 2.78 | 6.0 | 2.78 | 6.1 | 2.68 | 6.1 |
| | FeSSIF | 4.12 | 4.9 | 2.51 | 4.9 | 2.30 | 4.9 |
| Crystal form A of benzenesulfonate salt | $H_2O$ | 8.29 | 5.6 | 8.39 | 5.6 | 8.12 | 5.6 |
| | SGF | 9.94 | 3.4 | 9.95 | 3.5 | 9.70 | 3.5 |
| | FaSSIF | 3.98 | 6.1 | 3.64 | 6.1 | 3.55 | 6.1 |
| | FeSSIF | 3.15 | 4.9 | 2.89 | 4.9 | 2.91 | 4.9 |

(continued)

| Starting material | Solvent | 1 hour | | 4 hours | | 24 hours | |
|---|---|---|---|---|---|---|---|
| | | S | pH | S | pH | S | pH |
| Crystal form B of oxalate salt | $H_2O$ | 9.65 | 3.8 | 9.65 | 3.8 | 9.36 | 3.9 |
| | SGF | 9.99 | 3.1 | 9.79 | 3.1 | 9.61 | 3.1 |
| | FaSSIF | 9.27 | 4.2 | 8.89 | 4.2 | 8.47 | 4.2 |
| | FeSSIF | 3.80 | 4.7 | 3.66 | 4.6 | 3.48 | 4.6 |
| S: solubility (mg/mL); --: XRPD was not tested. | | | | | | | |

**Hygroscopicity test**

The crystal form A of the phosphate salt, crystal form A of the fumarate salt, crystal form A of the *p*-toluenesulfonate salt, crystal form A of the benzenesulfonate salt, crystal form B of the oxalate salt, and free form crystal form A were evaluated for hygroscopicity by dynamic vapor sorption (DVS). The percentage change in sample mass was collected under constant temperature at 25°C as humidity varied from 0%RH to 95%RH. The DVS test results and the XRPD results of the sample before and after the DVS test are shown in FIG. 48 to FIG. 59. The results indicate that the free form crystal form A is almost non-hygroscopic, while the crystal form A of the *p*-toluenesulfonate salt, the crystal form A of the benzenesulfonate salt, and the crystal form B of the oxalate salt are slightly hygroscopic. The crystal form A of the phosphate salt is a hydrate and rapidly dehydrates below 10% RH. The crystal form A of the fumarate salt is significantly hygroscopic above 70% RH. All of the above crystal forms showed no change in crystal form after the DVS test, indicating high stability under high-humidity conditions.

**Solid-state stability**

The crystal form A of the phosphate salt, crystal form A of the fumarate salt, crystal form A of the p-toluenesulfonate salt, crystal form A of the benzenesulfonate salt, crystal form B of the oxalate salt, and free form crystal form A were placed open at 25°C/60%RH and 40°C/75%RH for 1 week, respectively, the physical and chemical stability of the samples were measured by XRPD and HPLC. The purity data are listed in Table 39, and the XRPD results are listed in FIGS. 60-65. The results show that no significant purity changes were observed for free form crystal form A and different salt forms after being placed at 25°C/60% RH and 40°C/75% RH for one week, and the crystal forms of different samples remained unchanged after the stability test, indicating that all the above crystal forms had high solid-state stability.

Table 39 Summary of solid-state stability evaluation

| Crystal form | Condition | | HPLC results | | |
|---|---|---|---|---|---|
| | | | Purity (area%) | Relative to initial (%) | Crystal form |
| Free form crystal form A | Initial | | 96.86 | -- | -- |
| | 25°C/60%RH/open | 1 week | 96.76 | 99.9 | Free form crystal form A |
| | 40°C/75%RH/open | 1 week | 96.70 | 99.8 | |
| Crystal form A of phosphate salt | Onset | | 97.62 | -- | -- |
| | 25°C/60%RH/open | 1 week | 97.84 | 100.2 | Crystal form A of phosphate salt |
| | 40°C/75%RH/open | 1 week | 97.55 | 99.9 | |
| Crystal form A of fumarate salt | Onset | | 96.99 | -- | -- |
| | 25°C/60%RH/open | 1 week | 97.02 | 100.0 | Crystal form A of fumarate salt |
| | 40°C/75%RH/open | 1 week | 97.09 | 100.1 | |
| Crystal form A of*p*-toluenesulfonate salt | Onset | | 97.67 | -- | -- |
| | 25°C/60%RH/open | 1 week | 97.38 | 99.7 | Crystal form A of *p*-toluenesulfonate salt |
| | 40°C/75%RH/open | 1 week | 97.38 | 99.7 | |

(continued)

| Table 39 Summary of solid-state stability evaluation | | | | | |
|---|---|---|---|---|---|
| **Crystal form** | **Condition** | | **HPLC results** | | |
| | | | Purity (area%) | Relative to initial (%) | Crystal form |
| Crystal form A | Onset | | 97.78 | -- | -- |
| of benzenesulfonate salt | 25°C/60%RH/open | 1 week | 97.61 | 99.8 | Crystal form A of benzenesulfonate salt |
| | 40°C/75%RH/open | 1 week | 97.61 | 99.8 | |
| Crystal form B of oxalate salt | Onset | | 97.47 | -- | -- |
| | 25°C/60%RH/open | 1 week | 97.49 | 100.0 | Crystal form B of oxalate salt |
| | 40°C/75%RH/open | 1 week | 97.52 | 100.1 | |

The control compound (trade name: Tirbanibulin) in the test examples of the present disclosure is a dual-action inhibitor of Src kinase and tubulin polymerization, which has been approved by the FDA and the European Union for the topical treatment of actinic keratosis on the face or scalp. The preparation of the control compound refers to patent WO 2008/002676 A2, and the control compound has a structure as follows:

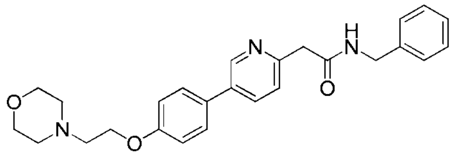

Control compound

**Test example 1: Inhibition test of tubulin monomer polymerization**

Tubulin polymerization assay kit (cytoskeleton, Cat. # BK011P) was used to conduct the compound inhibition test of tubulin monomer polymerization.

Before the test, the 96-well plate (Corning Costar, Cat. # 3686) supplied with the kit was first placed in a microplate reader (MD, SpectraMax M5) and heated to 37°C, maintained for 10 minutes. The 96-well plate was then removed, and 5 μL of 12.5 μM compound solution or blank solution was added thereto. The 96-well plate was then placed back into the microplate reader and incubated at 37°C for 1 minute to heat the compound solution to 37°C. The 96-well plate was removed and 50 μL of reaction mixture prepared according to the supplier's instructions was quickly added to each well, completing the sample addition within 1 minute to avoid the formation of bubbles. The 96-well plate was then immediately placed back into the microplate reader, shaken for 5 seconds, and using the Kinetic mode, continuous detection was carried out at 37°C for 30 to 60 minutes under the conditions of excitation light at 360 nm and emission light at 420 nm, with detection every 30 seconds. Taking the detection time as the X-axis and the fluorescence signal value as the Y-axis, the tubulin monomer polymerization curve was obtained. As shown in FIG. 66 and Table 40, the larger the Vmax value and the higher the maximum fluorescence signal value of the polymerization curve, the lower the inhibitory efficiency of the compound.

Table 40

| Compound | Blank control | Control compound | Compound of Formula I |
|---|---|---|---|
| Maximum signal value | 739 | 611 | 478 |

The test results of compounds inhibiting tubulin polymerization are shown in FIG. 66 and Table 40. The results show that the Vmax value and maximum fluorescence signal value of the polymerization curve corresponding to the compound of Formula I of the present disclosure are significantly smaller, indicating that the compound of Formula I of the present disclosure can significantly inhibit the polymerization of tubulin monomers and has the better inhibitory activity compared with the control compound.

**Test example 2: Inhibition test of compounds on cell proliferation**

Human cutaneous squamous cell carcinoma cell A-431 (ATCC, CRL-1555) and actinic keratosis patient-derived cell HT 297.T (ATCC, CRL-7782) proliferation assays were used to detect the inhibitory effect of small molecular compounds on cell proliferation.

A-431 cells and HT 297.T cells were cultured in DMEM medium containing 10% fetal bovine serum and grown at 37°C in a 5% $CO_2$ incubator. Cells on logarithmic phase were seeded into a 96-well cell culture plate at 1000 cells/well, 100 μL per well, and then cultured overnight at 37°C in a 5% $CO_2$ incubator. The next day, 100 μL of gradient-diluted 2× test compound solution was added to each well, with DMSO serving as a positive control, and 10 μM staurosporine (Aladdin, S102392) was set as a negative control group. After the addition of the compounds, the culture plate was incubated for another 4 days at 37°C in a 5% $CO_2$ incubator. After incubation, the Steady-Glo® luciferase assay system (Promega, G9243) was used according to the supplier's instructions to measure the fluorescence signal value on the Envision 2104 Multilabel Reader. The inhibition rate was calculated by the following formula, then the curve was plotted with the Log value of the inhibitor concentration as the X-axis and the inhibition rate as the Y-axis, and the $IC_{50}$ was calculated using Graphpad 8.0.

Inhibition % = (positive control group signal - test well signal) / (positive control group signal - negative control group signal) * 100

Table 41: Inhibition results of test compounds on proliferation activity of A-431 and HT 297.T cells

| Test compound | A-431 $IC_{50}$ (nM) | HT 297.T $IC_{50}$(nM) |
|---|---|---|
| Control compound | 40.30 | 85.38 |
| Compound of Formula I | 22.60 | 41.31 |

The results of the cell proliferation inhibition test of the compounds are shown in Table 41. The results show that the compound of Formula I of the present disclosure can significantly inhibit cell proliferation, especially the inhibitory effect on HT 297.T cells derived from patients with actinic keratosis is significantly better than that of the control compound, which indicates that the compound of Formula I of the present disclosure has a better therapeutic effect on actinic keratosis.

**Test example 3: Thermodynamic solubility test**

Phosphate buffer saline (PBS) with a pH of 7.4, FeSSIF solution with a pH of 6.5, and FaSSGF solution with a pH of 1.6 were prepared. The compound was accurately weighed and added to prepared phosphate buffer saline with a pH of 7.4, FeSSIF solution with a pH of 6.5, and FaSSGF solution with a pH of 1.6 to prepare into a solution with a concentration of 4 mg/mL. The solution was shaken at a speed of 1000 rpm for 1 hour, and then incubated overnight at room temperature. The incubated solution was centrifuged at 12,000 rpm for 10 minutes to remove undissolved particles, and the supernatant was transferred to a new centrifuge tube. After the supernatant was appropriately diluted, an acetonitrile solution containing internal standard was added, and a standard curve prepared with the same matrix was used for quantification.

Table 42: Thermodynamic solubility test results

| Test compound | Solubility (μg/mL) | |
|---|---|---|
| | FeSSIF (pH 6.5) | PBS (pH 7.4) |
| Control compound | 60.50 | 9.770 |
| Compound of Formula I | 649.6 | 101.1 |

The results of the thermodynamic solubility test are shown in Table 42. The results show that compared with the control compound, the compound of Formula I of the present disclosure has greater thermodynamic solubility under neutral conditions and has good druggability.

**Test example 4: Pharmacokinetic test**

For the pharmacokinetic test in mice, 3 male ICR mice, 20 to 25 g, were used, fasted overnight, and administered

by tail vein injection (1 mg/kg or 5 mg/kg). Blood was collected before the administration, and at 15 min, 30 min, 1 hour, 2 hours, 4 hours, 8 hours, and 24 hours after the administration. Another 3 mice were taken and administered by oral gavage (5 mg/kg), and blood was collected before the administration, and at 15 min, 30 min, 1 hour, 2 hours, 4 hours, 8 hours, and 24 hours after the administration. Blood samples were centrifuged at 6800 g at 2 to 8°C for 6 min, and plasma was collected and stored at -80°C. The plasma at each time point was taken and added with an acetonitrile solution containing internal standard in 3 to 5 times the amount. The mixture was vortexed and mixed for 1 min, and centrifuged at 13,000 rpm at 4°C for 10 min. The supernatant was collected, added with water in 3 times the amount, and mixed. An appropriate amount of the mixture was taken for LC-MS/MS analysis. The main pharmacokinetic parameters were analyzed using WinNonlin 7.0 software with non-compartmental model.

Table 43: Results 1 of pharmacokinetic tests in mice

| Test compound | Pharmacokinetic parameters of mice | |
|---|---|---|
| | Intravenous injection administration (1 mg/kg) | |
| | CL (L/h/kg) | $AUC_{0-t}$ (h * ng/mL) |
| Control compound | 1.88 | 525 |
| Compound of Formula I | 2.07 | 430 |

Table 44: Results 3 of pharmacokinetic tests in mice

| Test compound | Pharmacokinetic parameters of mice Oral gavage administration (5 mg/kg) | |
|---|---|---|
| | $C_{max}$ (ng/mL) | $AUC_{0-t}$ (h * ng/mL) |
| Control compound | 1010 | 1819 |
| Compound of Formula I | 322 | 443 |

The results of the pharmacokinetic tests in mice are shown in Table 43 and Table 44. The results show that compared with the control compound, the compound of Formula I of the present disclosure has rapid metabolism in mice *in vivo*, low potential systemic toxicity, and good druggability.

**Test example 5: Human liver microsome stability test**

The human liver microsome stability test was performed by incubating the compound and human liver microsomes *in vitro.* First, the compound to be tested was prepared into a 10 mM stock solution in DMSO solvent, and then the compound was diluted to 0.5 mM with acetonitrile. Human liver microsomes (Corning) were diluted with PBS into a microsome/buffer solution, and the solution was used to dilute 0.5 mM compound into a working solution, in which the concentration of the compound was 1.5 μM, and the concentration of human liver microsomes was 0.75 mg/mL. A deep-well plate was taken, 30 μL of the working solution was added per well, and then 15 μL of pre-heated 6 mM NADPH solution was added thereto to initiate a reaction, and the reaction was incubated at 37°C. At 0, 5, 15, 30 and 45 min of the incubation, 135 μL of acetonitrile was added to the corresponding wells to terminate the reaction. After the reaction was terminated with acetonitrile at the last time point of 45 min, the deep-well plate was vortexed and shaken for 10 min (600 rpm), and then centrifuged for 15 min. After centrifugation, the supernatant was collected, and added with purified water in a ratio of 1:1 to perform LC-MS/MS detection. A ratio of a peak area of compound to a peak area of internal standard at each time point was obtained, and the peak area ratios of the compound at 5, 15, 30 and 45 min were compared with the peak area ratio at 0 min to calculate the remaining percentage of the compound at each time point. $T_{1/2}$ was calculated by using GraphPad 5 software.

Table 45: Results of human liver microsome stability test

| Compound No. | $T_{1/2}$ (min) |
|---|---|
| Control compound | 32.23 |
| Compound of Formula I | 17.24 |

The results of the human liver microsome stability test are shown in Table 45. The results show that the compound of Formula I of the present disclosure is metabolized quickly by human liver microsomes, with low potential systemic

toxicity and good druggability.

**Test example 6: Inhibition of Src signaling pathway by compounds**

The Western Blot method was used to detect the compound's inhibition of p-SRC in A-431 skin cancer cells (ATCC, CRL-1555) to evaluate the inhibitory effect of the compound on the SRC signaling pathway.

A-431 cells were cultured in DMEM medium containing 10% fetal bovine serum and grown at 37°C in a 5% $CO_2$ incubator. 500,000 cells/well were seeded in a 12-well cell culture plate and cultured overnight at 37°C in a 5% $CO_2$ incubator. The next day, the culture medium was replaced and different concentrations of the compound were added, with DMSO serving as a control. After the addition of the compounds, the culture plate was cultured for another 24 hours at 37°C in a 5% $CO_2$ incubator. The cells were washed once with PBS, and lysed with RIPA lysis buffer (R0010, Solarbio) at low temperature for 30 minutes. Afterwards, the protein lysate was collected into a centrifuge tube, centrifuged at 14,000 g at 4°C for 10 minutes, and the supernatant was transferred to a new tube. The BCA protein concentration assay kit (P0009, Beyotime Biotechnology) was used to detect and calculate the protein concentration. A 5× protein loading buffer was used to quantify the protein of each sample consistently. The samples were boiled at 100°C for 5 minutes, and subjected to western blot to detect the level of p-SRC. The p-SRC antibody (Ab185617) was purchased from Abcam, and the GAPDH antibody (60004-1-Ig) was purchased from Proteintech.

The test results are shown in FIG. 67. The results show that the compound of Formula I of the present disclosure can significantly inhibit the phosphorylation of SRC at 80 nM, has better p-SRC inhibitory activity compared with the control compound, and can block the SRC downstream signaling pathway.

Although the examples of the present disclosure are illustrated and described above, it can be understood that the above examples are illustrative and should not be construed as limiting the present disclosure. Those skilled in the art can make changes, modifications, substitutions, and variations based on the above examples within the scope of the present disclosure.

## Claims

1. A crystal form or salt form of a compound of Formula I,

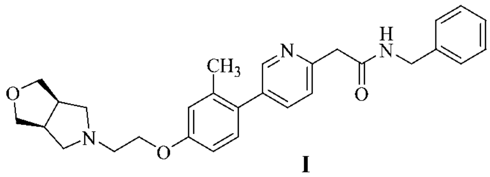

I

wherein the crystal form of the compound of Formula I is free form crystal form A or free form crystal form B,
the free form crystal form A has an X-ray powder diffraction pattern, measured using Cu-Kα radiation and expressed in terms of 2θ angles, comprising diffraction peaks at 14.49+0.2°, 17.02±0.2°, 18.42±0.2°, and 20.53 ±0.2°;
the free form crystal form B has an X-ray powder diffraction pattern, measured using Cu-Kα radiation and expressed in terms of 20 angles, comprising diffraction peaks at 16.55±0.2°, 17.77±0.2°, 20.36±0.2°, 21.24 ±0.2°, and 22.66±0.2°;
the salt form of the compound of Formula I is phosphate salt, fumarate salt, *p*-toluenesulfonate salt, benzenesulfonate salt, oxalate salt, maleate salt, malate salt, succinate salt, methanesulfonate salt, or 2-hydroxyethanesulfonate salt.

2. The crystal form or salt form of the compound of Formula I according to claim 1, wherein

the phosphate salt of the compound of Formula I is crystal form A of the phosphate salt or crystal form B of the phosphate salt of the compound of Formula I;
the crystal form A of the phosphate salt has an X-ray powder diffraction pattern, measured using Cu-Kα radiation and expressed in terms of 2θ angles, comprising diffraction peaks at 6.74±0.2°, 13.47±0.2°, 15.69±0.2°, 18.09 ±0.2°, and 20.26±0.2°;
the crystal form B of the phosphate salt has an X-ray powder diffraction pattern, measured using Cu-Kα radiation and expressed in terms of 20 angles, comprising diffraction peaks at 6.68±0.2°, 13.37±0.2°, 14.16±0.2°, 15.30 ±0.2°, 16.74±0.2°, and 20.12±0.2°;

or, the fumarate salt of the compound of Formula I is crystal form A of the fumarate salt of the compound of Formula I;

the crystal form A of the fumarate salt has an X-ray powder diffraction pattern, measured using Cu-Ka radiation and expressed in terms of 2θ angles, comprising diffraction peaks at 13.51±0.2°, 15.41±0.2°, 17.57±0.2°, 19.48 ±0.2°, and 20.11±0.2°;

or, the p-toluenesulfonate salt of the compound of Formula I is crystal form A of the *p*-toluenesulfonate salt of the compound of Formula I;

the crystal form A of the *p*-toluenesulfonate salt has an X-ray powder diffraction pattern, measured using Cu-Ka radiation and expressed in terms of 2θ angles, comprising diffraction peaks at 5.41±0.2°, 8.10±0.2°, 14.74 ±0.2°, 17.13±0.2°, 18.10±0.2°, and 19.98±0.2°;

or, the benzenesulfonate salt of the compound of Formula I is crystal form A of the benzenesulfonate salt of the compound of Formula I;

the crystal form A of the benzenesulfonate salt has an X-ray powder diffraction pattern, measured using Cu-Kα radiation and expressed in terms of 20 angles, comprising diffraction peaks at 4.29±0.2°, 8.49±0.2°, 10.02 ±0.2°, 13.81±0.2°, and 15.26±0.2°;

or, the oxalate salt of the compound of Formula I is crystal form B of the oxalate salt or crystal form A of the oxalate salt of the compound of Formula I;

the crystal form B of the oxalate salt has an X-ray powder diffraction pattern, measured using Cu-Ka radiation and expressed in terms of 20 angles, comprising diffraction peaks at 7.63±0.2°, 8.32±0.2°, 15.25±0.2°, 16.29±0.2°, 22.05±0.2°, and 23.84±0.2°;

the crystal form A of the oxalate salt has an X-ray powder diffraction pattern, measured using Cu-Kα radiation and expressed in terms of 2θ angles, comprising diffraction peaks at 7.32±0.2°, 8.47±0.2°, 14.62±0.2°, 17.00±0.2°, 18.58±0.2°, and 19.44±0.2°;

or, the maleate salt of the compound of Formula I is crystal form A of the maleate salt of the compound of Formula I;

the crystal form A of the maleate salt has an X-ray powder diffraction pattern, measured using Cu-Kα radiation and expressed in terms of 20 angles, comprising diffraction peaks at 13.54±0.2°, 16.92±0.2°, 17.64±0.2°, 19.61 ±0.2°, and 23.77±0.2°;

or, the malate salt of the compound of Formula I is crystal form A of the malate salt of the compound of Formula I;

the crystal form A of the malate salt has an X-ray powder diffraction pattern, measured using Cu-Ka radiation and expressed in terms of 2θ angles, comprising diffraction peaks at 8.95±0.2°, 9.89±0.2°, 13.21±0.2°, 16.53±0.2°, and 18.93±0.2°;

or, the succinate salt of the compound of Formula I is crystal form A of the succinate salt of the compound of Formula I;

the crystal form A of the succinate salt has an X-ray powder diffraction pattern, measured using Cu-Kα radiation and expressed in terms of 20 angles, comprising diffraction peaks at 6.94±0.2°, 9.20±0.2°, 13.87±0.2°, 16.29 ±0.2°, 17.94±0.2°, and 20.21±0.2°;

or, the methanesulfonate salt of the compound of Formula I is crystal form A of the methanesulfonate salt or crystal form B of the methanesulfonate salt of the compound of Formula I;

the crystal form A of the methanesulfonate salt has an X-ray powder diffraction pattern, measured using Cu-Kα radiation and expressed in terms of 2θ angles, comprising diffraction peaks at 5.03±0.2°, 17.03±0.2°, 18.11 ±0.2°, 20.60±0.2°, and 23.40±0.2°;

the crystal form B of the methanesulfonate salt has an X-ray powder diffraction pattern, measured using Cu-Kα radiation and expressed in terms of 20 angles, comprising diffraction peaks at 18.89±0.2°, 20.43±0.2°, 21.07 ±0.2°, 23.44±0.2°, and 24.46±0.2°;

or, the 2-hydroxyethanesulfonate salt of the compound of Formula I is crystal form A of the 2-hydroxyethanesulfonate salt of the compound of Formula I;

the crystal form A of the 2-hydroxyethanesulfonate salt has an X-ray powder diffraction pattern, measured using Cu-Ka radiation and expressed in terms of 20 angles, comprising diffraction peaks at 4.85±0.2°, 9.85±0.2°, 10.98±0.2°, 18.09±0.2°, 19.35±0.2°, and 20.33±0.2°.

**3.** The crystal form or salt form of the compound of Formula I according to claim 2, wherein

the X-ray powder diffraction pattern of the free form crystal form A, measured using Cu-Kα radiation and expressed in terms of 2θ angles, satisfies any one of the following conditions:

(1) the X-ray powder diffraction pattern comprises diffraction peaks at one or more of the following positions: 7.25±0.2°, 9.20±0.2°, 12.39±0.2°, and 13.77+0.2°;

(2) the X-ray powder diffraction pattern comprises diffraction peaks at 3.66±0.2°, 7.25±0.2°, 9.20±0.2°,

11.13±0.2°, 12.39±0.2°, 13.77±0.2°, and 14.49±0.2°;
(3) the X-ray powder diffraction pattern comprises diffraction peaks at 7.25±0.2°, 9.20±0.2°, 12.39±0.2°, 13.77±0.2°, 14.49±0.2°, 17.02±0.2°, 17.78±0.2°, 18.42±0.2°, and 20.53±0.2°;
(4) the X-ray powder diffraction pattern comprises diffraction peaks at 3.66±0.2°, 7.25±0.2°, 9.20±0.2°, 11.13±0.2°, 12.39±0.2°, 13.77±0.2°, 14.49±0.2°, 17.02±0.2°, 17.78±0.2°, 18.42±0.2°, and 20.53±0.2°;
(5) the X-ray powder diffraction pattern comprises diffraction peaks at 3.66±0.2°, 7.25±0.2°, 9.20±0.2°, 11.13±0.2°, 12.39±0.2°, 13.77±0.2°, 14.49±0.2°, 17.02±0.2°, 17.78±0.2°, 18.42±0.2°, 20.53±0.2°, and 20.92±0.2°;
(6) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 3.6586 | 7.14 | 22.9597 | 10.42 |
| 7.2523 | 15.19 | 23.3865 | 7.72 |
| 9.2043 | 13.87 | 23.9035 | 3.75 |
| 11.1262 | 7.27 | 25.7372 | 8.71 |
| 12.3913 | 13.21 | 26.2579 | 15.26 |
| 13.7658 | 13.84 | 26.6575 | 2.24 |
| 14.4926 | 31.62 | 27.2477 | 4.46 |
| 17.0216 | 38.40 | 27.6853 | 3.21 |
| 17.7818 | 24.47 | 28.1122 | 2.01 |
| 18.4187 | 100.00 | 28.5449 | 1.68 |
| 19.2078 | 6.70 | 29.1385 | 1.69 |
| 20.1286 | 12.32 | 31.0525 | 1.80 |
| 20.5296 | 39.57 | 34.1923 | 2.83 |
| 20.9229 | 15.31 | 35.5215 | 2.32 |
| 21.7756 | 6.40 | 37.2727 | 1.21 |
| 22.2614 | 4.58 | | |

;

or, the X-ray powder diffraction pattern of the free form crystal form B, measured using Cu-Kα radiation and expressed in terms of 20 angles, satisfies any one of the following conditions:

(1) the X-ray powder diffraction pattern comprises diffraction peaks at one or more of the following positions: 8.89±0.2°, 10.99±0.2°, 13.81±0.2°, 16.10±0.2°, and 19.65±0.2°;
(2) the X-ray powder diffraction pattern comprises diffraction peaks at 8.89±0.2°, 10.99±0.2°, 13.81±0.2°, 16.10±0.2°, 16.55±0.2°, 17.77±0.2°, 19.65±0.2°, 20.36±0.2°, 21.24±0.2°, and 22.66±0.2°;
(3) the X-ray powder diffraction pattern comprises diffraction peaks at 8.89±0.2°, 10.99±0.2°, 13.81±0.2°, 14.97±0.2°, 16.10±0.2°, 16.55±0.2°, 17.77±0.2°, 19.65±0.2°, 20.36±0.2°, 21.24±0.2°, 22.66±0.2°, 23.92±0.2°, 24.80±0.2°, 25.41±0.2°, and 26.65±0.2°;
(4) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 7.4873 | 3.10 | 22.6576 | 61.87 |
| 8.8864 | 9.12 | 23.9201 | 7.02 |
| 10.9912 | 18.04 | 24.7970 | 12.05 |

(continued)

| Position [°2θ] | Relative intensity [%] | Position [°2θ] | Relative intensity [%] |
|---|---|---|---|
| 13.8121 | 14.37 | 25.4143 | 15.92 |
| 14.9693 | 6.34 | 26.6488 | 16.69 |
| 16.1011 | 36.37 | 27.7637 | 6.02 |
| 16.5486 | 39.23 | 28.3501 | 3.63 |
| 16.9933 | 5.23 | 29.5259 | 2.44 |
| 17.7740 | 100.00 | 30.7717 | 5.46 |
| 18.6972 | 5.30 | 31.9792 | 2.29 |
| 19.6520 | 31.67 | 33.3167 | 3.42 |
| 20.3614 | 65.65 | 37.7157 | 3.02 |
| 21.2371 | 58.26 | 39.0657 | 1.38 |
| 22.0673 | 19.39 | | |

or, the X-ray powder diffraction pattern of the crystal form A of the phosphate salt, measured using Cu-Kα radiation and expressed in terms of 2θ angles, satisfies any one of the following conditions:

(1) the X-ray powder diffraction pattern comprises diffraction peaks at one or more of the following positions: 11.87±0.2°, 12.56±0.2°, 13.75±0.2°, 18.60±0.2°, 20.26±0.2°, and 25.26±0.2°;
(2) the X-ray powder diffraction pattern comprises diffraction peaks at 6.74±0.2°, 11.87±0.2°, 12.56±0.2°, 13.47±0.2°, 13.75±0.2°, 15.69±0.2°, 16.86±0.2°, 18.09±0.2°, and 20.26±0.2°;
(3) the X-ray powder diffraction pattern comprises diffraction peaks at 3.40±0.2°, 6.74±0.2°, 7.47±0.2°, 11.87±0.2°, 12.56±0.2°, 13.47±0.2°, 13.75±0.2°, 14.94±0.2°, 15.69±0.2°, 16.86±0.2°, 18.09±0.2°, 18.61±0.2°, 20.26±0.2°, and 25.26±0.2°;
(4) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 3.3971 | 3.92 | 20.7346 | 11.38 |
| 6.7420 | 64.07 | 21.0112 | 17.83 |
| 7.4735 | 11.63 | 22.1650 | 19.60 |
| 7.8430 | 6.09 | 22.4611 | 18.76 |
| 11.8665 | 6.89 | 22.6861 | 21.05 |
| 12.5613 | 11.06 | 23.3038 | 14.49 |
| 13.4735 | 48.13 | 23.6997 | 10.47 |
| 13.7472 | 23.38 | 24.2381 | 14.86 |
| 14.9424 | 15.60 | 24.6170 | 6.42 |
| 15.6909 | 48.98 | 25.2605 | 18.00 |
| 16.5665 | 19.30 | 26.0498 | 4.76 |
| 16.8571 | 100.00 | 27.1236 | 3.12 |
| 17.2241 | 19.19 | 27.6743 | 7.67 |
| 18.0887 | 33.50 | 28.2245 | 2.87 |
| 18.6053 | 18.77 | 29.6701 | 5.48 |
| 19.2940 | 2.78 | 30.6420 | 4.94 |

(continued)

| Position [°2θ] | Relative intensity [%] | Position [°2θ] | Relative intensity [%] |
|---|---|---|---|
| 20.2570 | 23.97 | | |

or, the X-ray powder diffraction pattern of the crystal form B of the phosphate salt, measured using Cu-Ka radiation and expressed in terms of 20 angles, satisfies any one of the following conditions:

(1) the X-ray powder diffraction pattern comprises diffraction peaks at one or more of the following positions: 3.35±0.2°, 14.53±0.2°, 21.27±0.2°, 22.53±0.2°, 24.43±0.2°, and 25.24±0.2°;
(2) the X-ray powder diffraction pattern comprises diffraction peaks at 6.68±0.2°, 13.37±0.2°, 14.16±0.2°, 14.53±0.2°, 15.30±0.2°, 16.74±0.2°, 20.12±0.2°, and 21.26±0.2°;
(3) the X-ray powder diffraction pattern comprises diffraction peaks at 3.35±0.2°, 6.68±0.2°, 13.37±0.2°, 14.16±0.2°, 14.53±0.2°, 15.30±0.2°, 16.74±0.2°, 20.12±0.2°, 21.26±0.2°, 22.53±0.2°, 24.42±0.2°, and 25.24±0.2°;
(4) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 3.3488 | 100.00 | 21.4946 | 10.39 |
| 6.6843 | 21.93 | 21.7492 | 10.41 |
| 7.6346 | 4.49 | 22.5344 | 11.77 |
| 10.0305 | 3.43 | 23.0287 | 5.88 |
| 12.1675 | 7.90 | 23.2345 | 5.21 |
| 13.3734 | 45.72 | 23.9394 | 3.17 |
| 13.7627 | 11.36 | 24.4279 | 12.17 |
| 14.1560 | 9.48 | 25.2438 | 15.76 |
| 14.5261 | 7.21 | 25.6966 | 5.56 |
| 15.0409 | 11.28 | 27.0403 | 1.05 |
| 15.3041 | 34.26 | 27.8899 | 2.69 |
| 16.7414 | 68.50 | 28.7762 | 1.94 |
| 17.2869 | 7.43 | 29.6849 | 3.25 |
| 17.4813 | 8.70 | 32.1272 | 1.17 |
| 17.7801 | 12.25 | 32.7569 | 1.84 |
| 18.1056 | 17.31 | 33.5155 | 1.11 |
| 18.8004 | 4.56 | 34.5572 | 1.79 |
| 19.6890 | 4.68 | 35.9222 | 0.83 |
| 20.1204 | 23.28 | 36.8640 | 1.30 |
| 21.2684 | 20.03 | | |

or, the X-ray powder diffraction pattern of the crystal form A of the fumarate salt, measured using Cu-Kα radiation and expressed in terms of 20 angles, satisfies any one of the following conditions:

(1) the X-ray powder diffraction pattern comprises diffraction peaks at one or more of the following positions: 9.03±0.2°, 9.78±0.2°, 16.894+0.2°, 22.04±0.2°, 23.71±0.2°, 24.24±0.2°, and 25.72±0.2°;
(2) the X-ray powder diffraction pattern comprises diffraction peaks at 9.03±0.2°, 9.78±0.2°, 13.51±0.2°, 15.41±0.2°, 16.89±0.2°, 17.57±0.2°, 19.48±0.2°, 20.11±0.2°, and 22.04±0.2°;
(3) the X-ray powder diffraction pattern comprises diffraction peaks at 9.03±0.2°, 9.78±0.2°, 13.51±0.2°,

15.41±0.2°, 16.89±0.2°, 17.57±0.2°, 19.48±0.2°, 20.11±0.2°, 22.04±0.2°, 23.71±0.2°, 24.24±0.2°, and 25.72±0.2°;

(4) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 9.0262 | 10.80 | 24.2390 | 26.28 |
| 9.7784 | 29.03 | 25.7165 | 23.45 |
| 13.5143 | 53.68 | 27.1984 | 7.74 |
| 15.4104 | 56.59 | 28.5027 | 2.01 |
| 16.8922 | 29.24 | 29.5519 | 5.77 |
| 17.5651 | 99.14 | 30.2545 | 6.02 |
| 18.3356 | 27.28 | 30.6842 | 8.40 |
| 18.6617 | 21.63 | 31.3364 | 4.48 |
| 19.4757 | 100.00 | 32.3634 | 2.45 |
| 20.1090 | 52.33 | 33.8625 | 1.35 |
| 21.1154 | 12.41 | 35.4107 | 3.44 |
| 22.0379 | 34.40 | 36.5349 | 3.18 |
| 23.0333 | 7.97 | 38.9309 | 1.92 |
| 23.7137 | 46.33 | | |

or, the X-ray powder diffraction pattern of the crystal form A of the p-toluenesulfonate salt, measured using Cu-Kα radiation and expressed in terms of 2θ angles, satisfies any one of the following conditions:

(1) the X-ray powder diffraction pattern comprises diffraction peaks at one or more of the following positions: 10.81±0.2°, 18.83±0.2°, 21.83±0.2°, and 24.37±0.2°;

(2) the X-ray powder diffraction pattern comprises diffraction peaks at 5.41±0.2°, 7.74±0.2°, 8.10±0.2°, 10.81±0.2°, 14.74±0.2°, 17.13±0.2°, 18.10±0.2°, 18.83±0.2°, and 19.98±0.2°;

(3) the X-ray powder diffraction pattern comprises diffraction peaks at 5.41±0.2°, 7.74±0.2°, 8.10±0.2°, 10.81±0.2°, 14.74±0.2°, 17.13±0.2°, 18.10±0.2°, 18.83±0.2°, 19.98±0.2°, 21.01±0.2°, 21.83±0.2°, 22.85±0.2°, 23.41±0.2°, 24.37±0.2°, 26.18±0.2°, and 28.58±0.2°;

(4) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 5.4061 | 50.32 | 19.9778 | 61.40 |
| 7.7375 | 20.44 | 21.0061 | 12.71 |
| 8.1037 | 40.15 | 21.8329 | 62.62 |
| 10.8067 | 22.25 | 22.8534 | 30.96 |
| 13.1118 | 2.94 | 23.4116 | 26.46 |
| 14.7415 | 61.45 | 24.3701 | 39.55 |
| 15.2264 | 7.77 | 26.1763 | 17.86 |
| 16.2076 | 5.59 | 27.2507 | 6.34 |
| 17.1253 | 54.33 | 28.5791 | 19.45 |
| 18.1038 | 100.00 | 30.4321 | 3.98 |
| 18.3589 | 38.45 | 31.2458 | 6.35 |

(continued)

| Position [°2θ] | Relative intensity [%] | Position [°2θ] | Relative intensity [%] |
|---|---|---|---|
| 18.8288 | 80.63 | | |

or, the X-ray powder diffraction pattern of the crystal form A of the benzenesulfonate salt, measured using Cu-Kα radiation and expressed in terms of 2θ angles, satisfies any one of the following conditions:

(1) the X-ray powder diffraction pattern comprises diffraction peaks at one or more of the following positions: 10.68±0.2°, 16.80±0.2°, 18.80±0.2°, 19.73±0.2°, 20.53±0.2°, and 20.99±0.2°;
(2) the X-ray powder diffraction pattern comprises diffraction peaks at 4.29±0.2°, 8.49±0.2°, 10.02±0.2°, 10.68±0.2°, 13.81±0.2°, 15.26±0.2°, 16.80±0.2°, 18.80±0.2°, 19.73±0.2°, 20.53±0.2°, and 20.99±0.2°;
(3) the X-ray powder diffraction pattern comprises diffraction peaks at 4.29±0.2°, 5.75±0.2°, 8.49±0.2°, 10.02±0.2°, 10.68±0.2°, 11.18±0.2°, 11.48±0.2°, 12.70±0.2°, 13.81±0.2°, 15.26±0.2°, 16.80±0.2°, 18.80±0.2°, 19.73±0.2°, 20.53±0.2°, 20.99±0.2°, 21.95±0.2°, and 23.90±0.2°;
(4) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 4.2927 | 90.89 | 20.2214 | 43.49 |
| 5.7545 | 6.24 | 20.5266 | 100.00 |
| 8.4936 | 76.94 | 20.9947 | 73.78 |
| 10.0153 | 39.96 | 21.2345 | 28.85 |
| 10.6755 | 22.03 | 21.4984 | 24.65 |
| 11.1754 | 8.55 | 21.9486 | 45.89 |
| 11.4806 | 9.68 | 22.3819 | 23.59 |
| 12.7029 | 7.82 | 22.6234 | 14.90 |
| 13.8134 | 41.83 | 23.4571 | 10.81 |
| 14.4981 | 11.11 | 23.8950 | 42.58 |
| 15.2579 | 54.96 | 25.5219 | 30.50 |
| 16.8048 | 46.57 | 25.7195 | 28.44 |
| 16.9835 | 26.02 | 27.6008 | 3.86 |
| 17.8361 | 20.18 | 28.7734 | 9.90 |
| 18.4629 | 22.00 | 29.2011 | 12.35 |
| 18.7971 | 56.58 | 30.3950 | 5.38 |
| 19.1714 | 26.12 | 31.7722 | 4.14 |
| 19.7264 | 74.71 | 33.1659 | 6.58 |

or, the X-ray powder diffraction pattern of the crystal form B of the oxalate salt, measured using Cu-Ka radiation and expressed in terms of 2θ angles, satisfies any one of the following conditions:

(1) the X-ray powder diffraction pattern comprises diffraction peaks at one or more of the following positions: 12.47±0.2°, 17.66±0.2°, 18.26±0.2°, 18.77±0.2°, and 19.16±0.2°;
(2) the X-ray powder diffraction pattern comprises diffraction peaks at 7.63±0.2°, 8.32±0.2°, 12.47±0.2°, 15.25±0.2°, 16.29±0.2°, 17.66±0.2°, 18.26±0.2°, 18.77±0.2°, 19.16±0.2°, 22.05±0.2°, and 23.84±0.2°;
(3) the X-ray powder diffraction pattern comprises diffraction peaks at 7.63±0.2°, 8.32±0.2°, 12.47±0.2°, 15.25±0.2°, 16.29±0.2°, 17.66±0.2°, 18.26±0.2°, 18.77±0.2°, 19.16±0.2°, 19.92±0.2°, 20.83±0.2°, 21.28±0.2°, 22.05±0.2°, 23.84±0.2°, 25.05±0.2°, 26.40±0.2°, 27.41±0.2°, and 29.25±0.2°;
(4) the X-ray powder diffraction pattern comprises diffraction peak positions and relative intensities as shown

in the following table:

| Position [°2θ] | Relative intensity [%] | Position [°2θ] | Relative intensity [%] |
|---|---|---|---|
| 7.6303 | 39.67 | 20.8286 | 39.95 |
| 8.3152 | 56.34 | 21.2807 | 28.72 |
| 12.4726 | 31.39 | 22.0493 | 70.88 |
| 15.2505 | 70.07 | 23.8443 | 93.78 |
| 16.2944 | 100.00 | 24.5635 | 18.67 |
| 17.6634 | 71.80 | 25.0476 | 23.37 |
| 18.2646 | 82.23 | 26.4010 | 16.45 |
| 18.4892 | 59.67 | 27.4132 | 20.10 |
| 18.7712 | 90.80 | 28.3635 | 5.97 |
| 19.1571 | 67.01 | 29.2511 | 13.78 |
| 19.9191 | 16.34 | | |

or, the X-ray powder diffraction pattern of the crystal form A of the oxalate salt, measured using Cu-Ka radiation and expressed in terms of 2θ angles, satisfies any one of the following conditions:

(1) the X-ray powder diffraction pattern comprises diffraction peaks at one or more of the following positions: 12.93±0.2°, 13.47±0.2°, 15.23±0.2°, 22.38±0.2°, and 26.00±0.2°;
(2) the X-ray powder diffraction pattern comprises diffraction peaks at 7.32±0.2°, 8.47±0.2°, 12.93±0.2°, 13.47±0.2°, 14.62±0.2°, 15.23±0.2°, 17.00±0.2°, 18.58±0.2°, 19.44±0.2°, 22.38±0.2°, and 26.00±0.2°;
(3) the X-ray powder diffraction pattern comprises diffraction peaks at 4.64±0.2°, 7.32±0.2°, 8.47±0.2°, 12.93±0.2°, 13.47±0.2°, 14.62±0.2°, 15.23±0.2°, 17.00±0.2°, 18.05±0.2°, 18.58±0.2°, 19.44±0.2°, 20.57±0.2°, 21.49±0.2°, 22.38±0.2°, 23.26±0.2°, 23.59±0.2°, and 26.00±0.2°;
(4) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 4.6409 | 9.67 | 20.5689 | 32.57 |
| 7.3243 | 34.07 | 21.0873 | 24.03 |
| 8.4725 | 100.00 | 21.4883 | 39.50 |
| 12.9334 | 19.51 | 22.3807 | 86.67 |
| 13.4699 | 12.40 | 23.2608 | 31.24 |
| 14.6183 | 86.76 | 23.5935 | 30.33 |
| 15.2278 | 7.68 | 24.0272 | 21.48 |
| 16.9988 | 89.93 | 25.9955 | 32.08 |
| 18.0487 | 48.94 | 27.5449 | 4.60 |
| 18.5824 | 84.16 | 28.6854 | 3.47 |
| 18.8319 | 39.40 | 30.1750 | 12.59 |
| 19.4386 | 62.43 | 30.7971 | 5.66 |

or, the X-ray powder diffraction pattern of the crystal form A of the maleate salt, measured using Cu-Kα radiation and expressed in terms of 2θ angles, satisfies any one of the following conditions:

(1) the X-ray powder diffraction pattern comprises diffraction peaks at one or more of the following positions:

3.44±0.2°, 9.64±0.2°, 18.08±0.2°, 20.82±0.2°, and 27.20±0.2°;
(2) the X-ray powder diffraction pattern comprises diffraction peaks at 3.44±0.2°, 9.64±0.2°, 13.54±0.2°, 16.92±0.2°, 17.64±0.2°, 18.08±0.2°, 19.61±0.2°, 20.82±0.2°, 23.77±0.2°, and 27.20±0.2°;
(3) the X-ray powder diffraction pattern comprises diffraction peaks at 3.44±0.2°, 9.64±0.2°, 13.54±0.2°, 16.92±0.2°, 17.64±0.2°, 18.08±0.2°, 19.61±0.2°, 20.82±0.2°, 22.36±0.2°, 22.71±0.2°, 23.77±0.2°, 27.20±0.2°, 28.90±0.2°, and 30.67±0.2°;
(4) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 3.4417 | 16.32 | 21.6390 | 7.94 |
| 9.6432 | 27.14 | 22.3570 | 27.06 |
| 10.1448 | 5.95 | 22.7123 | 28.31 |
| 13.5352 | 100.00 | 23.7674 | 95.50 |
| 15.7013 | 7.54 | 24.4456 | 6.76 |
| 16.9220 | 49.89 | 24.8503 | 8.90 |
| 17.6360 | 60.94 | 25.5895 | 12.79 |
| 18.0813 | 36.58 | 26.7126 | 5.99 |
| 18.5713 | 13.66 | 27.2013 | 26.88 |
| 18.9912 | 11.46 | 27.7331 | 8.29 |
| 19.6099 | 51.55 | 28.8950 | 13.37 |
| 20.8195 | 31.63 | 30.6662 | 21.93 |

or, the X-ray powder diffraction pattern of the crystal form A of the malate salt, measured using Cu-Kα radiation and expressed in terms of 2θ angles, satisfies any one of the following conditions:

(1) the X-ray powder diffraction pattern comprises diffraction peaks at 8.95±0.2°, 9.89±0.2°, 13.21±0.2°, 16.17±0.2°, 16.53±0.2°, 18.93±0.2°, and 24.77±0.2°;
(2) the X-ray powder diffraction pattern of the crystal form A of the malate salt, measured using Cu-Ka radiation and expressed in terms of 2θ angles, comprises diffraction peaks at 6.63±0.2°, 8.95±0.2°, 9.89 ±0.2°, 13.21±0.2°, 16.17±0.2°, 16.53±0.2°, 18.93±0.2°, 21.62±0.2°, 23.20±0.2°, 23.80±0.2°, and 24.77 ±0.2°;
(3) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 6.6262 | 7.38 | 19.7373 | 21.21 |
| 8.9534 | 20.94 | 21.6214 | 25.16 |
| 9.8936 | 38.20 | 23.1988 | 17.64 |
| 13.2054 | 50.38 | 23.8001 | 22.22 |
| 16.1763 | 29.75 | 24.7654 | 32.77 |
| 16.5259 | 50.45 | 27.2491 | 13.36 |
| 17.8951 | 33.19 | 31.4661 | 5.26 |
| 18.9290 | 100.00 | | |

or, the X-ray powder diffraction pattern of the crystal form A of the succinate salt, measured using Cu-Kα radiation and expressed in terms of 2θ angles, satisfies any one of the following conditions:

(1) the X-ray powder diffraction pattern comprises diffraction peaks at one or more of the following positions: 14.70±0.2°, 17.40±0.2°, 19.48±0.2°, 20.96±0.2°, 22.18±0.2°, 23.04±0.2°, 23.92±0.2°, and 26.18±0.2°;
(2) the X-ray powder diffraction pattern comprises diffraction peaks at 6.94±0.2°, 9.20±0.2°, 13.87±0.2°, 16.29±0.2°, 17.94±0.2°, 20.21±0.2°, 20.96±0.2°, 22.18±0.2°, and 26.18±0.2°;
(3) the X-ray powder diffraction pattern comprises diffraction peaks at 6.94±0.2°, 9.20±0.2°, 13.87±0.2°, 14.70±0.2°, 16.29±0.2°, 17.40±0.2°, 17.94±0.2°, 19.48±0.2°, 20.21±0.2°, 20.96±0.2°, 22.18±0.2°, 23.04±0.2°, 23.92±0.2°, and 26.18±0.2°;
(4) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 6.9349 | 100.00 | 19.4760 | 19.47 |
| 9.1969 | 38.52 | 20.2096 | 35.96 |
| 11.7777 | 6.58 | 20.9648 | 45.17 |
| 13.8697 | 56.93 | 22.1780 | 31.28 |
| 14.6969 | 18.62 | 23.0453 | 19.19 |
| 16.2949 | 72.71 | 23.9225 | 28.52 |
| 17.3959 | 14.28 | 26.1847 | 32.80 |
| 17.9354 | 39.73 | 28.0540 | 5.21 |
| 18.3860 | 12.73 | 34.8694 | 3.06 |

;

or, the X-ray powder diffraction pattern of the crystal form A of the methanesulfonate salt, measured using Cu-Kα radiation and expressed in terms of 2θ angles, comprises diffraction peak positions and relative intensities as shown in the following table:

| Position [°2θ] | Relative intensity [%] | Position [°2θ] | Relative intensity [%] |
|---|---|---|---|
| 5.0258 | 56.54 | 20.5965 | 100.00 |
| 17.0335 | 38.98 | 23.3964 | 71.59 |
| 18.1100 | 61.40 | | |

or, the X-ray powder diffraction pattern of the crystal form B of the methanesulfonate salt, measured using Cu-Kα radiation and expressed in terms of 2θ angles, satisfies any one of the following conditions:

(1) the X-ray powder diffraction pattern comprises diffraction peaks at one or more of the following positions: 9.83±0.2°, 11.32±0.2°, 15.46±0.2°, and 17.26±0.2°;
(2) the X-ray powder diffraction pattern comprises diffraction peaks at 9.83±0.2°, 11.32±0.2°, 15.46±0.2°, 17.26±0.2°, 18.89±0.2°, 20.43±0.2°, 21.07±0.2°, 23.44±0.2°, and 24.46±0.2°;
(3) the X-ray powder diffraction pattern comprises diffraction peaks at 9.83±0.2°, 11.32±0.2°, 15.46±0.2°, 16.98±0.2°, 17.26±0.2°, 17.63±0.2°, 18.89±0.2°, 19.68±0.2°, 20.43±0.2°, 21.07±0.2°, 21.58±0.2°, 22.09±0.2°, 22.69±0.2°, 23.44±0.2°, and 24.46±0.2°;
(4) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 9.8298 | 9.76 | 22.6933 | 23.52 |
| 11.3186 | 15.66 | 23.4414 | 73.53 |
| 11.8183 | 5.19 | 23.9398 | 15.74 |

(continued)

| Position [°2θ] | Relative intensity [%] | Position [°2θ] | Relative intensity [%] |
|---|---|---|---|
| 12.6882 | 3.11 | 24.4552 | 60.92 |
| 13.7455 | 5.81 | 25.3114 | 10.00 |
| 14.3775 | 6.16 | 25.6224 | 15.39 |
| 15.4572 | 22.47 | 25.8897 | 14.19 |
| 16.5409 | 6.51 | 27.0121 | 4.85 |
| 16.9751 | 29.83 | 27.4334 | 6.22 |
| 17.2607 | 52.29 | 27.9938 | 6.98 |
| 17.6280 | 39.70 | 28.5662 | 8.75 |
| 18.4451 | 35.15 | 29.3314 | 8.75 |
| 18.8911 | 100.00 | 29.7831 | 9.39 |
| 19.6841 | 30.75 | 30.3249 | 5.40 |
| 20.4301 | 65.78 | 31.3892 | 5.83 |
| 21.0733 | 65.86 | 32.6597 | 5.59 |
| 21.5827 | 20.17 | 35.2268 | 6.84 |
| 22.0878 | 29.37 | 37.3650 | 3.62 |

or, the X-ray powder diffraction pattern of the crystal form A of the 2-hydroxyethanesulfonate salt, measured using Cu-Ka radiation and expressed in terms of 2θ angles, satisfies any one of the following conditions:

(1) the X-ray powder diffraction pattern comprises diffraction peaks at one or more of the following positions: 15.54±0.2°, 15.94±0.2°, 21.83±0.2°, 22.33±0.2°, and 22.87±0.2°;
(2) the X-ray powder diffraction pattern comprises diffraction peaks at 4.85±0.2°, 9.85±0.2°, 10.98±0.2°, 15.54±0.2°, 15.94±0.2°, 18.09±0.2°, 19.35±0.2°, 20.33±0.2°, 21.83±0.2°, 22.33±0.2°, and 22.87±0.2°;
(3) the X-ray powder diffraction pattern comprises diffraction peaks at 4.85±0.2°, 9.85±0.2°, 10.98±0.2°, 12.83±0.2°, 15.54±0.2°, 15.94±0.2°, 16.66±0.2°, 18.09±0.2°, 19.35±0.2°, 20.33±0.2°, 21.83±0.2°, 22.33±0.2°, and 22.87±0.2°;
(4) the X-ray powder diffraction pattern comprises diffraction peak positions and relative intensities as shown in the following table:

| Position [°2θ] | Relative intensity [%] | Position [°2θ] | Relative intensity [%] |
|---|---|---|---|
| 4.8473 | 97.56 | 20.3297 | 56.13 |
| 9.8479 | 49.33 | 21.8277 | 45.05 |
| 10.9792 | 82.75 | 22.3258 | 67.31 |
| 12.8290 | 12.79 | 22.8682 | 63.84 |
| 14.2549 | 4.53 | 24.9854 | 21.98 |
| 15.5419 | 25.71 | 26.2466 | 10.34 |
| 15.9356 | 30.48 | 26.9452 | 8.37 |
| 16.6623 | 12.53 | 28.5116 | 17.43 |
| 18.0907 | 88.25 | 31.1759 | 4.85 |
| 19.3520 | 100.00 | | |

**4.** The crystal form or salt form of the compound of Formula I according to claim 3, wherein

the free form crystal form A of the compound of Formula I satisfies one or more of the following conditions:

(1) the free form crystal form A is an anhydrous crystal form;
(2) the free form crystal form A has an X-ray powder diffraction pattern, measured using Cu-Ka radiation and expressed in terms of 2θ angles, as shown in FIG. 1;
(3) the free form crystal form A has a differential scanning calorimetry curve showing an onset of an endothermic peak at 94.7±3°C;
(4) the free form crystal form A has a differential scanning calorimetry curve showing an endothermic peak at 96.6±3°C;
(5) the free form crystal form A has a thermogravimetric analysis curve showing a weight loss of approximately 0.98% at 150°C;
(6) the free form crystal form A has a differential scanning calorimetry curve as shown in FIG. 3;
(7) the free form crystal form A has a thermogravimetric analysis curve as shown in FIG. 2;

or, the free form crystal form B satisfies one or more of the following conditions:

(1) the free form crystal form B is an anhydrous crystal form;
(2) the free form crystal form B has an X-ray powder diffraction pattern, measured using Cu-Ka radiation and expressed in terms of 2θ angles, as shown in FIG. 4;
(3) the free form crystal form B has a differential scanning calorimetry curve showing an onset of an endothermic peak at 77.2±3°C;
(4) the free form crystal form B has a differential scanning calorimetry curve showing an endothermic peak at 84.5±3°C;
(5) the free form crystal form B has a differential scanning calorimetry curve showing an endothermic peak at 92.4±3°C;
(6) the free form crystal form B has a thermogravimetric analysis curve showing a weight loss of approximately 0.48% at 150°C;
(7) the free form crystal form B has a differential scanning calorimetry curve as shown in FIG. 6;
(8) the free form crystal form B has a thermogravimetric analysis curve as shown in FIG. 5;

or, the crystal form A of the phosphate salt satisfies one or more of the following conditions:

(1) the crystal form A of the phosphate salt has an X-ray powder diffraction pattern, measured using Cu-Kα radiation and expressed in terms of 2θ angles, as shown in FIG. 7;
(2) the crystal form A of the phosphate salt has a differential scanning calorimetry curve showing an onset of an endothermic peak at 172.8±3°C;
(3) the crystal form A of the phosphate salt has a differential scanning calorimetry curve showing an endothermic peak at 174.5±3°C;
(4) the crystal form A of the phosphate salt has a thermogravimetric analysis curve showing a weight loss of approximately 4.40% at 150°C;
(5) the crystal form A of the phosphate salt has a differential scanning calorimetry curve as shown in FIG. 9;
(6) the crystal form A of the phosphate salt has a thermogravimetric analysis curve as shown in FIG. 8;
(7) in the crystal form A of the phosphate salt, the molar ratio of the compound of Formula I to phosphoric acid is 1:1;

or, the crystal form B of the phosphate salt satisfies one or more of the following conditions:

(1) the crystal form B of the phosphate salt is an anhydrous crystal form;
(2) the crystal form B of the phosphate salt has an X-ray powder diffraction pattern, measured using Cu-Ka radiation and expressed in terms of 2θ angles, as shown in FIG. 11;
(3) in the crystal form B of the phosphate salt, the molar ratio of the compound of Formula I to phosphoric acid is 1:1;

or, the crystal form A of the fumarate salt satisfies one or more of the following conditions:

(1) the crystal form A of the fumarate salt is an anhydrous crystal form;
(2) the crystal form A of the fumarate salt has an X-ray powder diffraction pattern, measured using Cu-Ka radiation and expressed in terms of 2θ angles, as shown in FIG. 12;
(3) the crystal form A of the fumarate salt has a differential scanning calorimetry curve showing an onset of an endothermic peak at 128.2±3°C;

(4) the crystal form A of the fumarate salt has a differential scanning calorimetry curve showing an endothermic peak at 130.3±3°C;
(5) the crystal form A of the fumarate salt has a thermogravimetric analysis curve showing a weight loss of approximately 1.17% at 150°C;
(6) the crystal form A of the fumarate salt has a differential scanning calorimetry curve as shown in FIG. 14;
(7) the crystal form A of the fumarate salt has a thermogravimetric analysis curve as shown in FIG. 13;
(8) in the crystal form A of the fumarate salt, the molar ratio of the compound of Formula I to fumaric acid is 1:1;

or, the crystal form A of the *p*-toluenesulfonate salt satisfies one or more of the following conditions:

(1) the crystal form A of the *p*-toluenesulfonate salt has an X-ray powder diffraction pattern, measured using Cu-Ka radiation and expressed in terms of 2θ angles, as shown in FIG. 16;
(2) the crystal form A of the *p*-toluenesulfonate salt has a differential scanning calorimetry curve showing an onset of an endothermic peak at 149.9±3°C;
(3) the crystal form A of the *p*-toluenesulfonate salt has a differential scanning calorimetry curve showing an endothermic peak at 152.1±3°C;
(4) the crystal form A of the *p*-toluenesulfonate salt has a thermogravimetric analysis curve showing a weight loss of approximately 0.94% at 150°C;
(5) the crystal form A of the *p*-toluenesulfonate salt has a differential scanning calorimetry curve as shown in FIG. 18;
(6) the crystal form A of the *p*-toluenesulfonate salt has a thermogravimetric analysis curve as shown in FIG. 17;
(7) in the crystal form A of the *p*-toluenesulfonate salt, the molar ratio of the compound of Formula I to *p*-toluenesulfonic acid is 1:1;

or, the crystal form A of the benzenesulfonate salt satisfies one or more of the following conditions:

(1) the crystal form A of the benzenesulfonate salt has an X-ray powder diffraction pattern, measured using Cu-Ka radiation and expressed in terms of 2θ angles, as shown in FIG. 19;
(2) the crystal form A of the benzenesulfonate salt has a differential scanning calorimetry curve showing an onset of an endothermic peak at 144.5±3°C;
(3) the crystal form A of the benzenesulfonate salt has a differential scanning calorimetry curve showing an endothermic peak at 146.4±3°C;
(4) the crystal form A of the benzenesulfonate salt has a thermogravimetric analysis curve showing a weight loss of approximately 1.28% at 140°C;
(5) the crystal form A of the benzenesulfonate salt has a differential scanning calorimetry curve as shown in FIG. 21;
(6) the crystal form A of the benzenesulfonate salt has a thermogravimetric analysis curve as shown in FIG. 20;
(7) in the crystal form A of the benzenesulfonate salt, the molar ratio of the compound of Formula I to benzenesulfonic acid is 1:1;

or, the crystal form B of the oxalate salt satisfies one or more of the following conditions:

(1) the crystal form B of the oxalate salt has an X-ray powder diffraction pattern, measured using Cu-Kα radiation and expressed in terms of 20 angles, as shown in FIG. 22;
(2) the crystal form B of the oxalate salt has a differential scanning calorimetry curve showing an onset of an endothermic peak at 155.5±3°C;
(3) the crystal form B of the oxalate salt has a differential scanning calorimetry curve showing an endothermic peak at 158.2±3°C;
(4) the crystal form B of the oxalate salt has a differential scanning calorimetry curve showing an endothermic peak at 218.7±3°C;
(5) the crystal form B of the oxalate salt has a thermogravimetric analysis curve showing a weight loss of approximately 1.21% at 150°C;
(6) the crystal form B of the oxalate salt has a differential scanning calorimetry curve as shown in FIG. 24;
(7) the crystal form B of the oxalate salt has a thermogravimetric analysis curve as shown in FIG. 23;
(8) in the crystal form B of the oxalate salt, the molar ratio of the compound of Formula I to oxalic acid is 1:1;

or, the crystal form A of the oxalate salt satisfies one or more of the following conditions:

(1) the crystal form A of the oxalate salt has an X-ray powder diffraction pattern, measured using Cu-Ka radiation and expressed in terms of 2θ angles, as shown in FIG. 38;
(2) the crystal form A of the oxalate salt has a differential scanning calorimetry curve showing an onset of an endothermic peak at 151.3±3°C;
(3) the crystal form A of the oxalate salt has a differential scanning calorimetry curve showing an endothermic peak at 153.6±3°C;
(4) the crystal form A of the oxalate salt has a differential scanning calorimetry curve showing an endothermic peak at 162.8±3°C;
(5) the crystal form A of the oxalate salt has a differential scanning calorimetry curve showing an endothermic peak at 218.8±3°C;
(6) the crystal form A of the oxalate salt has a thermogravimetric analysis curve showing a weight loss of approximately 4.10% at 150°C;
(7) the crystal form A of the oxalate salt has a differential scanning calorimetry curve as shown in FIG. 40;
(8) the crystal form A of the oxalate salt has a thermogravimetric analysis curve as shown in FIG. 39;

or, the crystal form A of the maleate salt satisfies one or more of the following conditions:

(1) the crystal form A of the maleate salt has an X-ray powder diffraction pattern, measured using Cu-Kα radiation and expressed in terms of 2θ angles, as shown in FIG. 25;
(2) the crystal form A of the maleate salt has a differential scanning calorimetry curve showing an onset of an endothermic peak at 123.4±3°C;
(3) the crystal form A of the maleate salt has a differential scanning calorimetry curve showing an endothermic peak at 125.2±3°C;
(4) the crystal form A of the maleate salt has a thermogravimetric analysis curve showing a weight loss of approximately 2.56% at 120°C;
(5) the crystal form A of the maleate salt has a differential scanning calorimetry curve as shown in FIG. 27;
(6) the crystal form A of the maleate salt has a thermogravimetric analysis curve as shown in FIG. 26;
(7) in the crystal form A of the maleate salt, the molar ratio of the compound of Formula I to maleic acid is 1:1;

or, the crystal form A of the malate salt satisfies one or more of the following conditions:

(1) the crystal form A of the malate salt has an X-ray powder diffraction pattern, measured using Cu-Ka radiation and expressed in terms of 2θ angles, as shown in FIG. 28;
(2) the crystal form A of the malate salt has a differential scanning calorimetry curve showing an onset of an endothermic peak at 86.3±3°C;
(3) the crystal form A of the malate salt has a differential scanning calorimetry curve showing an endothermic peak at 96.3±3°C;
(4) the crystal form A of the malate salt has a thermogravimetric analysis curve showing a weight loss of approximately 2.26% at 150°C;
(5) the crystal form A of the malate salt has a differential scanning calorimetry curve as shown in FIG. 30;
(6) the crystal form A of the malate salt has a thermogravimetric analysis curve as shown in FIG. 29;
(7) in the crystal form A of the malate salt, the molar ratio of the compound of Formula I to malic acid is 1:1;

or, the crystal form A of the succinate salt satisfies one or more of the following conditions:

(1) in the crystal form A of the succinate salt, the molar ratio of the compound of Formula I to succinic acid is 1:1.2, 1:1.1, or 1:1;
(2) the crystal form A of the succinate salt has an X-ray powder diffraction pattern, measured using Cu-Ka radiation and expressed in terms of 2θ angles, as shown in FIG. 31;
(3) the crystal form A of the succinate salt has a differential scanning calorimetry curve showing an onset of an endothermic peak at 75.9±3°C;
(4) the crystal form A of the succinate salt has a differential scanning calorimetry curve showing an endothermic peak at 85.4±3°C;
(5) the crystal form A of the succinate salt has a thermogravimetric analysis curve showing a weight loss of approximately 4.49% at 150°C;
(6) the crystal form A of the succinate salt has a differential scanning calorimetry curve as shown in FIG. 33;

(7) the crystal form A of the succinate salt has a thermogravimetric analysis curve as shown in FIG. 32;
(8) in the crystal form A of the succinate salt, the molar ratio of the compound of Formula I to succinic acid is 1:1;

or, the crystal form A of the methanesulfonate salt has an X-ray powder diffraction pattern, measured using Cu-Kα radiation and expressed in terms of 2θ angles, as shown in FIG. 34;
or, the crystal form B of the methanesulfonate salt satisfies one or more of the following conditions:

(1) in the crystal form B of the methanesulfonate salt, the molar ratio of the compound of Formula I to methanesulfonic acid is 1:2.1 or 1:2;
(2) the crystal form B of the methanesulfonate salt has an X-ray powder diffraction pattern, measured using Cu-Kα radiation and expressed in terms of 2θ angles, as shown in FIG. 35;
(3) the crystal form B of the methanesulfonate salt has a differential scanning calorimetry curve showing an onset of an endothermic peak at 97.3±3°C;
(4) the crystal form B of the methanesulfonate salt has a differential scanning calorimetry curve showing an endothermic peak at 109.4:1::3 °C;
(5) the crystal form B of the methanesulfonate salt has a thermogravimetric analysis curve showing a weight loss of approximately 0.91% at 80°C and a weight loss of approximately 2.40% at 80-130°C;
(6) the crystal form B of the methanesulfonate salt has a differential scanning calorimetry curve as shown in FIG. 37;
(7) the crystal form B of the methanesulfonate salt has a thermogravimetric analysis curve as shown in FIG. 36;
(8) in the crystal form B of the methanesulfonate salt, the molar ratio of the compound of Formula I to methanesulfonic acid is 1:2;

or, the crystal form A of the 2-hydroxyethanesulfonate salt satisfies one or more of the following conditions:

(1) the crystal form A of the 2-hydroxyethanesulfonate salt has an X-ray powder diffraction pattern, measured using Cu-Kα radiation and expressed in terms of 20 angles, as shown in FIG. 41;
(2) the crystal form A of the 2-hydroxyethanesulfonate salt has a differential scanning calorimetry curve showing an onset of an endothermic peak at 109.5±3°C;
(3) the crystal form A of the 2-hydroxyethanesulfonate salt has a differential scanning calorimetry curve showing an endothermic peak at 118.4±3°C;
(4) the crystal form A of the 2-hydroxyethanesulfonate salt has a thermogravimetric analysis curve showing a weight loss of approximately 1.28% at 110°C;
(5) the crystal form A of the 2-hydroxyethanesulfonate salt has a differential scanning calorimetry curve as shown in FIG. 43;
(6) the crystal form A of the 2-hydroxyethanesulfonate salt has a thermogravimetric analysis curve as shown in FIG. 42;
(7) in the crystal form A of the 2-hydroxyethanesulfonate salt, the molar ratio of the compound of Formula I to 2-hydroxyethanesulfonic acid is 1:1.

**5.** A preparation method for a crystal form or salt form of a compound of Formula I, wherein the crystal form of the compound of Formula I is the free form crystal form A or the free form crystal form B according to any one of claims 1-4;

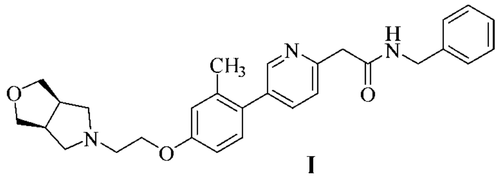

I

the salt form of the compound of Formula I is the crystal form A of the phosphate salt, the crystal form B of the phosphate salt, the crystal form A of the fumarate salt, the crystal form A of the p-toluenesulfonate salt, the crystal form A of the benzenesulfonate salt, the crystal form B of the oxalate salt, the crystal form A of the oxalate salt, the crystal form A of the maleate salt, the crystal form A of the malate salt, the crystal form A of the succinate salt, the crystal form A of the methanesulfonate salt, the crystal form B of the methanesulfonate salt, or the crystal form A of the 2-hydroxyethanesulfonate salt of the compound of Formula I according to any one of claims 2-4;

wherein
a preparation method for the free form crystal form A comprises the following steps: dissolving the compound of Formula I in a solvent and evaporating at room temperature to obtain the free form crystal form A; the solvent is an organic solvent or a mixed solvent, wherein the organic solvent is preferably $CHCl_3$, THF, acetone, toluene, cyclopentyl methyl ether, 2-methyltetrahydrofuran, isopropanol, IPAc, or 2-butanone; the mixed solvent is a mixed solvent of an organic solvent and water, the mixed solvent is preferably a mixed solvent of MeOH and $H_2O$ or a mixed solvent of acetonitrile and $H_2O$;
a preparation method for the free form crystal form B comprises the following steps: concentrating a solution of the compound of Formula I under reduced pressure and drying at 40-60°C to obtain the free form crystal form B; the solution is an alcohol solution or a halogenated hydrocarbon solution;
a preparation method for the crystal form A of the phosphate salt comprises the following steps: mixing the compound of Formula I, phosphoric acid, and a solvent, and slurrying at room temperature to obtain the crystal form A of the phosphate salt of the compound of Formula I; the solvent is an ester solvent, an ether solvent, a mixed solvent of an alcohol solvent and water, or a mixed solvent of a ketone solvent and a saturated alkane solvent;
a preparation method for the crystal form B of the phosphate salt comprises the following step: heating the crystal form A of the phosphate salt to $150\pm10$°C to obtain the crystal form B of the phosphate salt;
a preparation method for the crystal form A of the fumarate salt comprises the following steps: mixing the compound of Formula I, fumaric acid, and a solvent, and slurrying at room temperature to obtain the crystal form A of the fumarate salt; the solvent is an ester solvent, an ether solvent, a mixed solvent of an alcohol solvent and water, or a mixed solvent of a ketone solvent and a saturated alkane solvent;
a preparation method for the crystal form A of the p-toluenesulfonate salt comprises the following steps: mixing the compound of Formula I, p-toluenesulfonic acid, and a solvent, and slurrying at room temperature to obtain the crystal form A of the p-toluenesulfonate salt; the solvent is an ester solvent or an ether solvent;
a preparation method for the crystal form A of the benzenesulfonate salt comprises the following steps: mixing the compound of Formula I, benzenesulfonic acid, and a solvent, and slurrying at room temperature to obtain the crystal form A of the benzenesulfonate salt; the solvent is an ester solvent, or a mixed solvent of an alcohol solvent and water;
a preparation method for the crystal form B of the oxalate salt comprises the following steps: mixing the compound of Formula I, oxalic acid, and a solvent, and slurrying at room temperature to obtain the crystal form B of the oxalate salt; the solvent is an ester solvent, an ether solvent, or a mixed solvent of a ketone solvent and a saturated alkane solvent;
a preparation method for the crystal form A of the oxalate salt comprises the following steps: mixing the compound of Formula I, oxalic acid, and a solvent, and slurrying at room temperature to obtain the crystal form A of the oxalate salt; the solvent is a mixed solvent of an alcohol solvent and water;
a preparation method for the crystal form A of the maleate salt comprises the following steps: mixing the compound of Formula I, maleic acid, and a solvent, and slurrying at room temperature to obtain the crystal form A of the maleate salt; the solvent is an ester solvent, an ether solvent, or a mixed solvent of a ketone solvent and a saturated alkane solvent;
a preparation method for the crystal form A of the malate salt comprises the following steps: mixing the compound of Formula I, malic acid, and a solvent, and slurrying at room temperature to obtain the crystal form A of the malate salt; the solvent is a mixed solvent of a ketone solvent and a saturated alkane solvent;
a preparation method for the crystal form A of the succinate salt comprises the following steps: mixing the compound of Formula I, succinic acid, and a solvent, and slurrying at room temperature to obtain the crystal form A of the succinate salt; the solvent is an ether solvent;
a preparation method for the crystal form A of the methanesulfonate salt comprises the following steps: mixing the compound of Formula I, methanesulfonic acid, and a solvent, and slurrying at room temperature to obtain the crystal form A of the methanesulfonate salt; the solvent is a mixed solvent of a ketone solvent and a saturated alkane solvent;
a preparation method for the crystal form B of the methanesulfonate salt comprises the following steps: mixing the compound of Formula I, methanesulfonic acid, and a solvent, and slurrying at room temperature to obtain the crystal form B of the methanesulfonate salt; the solvent is an ester solvent, an ether solvent, or a mixed solvent of a ketone solvent and a saturated alkane solvent;
a preparation method for the crystal form A of the 2-hydroxyethanesulfonate salt comprises the following steps: mixing the compound of Formula I, 2-hydroxyethanesulfonic acid, and a solvent, and slurrying at room temperature to obtain the crystal form A of the 2-hydroxyethanesulfonate salt; the solvent is an ester solvent, or a mixed solvent of a ketone solvent and a saturated alkane solvent.

**6.** The preparation method according to claim 5, wherein:

in the preparation method for the free form crystal form B, the compound of Formula I is dissolved in MeOH or dichloromethane, followed by rotary evaporation under reduced pressure, and the resulting gel-like sample is transferred for vacuum drying at 50°C to obtain the free form crystal form B;
or, in the preparation method for the crystal form A of the phosphate salt, the preparation method for the crystal form A of the phosphate salt satisfies one or more of the following conditions:

(1) the molar ratio of the phosphoric acid to the compound of Formula I is (1.0-1.7):1; preferably 1.2:1;
(2) the molar volume ratio of the compound of Formula I to the solvent is 0.11-0.21 mol/L; preferably 0.16 mol/L;
(3) in the mixed solvent of the alcohol solvent and water, the volume ratio of the alcohol solvent to water is (14-24):1, preferably 19:1;
(4) in the mixed solvent of the ketone solvent and the saturated alkane solvent, the volume ratio of the ketone solvent to the saturated alkane solvent is (0.5-2):1;
(5) the solvent is a mixed solvent of IPA/$H_2O$ (19:1, v/v), a mixed solvent of acetone/n-heptane (1:1, v/v), isopropyl acetate, or methyl *tert*-butyl ether;

or, in the preparation method for the crystal form B of the phosphate salt, the preparation method for the crystal form B of the phosphate salt satisfies one or two of the following conditions:

(1) the crystal form A of the phosphate salt is heated under a nitrogen atmosphere;
(2) the temperature for the heating is 145-155°C, preferably 150°C;

or, in the preparation method for the crystal form A of the fumarate salt, the preparation method for the crystal form A of the fumarate salt satisfies one or more of the following conditions:

(1) the molar ratio of the fumaric acid to the compound of Formula I is (1.0-1.5):1; preferably 1.0:1;
(2) the molar volume ratio of the compound of Formula I to the solvent is 0.06-0.16 mol/L; preferably 0.11 mol/L;
(3) in the mixed solvent of the alcohol solvent and water, the volume ratio of the alcohol solvent to water is (14-24):1, preferably 19:1;
(4) in the mixed solvent of the ketone solvent and the saturated alkane solvent, the volume ratio of the ketone solvent to the saturated alkane solvent is (0.5-2):1;
(5) the solvent is a mixed solvent of IPA/$H_2O$ (19:1, v/v), a mixed solvent of acetone/n-heptane (1:1, v/v), isopropyl acetate, or methyl *tert*-butyl ether;

or, in the preparation method for the crystal form A of the p-toluenesulfonate salt, the preparation method for the crystal form A of the *p*-toluenesulfonate salt satisfies one or more of the following conditions:

(1) the molar ratio of the p-toluenesulfonic acid to the compound of Formula I is (1.0-1.5):1; preferably 1.0:1;
(2) the molar volume ratio of the compound of Formula I to the solvent is 0.06-0.16 mol/L; preferably 0.11 mol/L;
(3) the solvent is isopropyl acetate or methyl tert-butyl ether;

or, in the preparation method for the crystal form A of the benzenesulfonate salt, the preparation method for the crystal form A of the benzenesulfonate salt satisfies one or more of the following conditions:

(1) the molar ratio of the benzenesulfonic acid to the compound of Formula I is (1.0-1.5):1; preferably 1.0:1;
(2) the molar volume ratio of the compound of Formula I to the solvent is 0.06-0.16 mol/L; preferably 0.11 mol/L;
(3) in the mixed solvent of the alcohol solvent and water, the volume ratio of the alcohol solvent to water is (14-24):1, preferably 19:1;
(4) the solvent is a mixed solvent of IPA/$H_2O$ (19:1, v/v) or isopropyl acetate;

or, in the preparation method for the crystal form B of the oxalate salt, the preparation method for the crystal form B of the oxalate salt satisfies one or more of the following conditions:

(1) the molar ratio of the oxalic acid to the compound of Formula I is (1.0-1.5):1; preferably 1.0:1;
(2) the molar volume ratio of the compound of Formula I to the solvent is 0.02-0.08 mol/L; preferably 0.05 mol/L;
(3) in the mixed solvent of the ketone solvent and the saturated alkane solvent, the volume ratio of the ketone solvent to the saturated alkane solvent is (0.5-2):1;
(4) the solvent is a mixed solvent of acetone/*n*-heptane (1:1, v/v), isopropyl acetate, or methyl tert-butyl ether;

or, in the preparation method for the crystal form A of the oxalate salt, the preparation method for the crystal form A of the oxalate salt satisfies one or more of the following conditions:

(1) the molar ratio of the oxalic acid to the compound of Formula I is (1.0-1.5):1; preferably 1.0:1;
(2) in the preparation method for the crystal form A of the oxalate salt, in the mixed solvent of the alcohol solvent and water, the volume ratio of the alcohol solvent to water is (14-24):1, preferably 19:1;
(3) in the preparation method for the crystal form A of the oxalate salt, the solvent is a mixed solvent of IPA/$H_2O$ (19:1, v/v);

or, in the preparation method for the crystal form A of the maleate salt, the preparation method for the crystal form A of the maleate salt satisfies one or more of the following conditions:

(1) the molar ratio of the maleic acid to the compound of Formula I is (1.0-1.5):1; preferably 1.0:1;
(2) in the mixed solvent of the ketone solvent and the saturated alkane solvent, the volume ratio of the ketone solvent to the saturated alkane solvent is (0.5-2):1;
(3) the solvent is a mixed solvent of acetone/n-heptane (1:1, v/v), isopropyl acetate, or methyl tert-butyl ether;

or, in the preparation method for the crystal form A of the malate salt, the preparation method for the crystal form A of the malate salt satisfies one or more of the following conditions:

(1) the molar ratio of the malic acid to the compound of Formula I is (1.0-1.5):1; preferably 1.0:1;
(2) in the preparation method for the crystal form A of the malate salt, in the mixed solvent of the ketone solvent and the saturated alkane solvent, the volume ratio of the ketone solvent to the saturated alkane solvent is (0.5-2):1, preferably 1:1;
(3) the solvent is a mixed solvent of acetone/n-heptane (1:1, v/v);

or, in the preparation method for the crystal form A of the succinate salt, the preparation method for the crystal form A of the succinate salt satisfies one or two of the following conditions:

(1) the molar ratio of the succinic acid to the compound of Formula I is (1.0-1.5):1; preferably 1.0:1;
(2) the solvent is methyl *tert*-butyl ether;

or, in the preparation method for the crystal form A of the methanesulfonate salt, the preparation method for the crystal form A of the methanesulfonate salt satisfies one or more of the following conditions:

(1) the molar ratio of the methanesulfonic acid to the compound of Formula I is (1.0-1.2):1; preferably 1:1;
(2) in the mixed solvent of the ketone solvent and the saturated alkane solvent, the volume ratio of the ketone solvent to the saturated alkane solvent is (0.5-2):1, preferably 1:1;
(3) the solvent is a mixed solvent of acetone/n-heptane (1:1, v/v);

or, in the preparation method for the crystal form B of the methanesulfonate salt, the preparation method for the crystal form B of the methanesulfonate salt satisfies one or more of the following conditions:

(1) the molar ratio of the methanesulfonic acid to the compound of Formula I is (1.5-2.5):1; preferably 2:1;
(2) in the mixed solvent of the ketone solvent and the saturated alkane solvent, the volume ratio of the ketone solvent to the saturated alkane solvent is (0.5-2):1;
(3) the solvent is a mixed solvent of acetone/n-heptane (1:1, v/v), isopropyl acetate, or methyl tert-butyl ether;

or, the preparation method for the crystal form A of the 2-hydroxyethanesulfonate salt satisfies one or more of the following conditions:

(1) the molar ratio of the 2-hydroxyethanesulfonic acid to the compound of Formula I is (1.5-2.5):1; preferably 2:1;
(2) in the mixed solvent of the ketone solvent and the saturated alkane solvent, the volume ratio of the ketone solvent to the saturated alkane solvent is (0.5-2):1;
(3) the solvent is a mixed solvent of acetone/n-heptane (1:1, v/v) or isopropyl acetate.

**7.** A pharmaceutical composition comprising the crystal form or salt form of the compound of Formula I according to any one of claims 1-6, and at least one pharmaceutical excipient.

**8.** Use of a substance M, wherein the substance M is the crystal form or salt form of the compound of Formula I according to any one of claims 1-6, or the pharmaceutical composition according to claim 7,
the use comprises:

1) inhibiting tubulin polymerization and/or Src kinase;
2) preventing and/or treating a disease related to tubulin polymerization and/or Src kinase;
3) preparing a tubulin polymerization and/or Src kinase inhibitor;
4) preparing a medicament or formulation for preventing and/or treating a disease related to tubulin polymerization and/or Src kinase.

**9.** Use of a substance M in the preparation of a medicament for treating a tumor and/or a skin disease, wherein the substance M is the crystal form or salt form of the compound of Formula I according to any one of claims 1-6, or the pharmaceutical composition according to claim 7.

**10.** The use according to claim 9, wherein the tumor comprises: solid tumor, sarcoma, and hematological cancer; preferably, the tumor comprises: breast cancer, ovarian cancer, prostate cancer, cervical cancer, testicular cancer, colon cancer, colorectal cancer, liver cancer, non-small cell lung cancer, squamous cell carcinoma (such as cutaneous squamous cell carcinoma), small cell lung cancer, gastric cancer, gastrointestinal stromal tumor, pancreatic cancer, bladder cancer, germ cell tumor, mast cell tumor, mastocytosis, glioblastoma, neuroblastoma, astrocytoma, melanoma, B-cell lymphoma, T-cell lymphoma, slowly progressive lymphoma, Hodgkin lymphoma, non-Hodgkin lymphoma, acute myeloid leukemia, acute lymphoblastic leukemia, chronic myeloid leukemia, chronic lymphocytic leukemia, myeloma, and/or myelodysplastic syndrome;
the skin disease comprises: actinic keratosis, psoriasis, atopic dermatitis, psoriasis, vitiligo, roseola, and/or systemic lupus erythematosus.

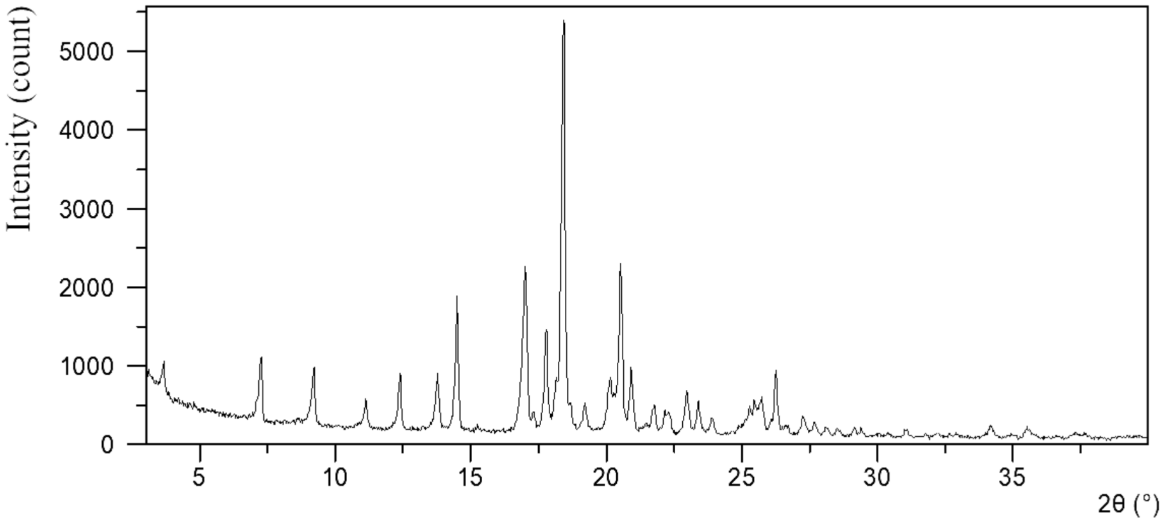
Intensity (count)
5000
4000
3000
2000
1000
0
5
10
15
20
25
30
35
2θ (°)

FIG. 1

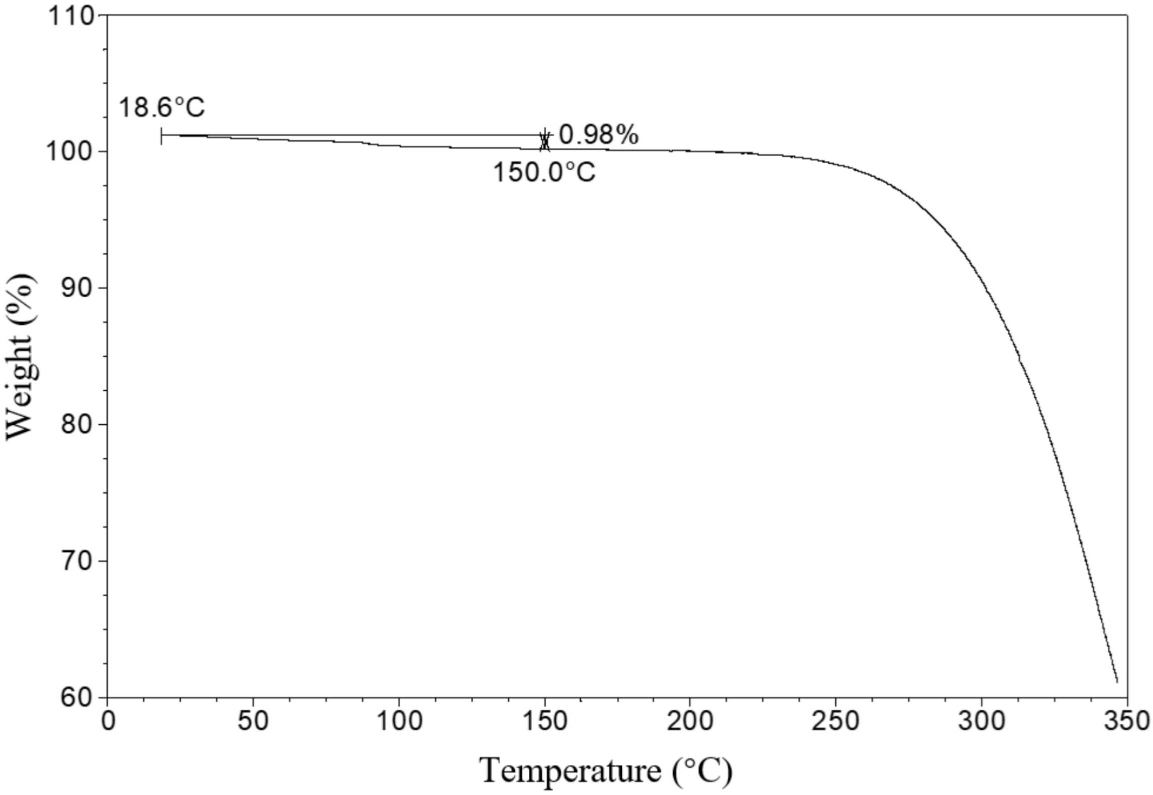
110
100
90
80
70
60
Weight (%)
18.6°C
0.98%
150.0°C
0
50
100
150
200
250
300
350
Temperature (°C)

FIG. 2

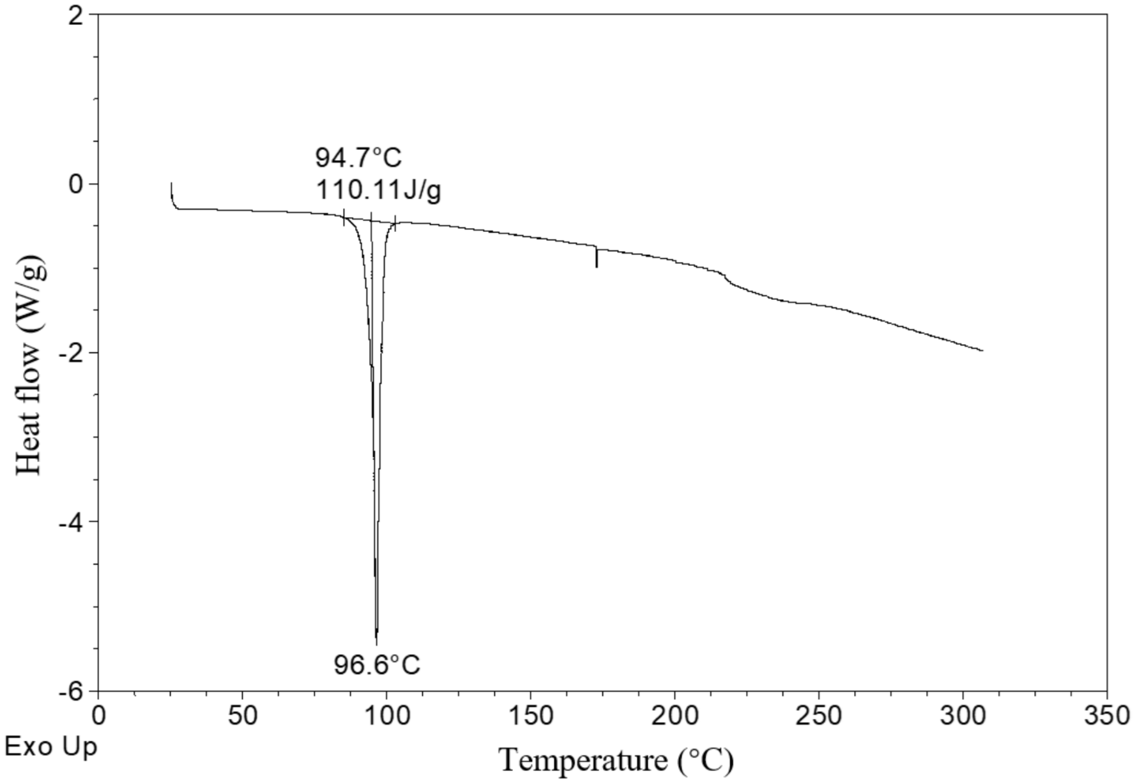
2
0
-2
-4
-6
0
50
100
150
200
250
300
350
94.7°C
110.11J/g
96.6°C
Heat flow (W/g)
Exo Up
Temperature (°C)

FIG. 3

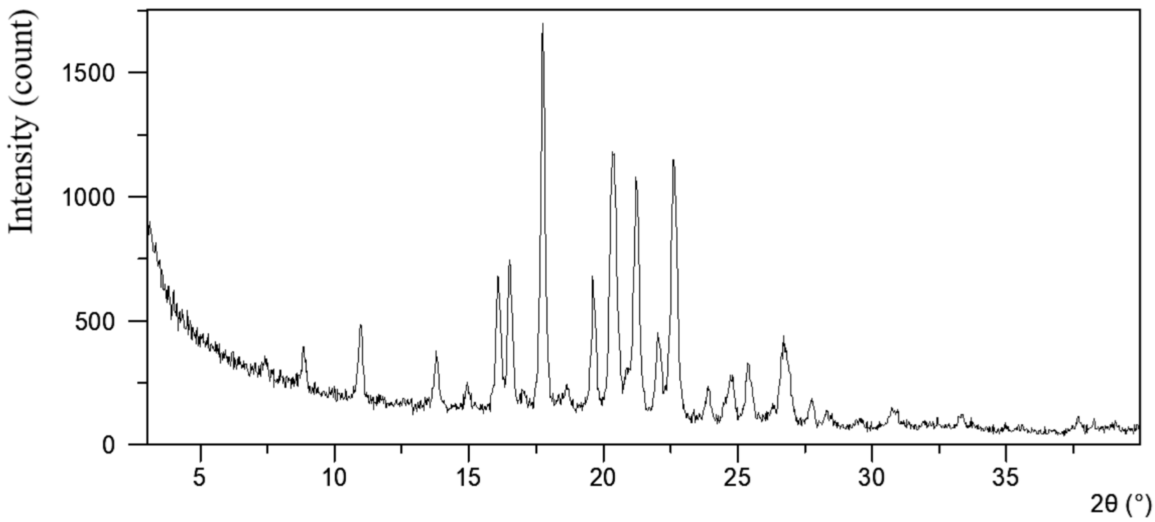
1500
1000
500
0
5
10
15
20
25
30
35
Intensity (count)
2θ (°)

FIG. 4

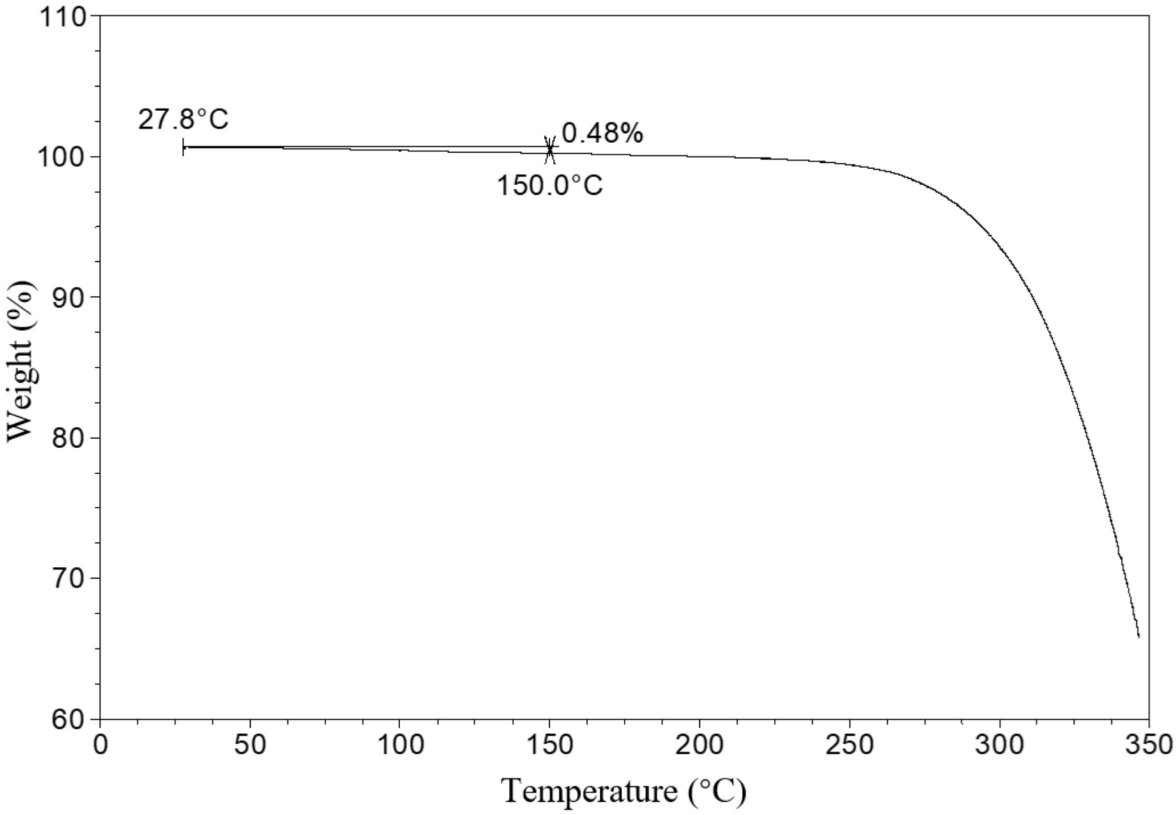
110
100
90
80
70
60
Weight (%)
0
50
100
150
200
250
300
350
Temperature (°C)
27.8°C
0.48%
150.0°C

FIG. 5

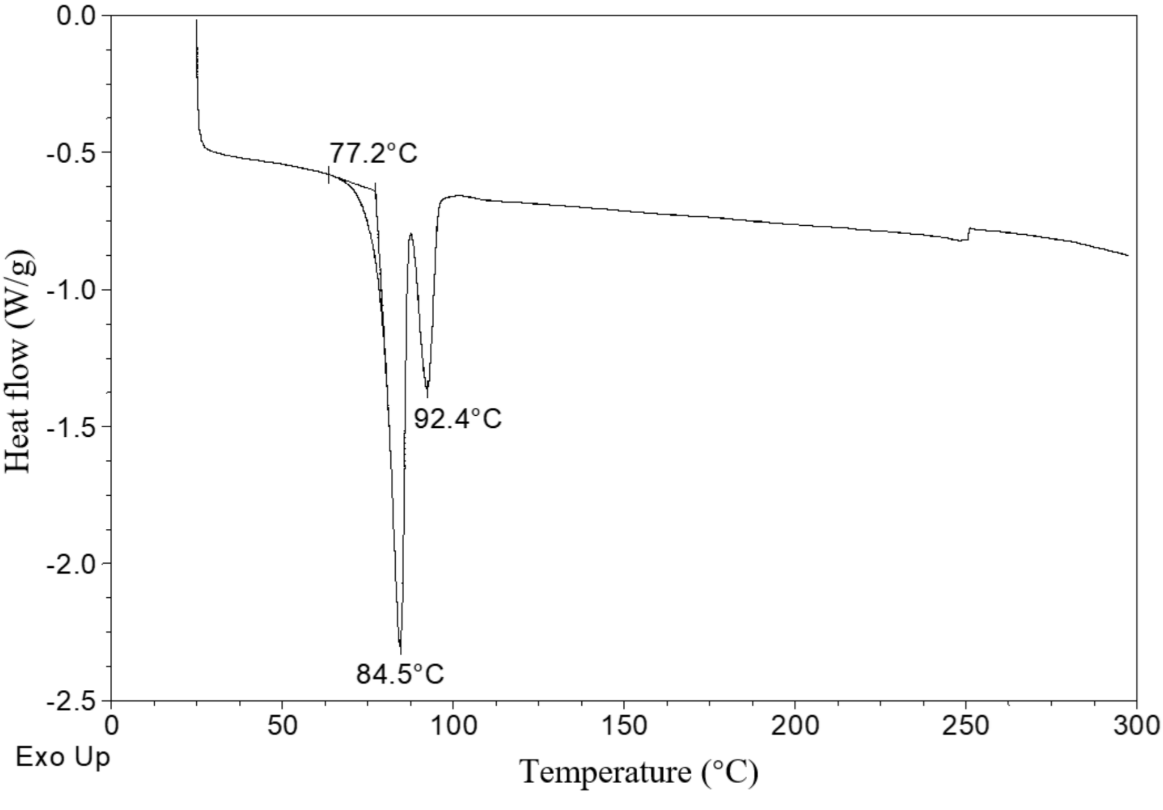
0.0
-0.5
-1.0
-1.5
-2.0
-2.5
Heat flow (W/g)
0
50
100
150
200
250
300
Exo Up
Temperature (°C)
77.2°C
92.4°C
84.5°C

FIG. 6

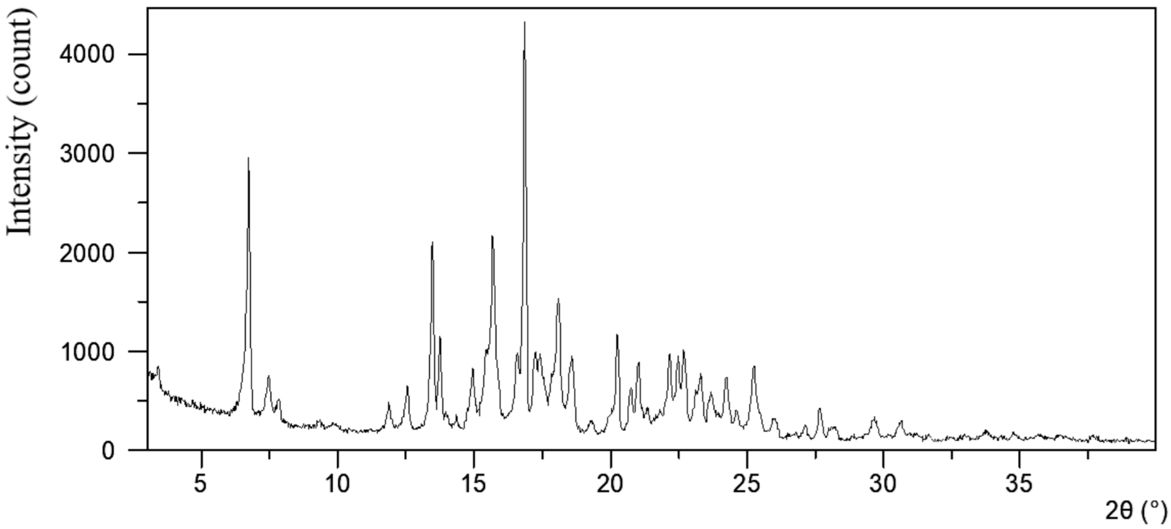
Intensity (count)
4000
3000
2000
1000
0
5
10
15
20
25
30
35
2θ (°)

FIG. 7

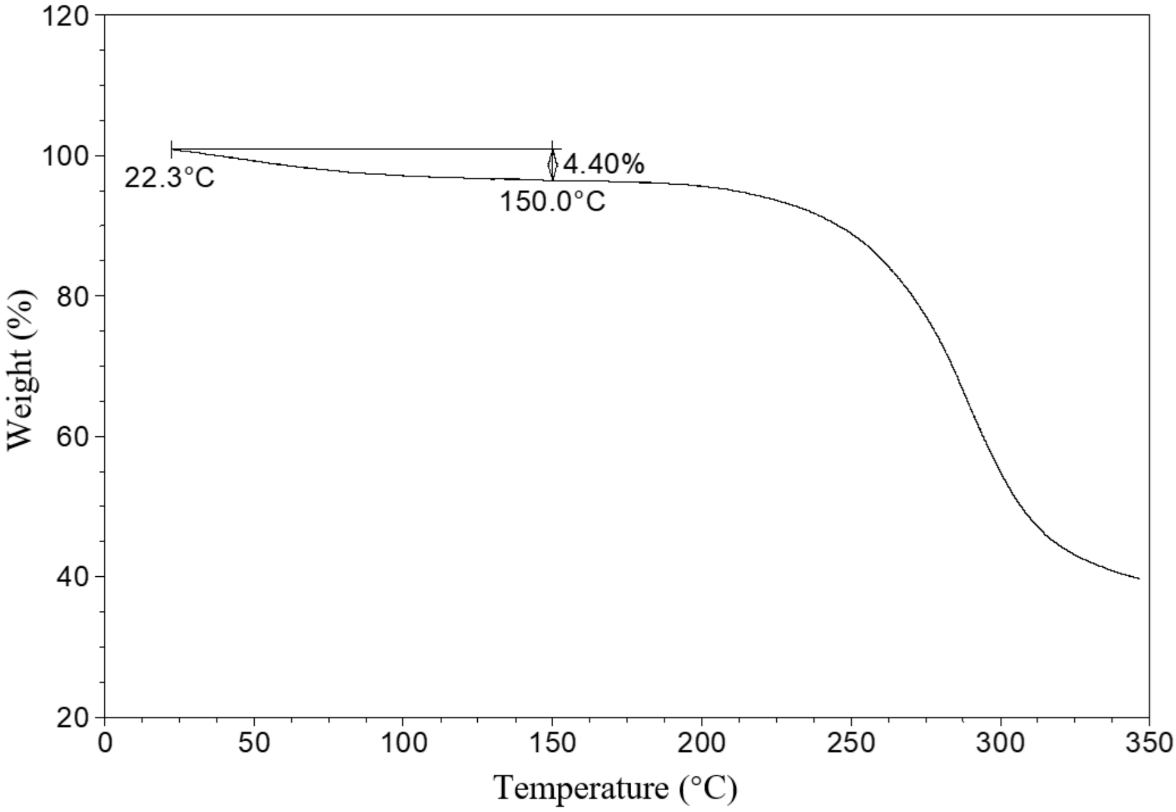
120
100
80
60
40
20
Weight (%)
22.3°C
4.40%
150.0°C
0
50
100
150
200
250
300
350
Temperature (°C)

FIG. 8

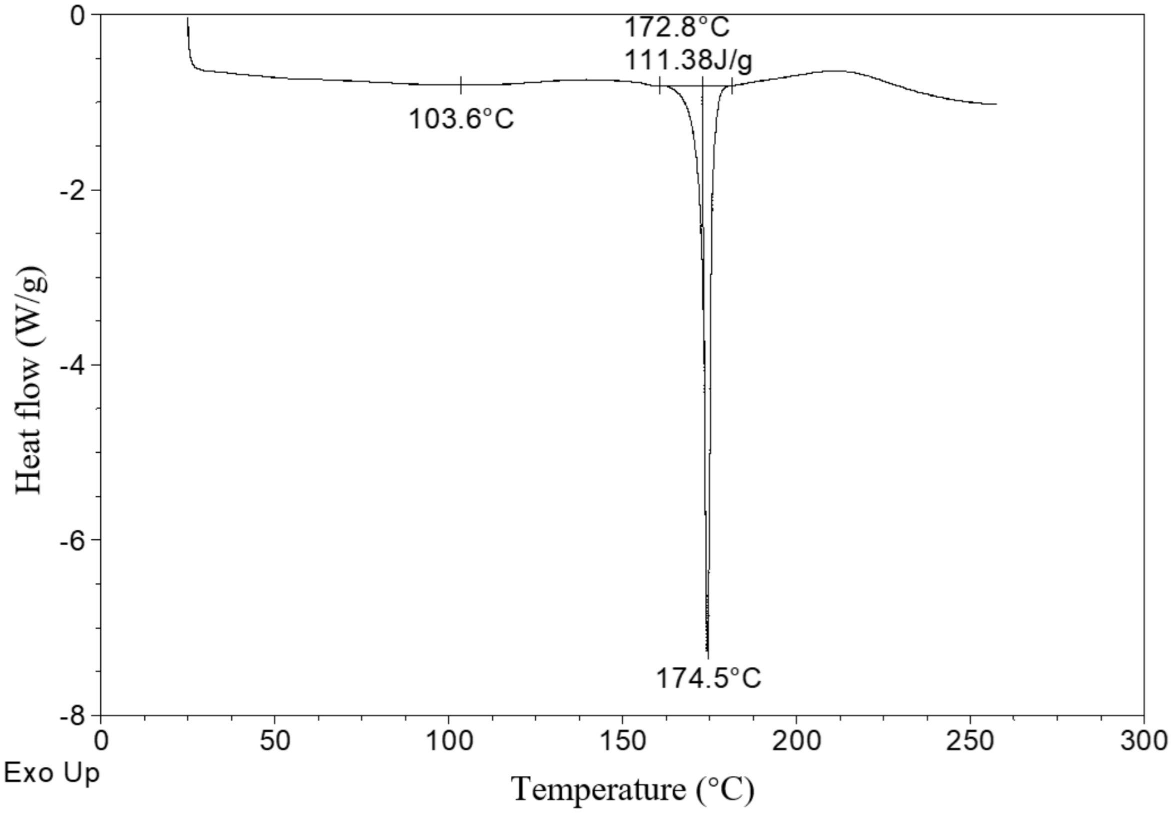
0
-2
-4
-6
-8
Heat flow (W/g)
172.8°C
111.38J/g
103.6°C
174.5°C
0
50
100
150
200
250
300
Exo Up
Temperature (°C)

FIG. 9

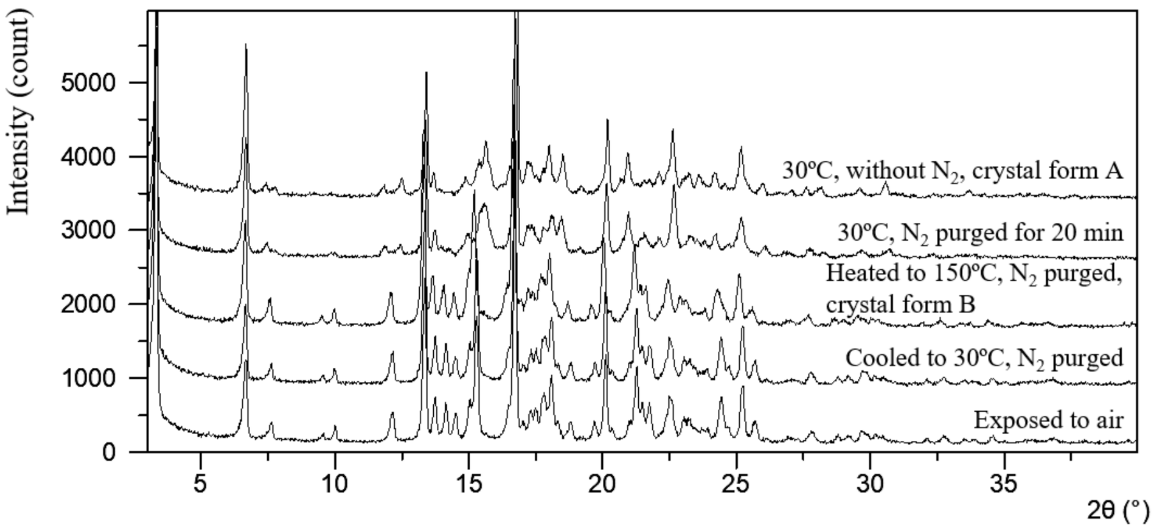
Intensity (count)
5000
4000
3000
2000
1000
0
30ºC, without $N_2$, crystal form A
30ºC, $N_2$ purged for 20 min
Heated to 150ºC, $N_2$ purged,
crystal form B
Cooled to 30ºC, $N_2$ purged
Exposed to air
5
10
15
20
25
30
35
2θ (°)

FIG. 10

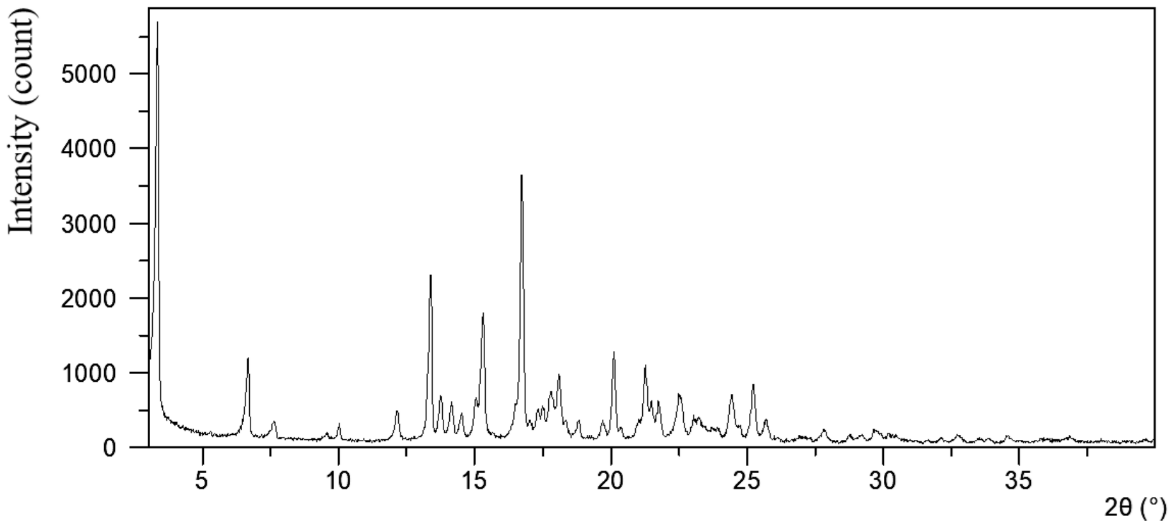
Intensity (count)
5000
4000
3000
2000
1000
0
5
10
15
20
25
30
35
2θ (°)

FIG. 11

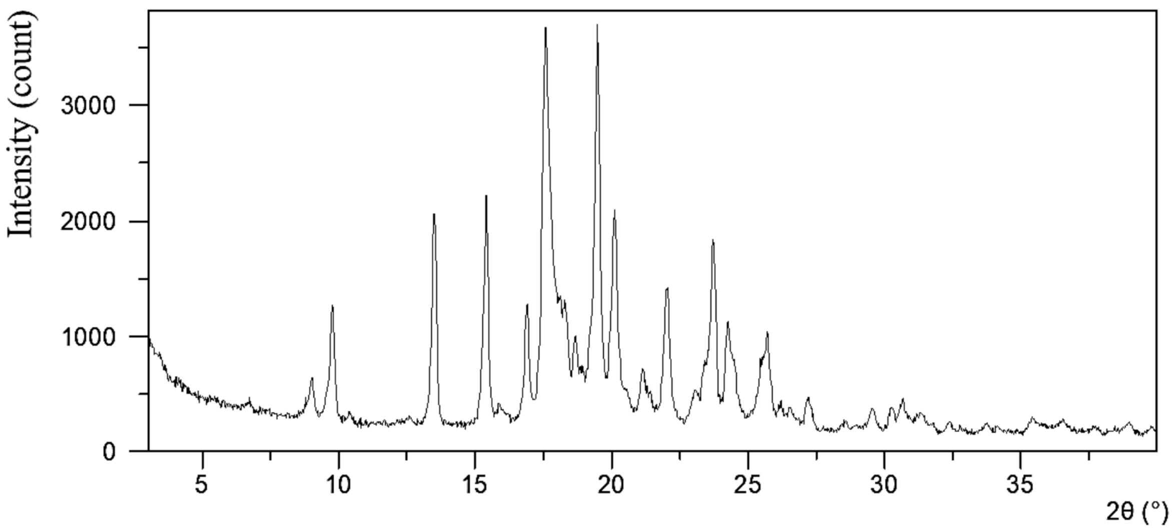
Intensity (count)
3000
2000
1000
0
5
10
15
20
25
30
35
2θ (°)

FIG. 12

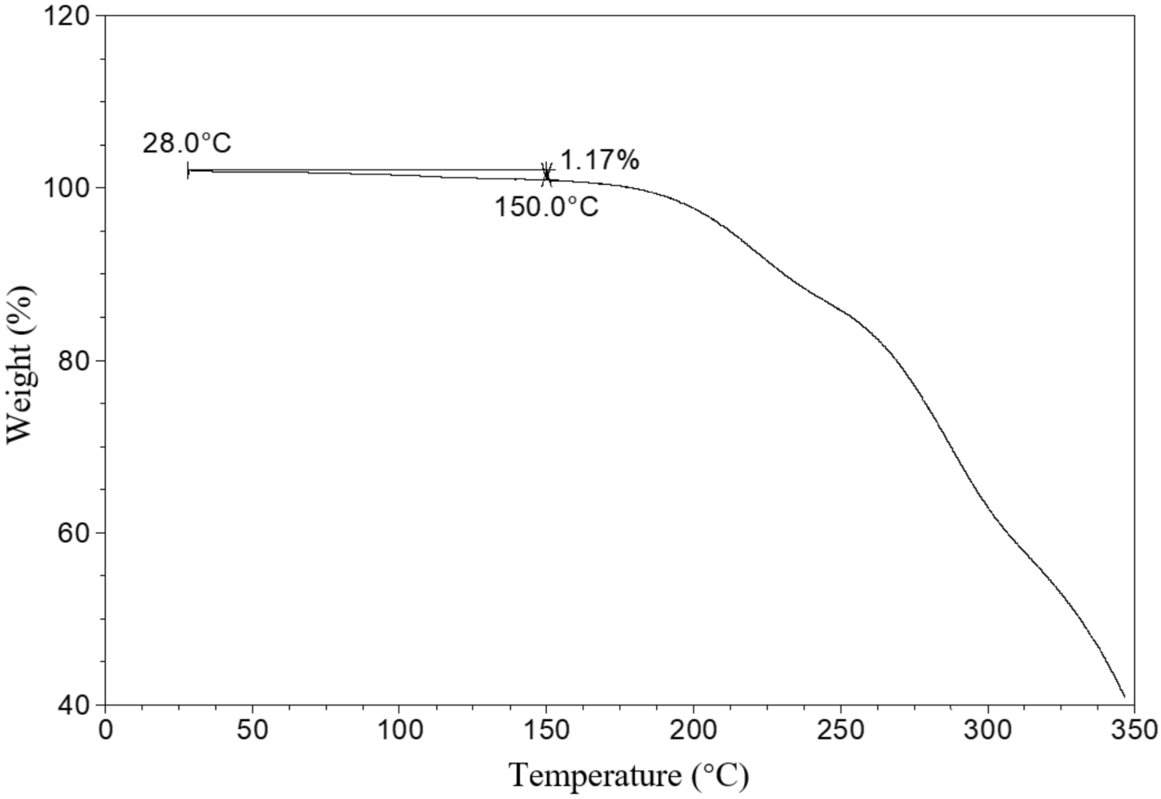
120
100
80
60
40
Weight (%)
28.0°C
1.17%
150.0°C
0
50
100
150
200
250
300
350
Temperature (°C)

FIG. 13

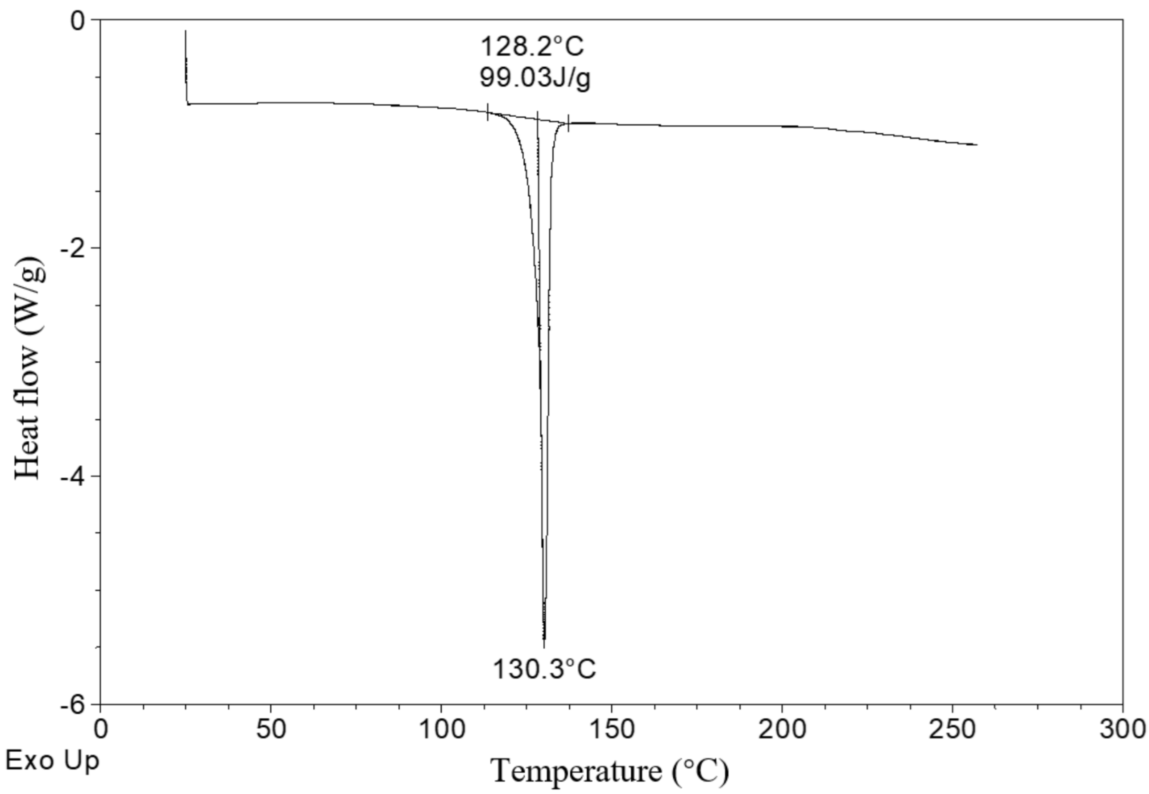
0
-2
-4
-6
Heat flow (W/g)
128.2°C
99.03J/g
130.3°C
0
50
100
150
200
250
300
Exo Up
Temperature (°C)

FIG. 14

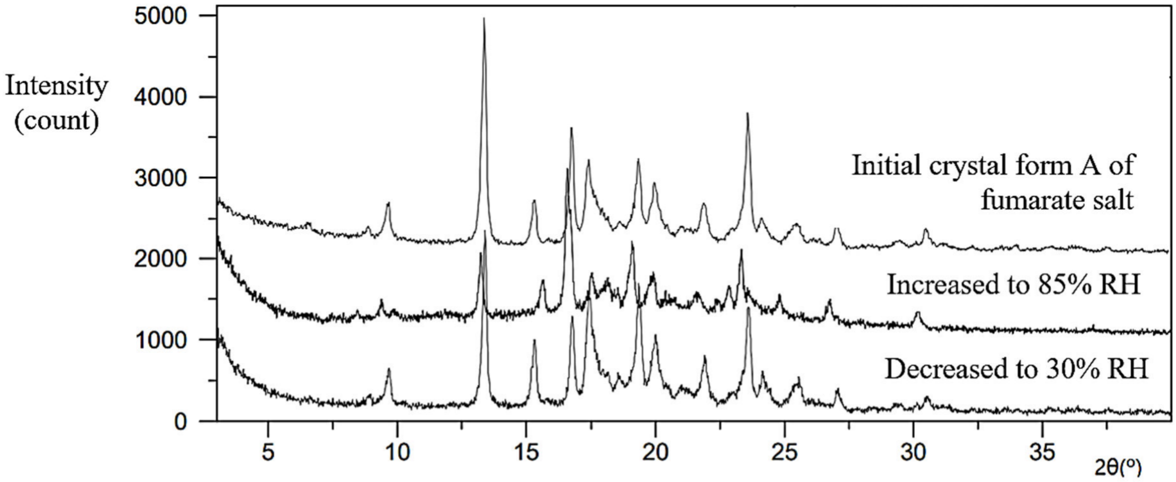
Intensity
(count)
5000
4000
3000
2000
1000
0
5
10
15
20
25
30
35
2θ(°)
Initial crystal form A of
fumarate salt
Increased to 85% RH
Decreased to 30% RH

FIG. 15

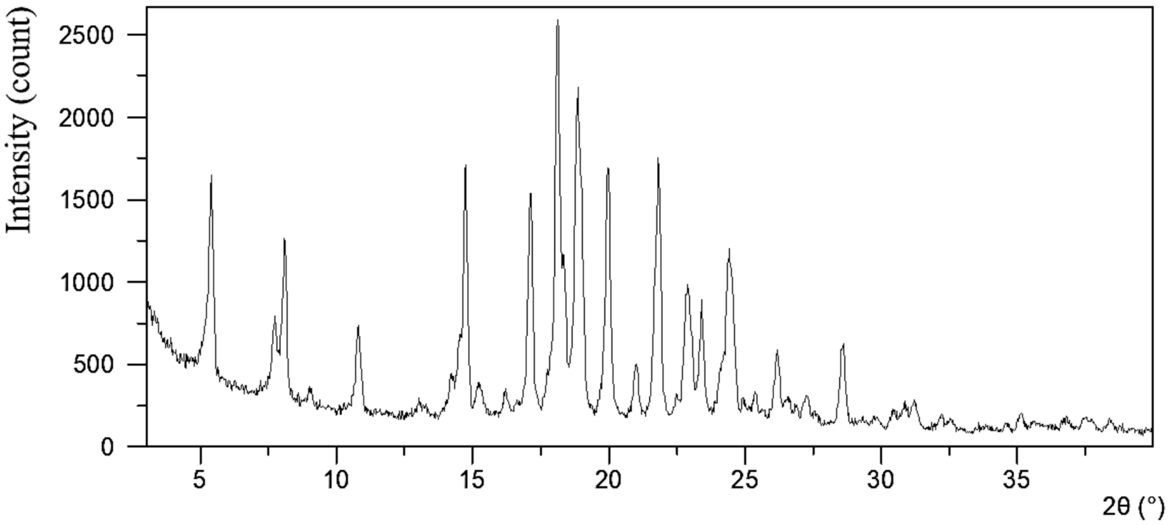
Intensity (count)
2500
2000
1500
1000
500
0
5
10
15
20
25
30
35
2θ (°)

FIG. 16

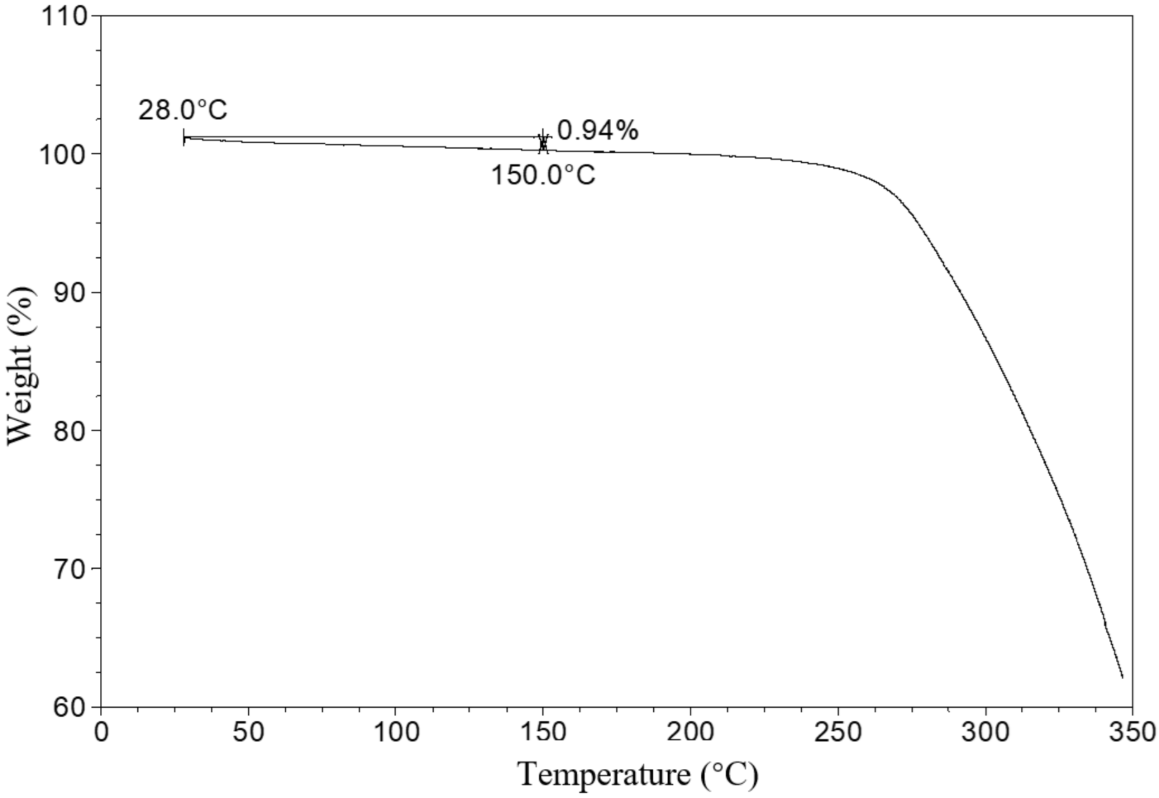
110
100
90
80
70
60
Weight (%)
28.0°C
0.94%
150.0°C
0
50
100
150
200
250
300
350
Temperature (°C)

FIG. 17

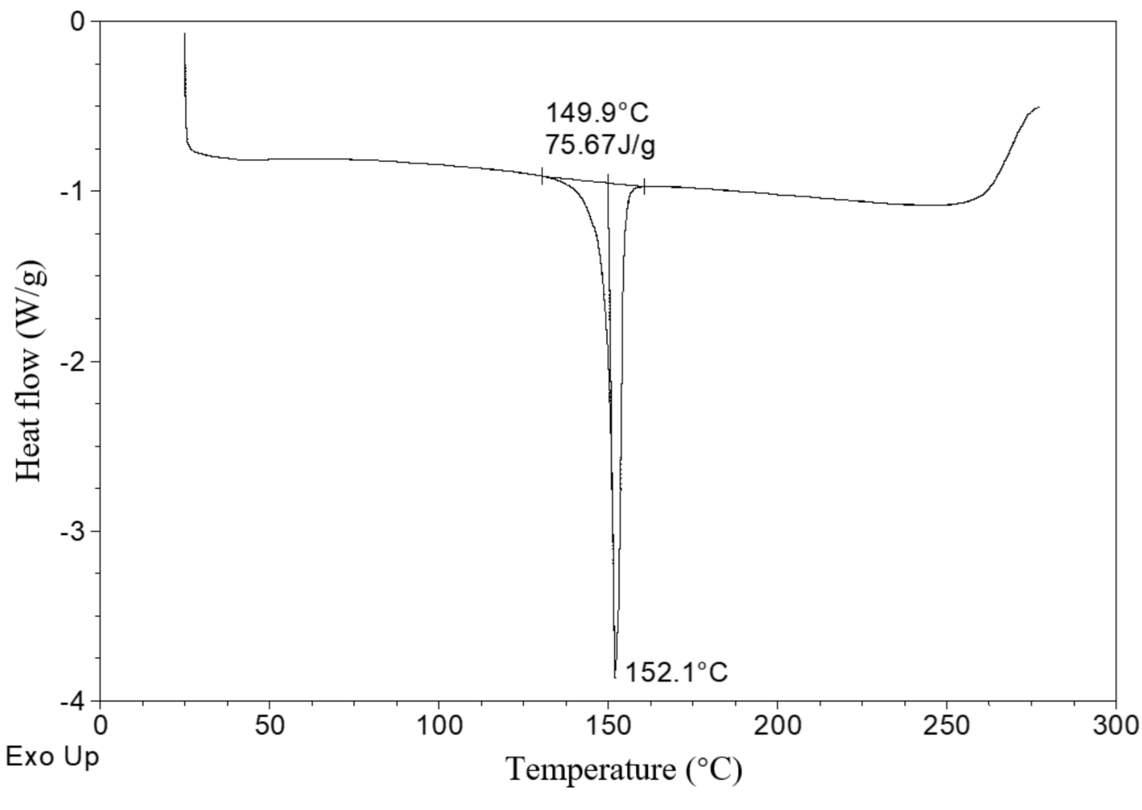
0
-1
-2
-3
-4
Heat flow (W/g)
149.9°C
75.67J/g
152.1°C
0
50
100
150
200
250
300
Exo Up
Temperature (°C)

FIG. 18

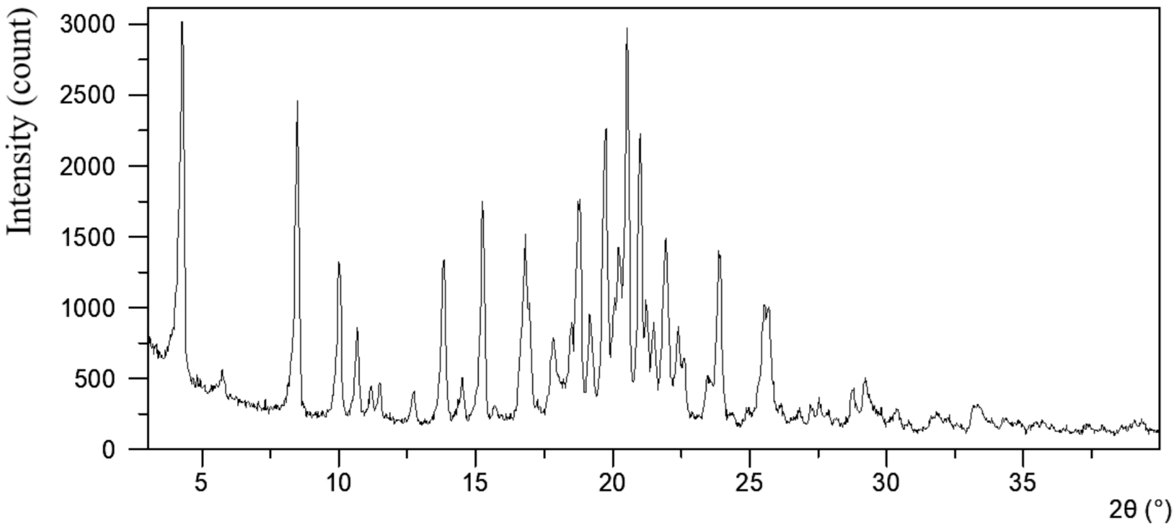
Intensity (count)
3000
2500
2000
1500
1000
500
0
5
10
15
20
25
30
35
2θ (°)

FIG. 19

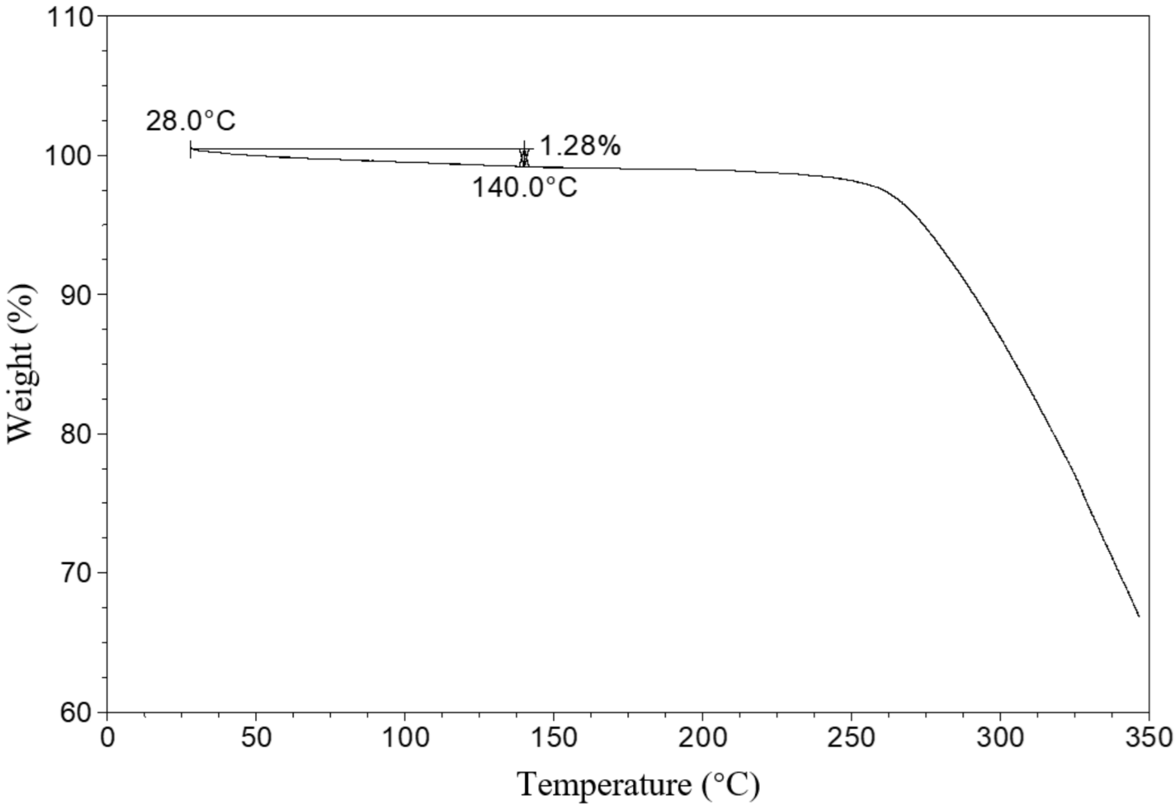
110
100
90
80
70
60
Weight (%)
28.0°C
1.28%
140.0°C
0
50
100
150
200
250
300
350
Temperature (°C)

FIG. 20

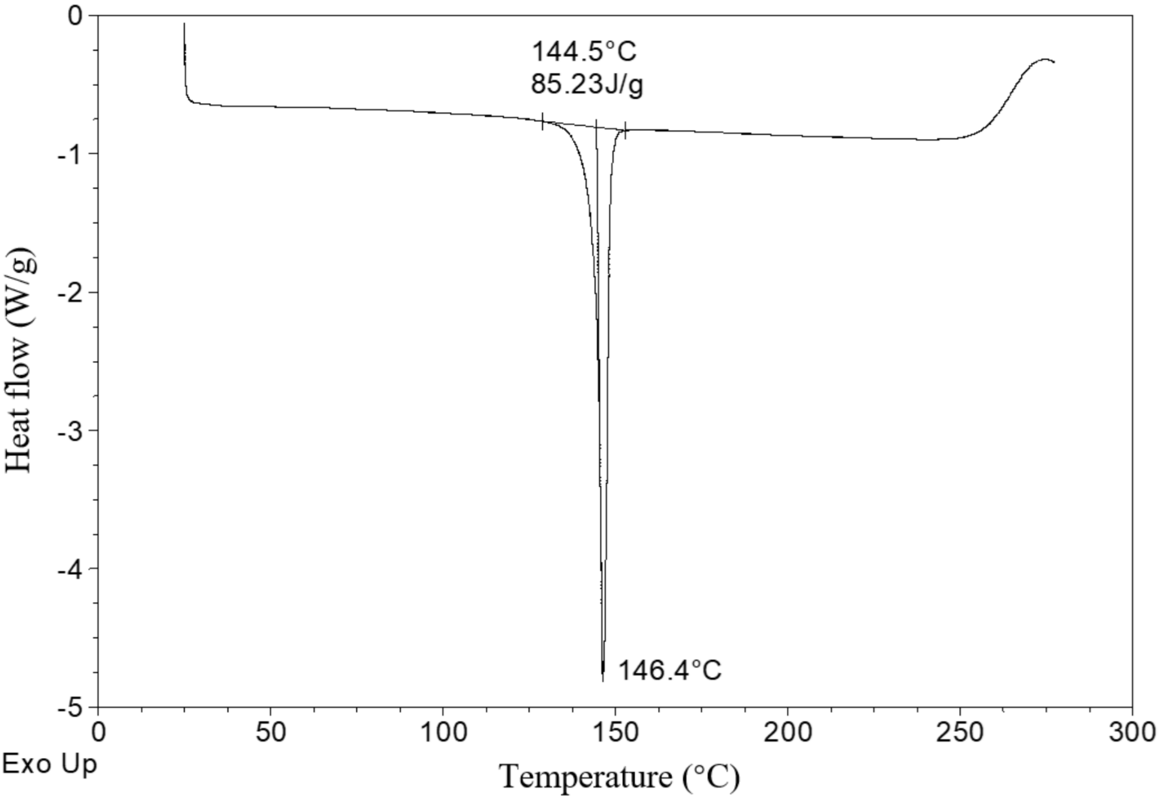
144.5°C
85.23J/g
146.4°C
Heat flow (W/g)
Temperature (°C)
Exo Up
0
-1
-2
-3
-4
-5
0
50
100
150
200
250
300

FIG. 21

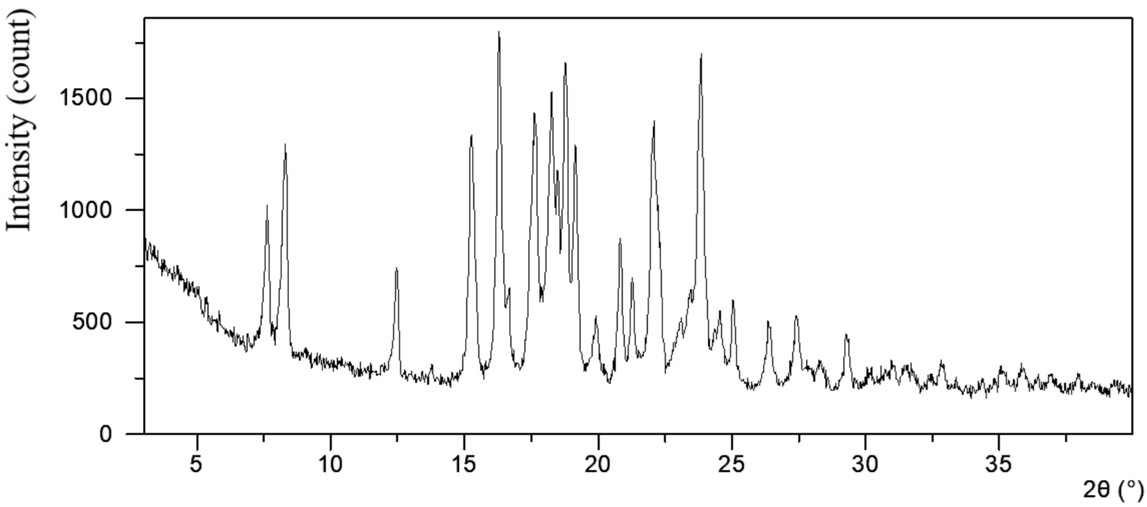
Intensity (count)
2θ (°)
0
500
1000
1500
5
10
15
20
25
30
35

FIG. 22

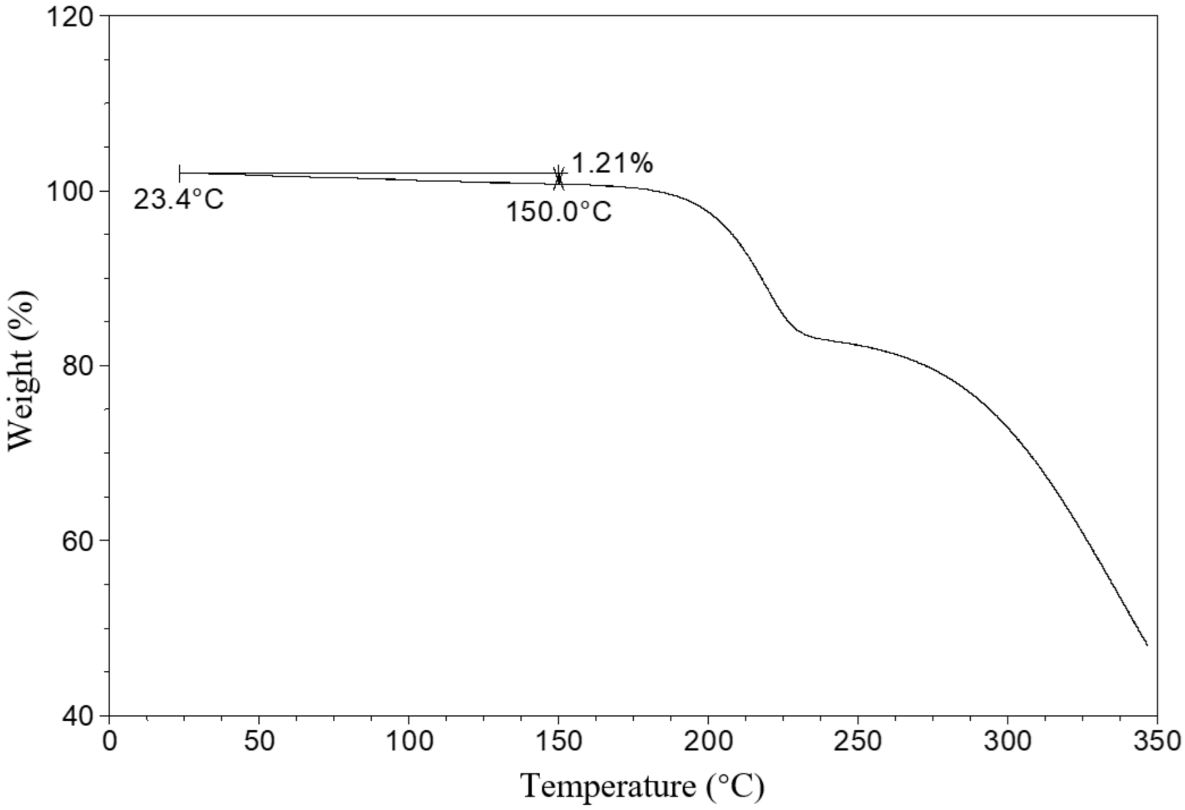
120
100
80
60
40
Weight (%)
1.21%
23.4°C
150.0°C
0
50
100
150
200
250
300
350
Temperature (°C)

FIG. 23

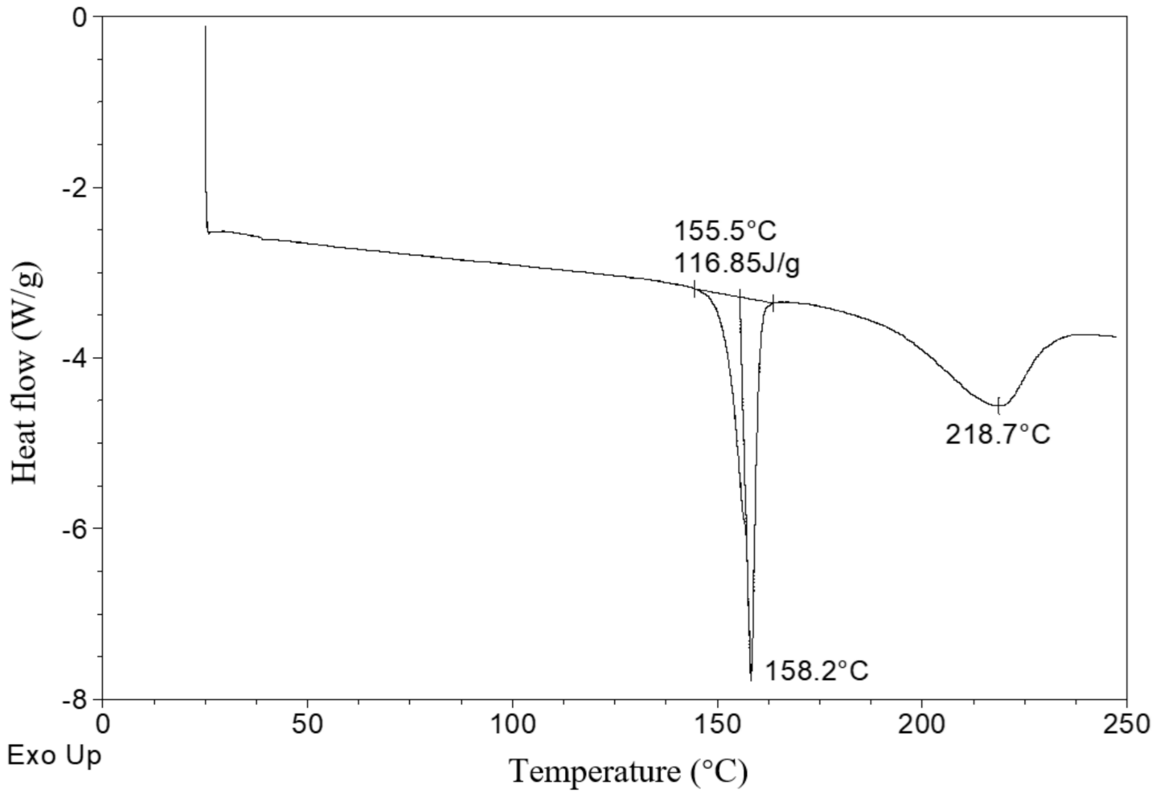
0
-2
-4
-6
-8
Heat flow (W/g)
155.5°C
116.85J/g
218.7°C
158.2°C
0
50
100
150
200
250
Exo Up
Temperature (°C)

FIG. 24

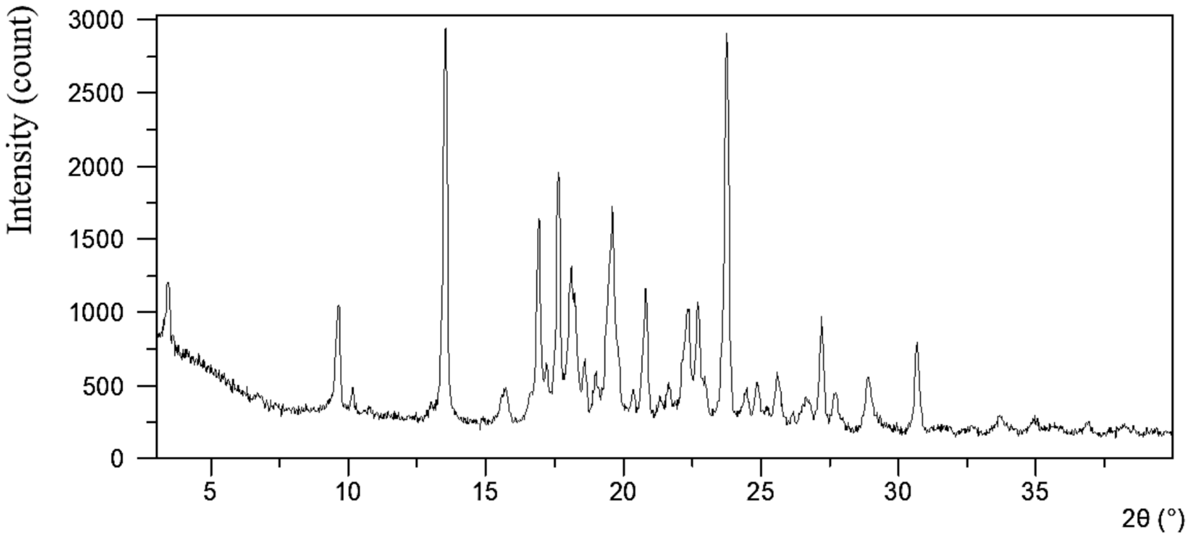
Intensity (count)
3000
2500
2000
1500
1000
500
0
5
10
15
20
25
30
35
2θ (°)

FIG. 25

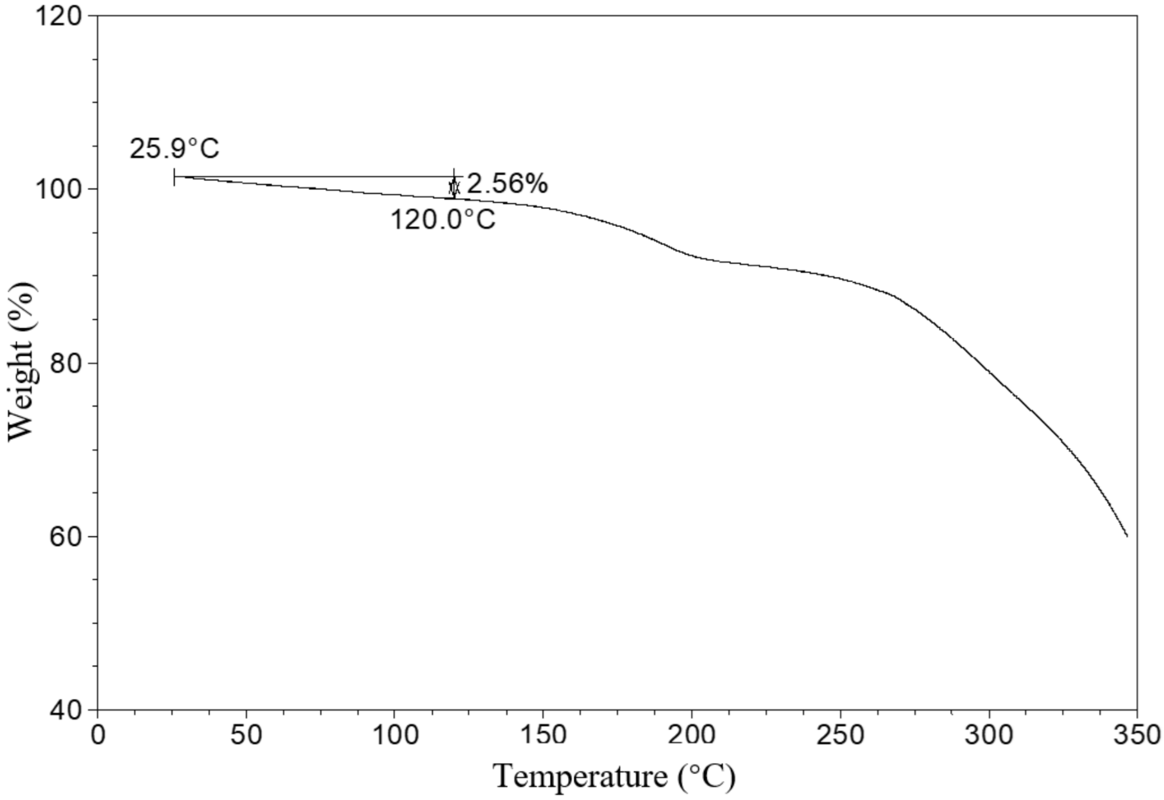
120
100
80
60
40
Weight (%)
25.9°C
2.56%
120.0°C
0
50
100
150
200
250
300
350
Temperature (°C)

FIG. 26

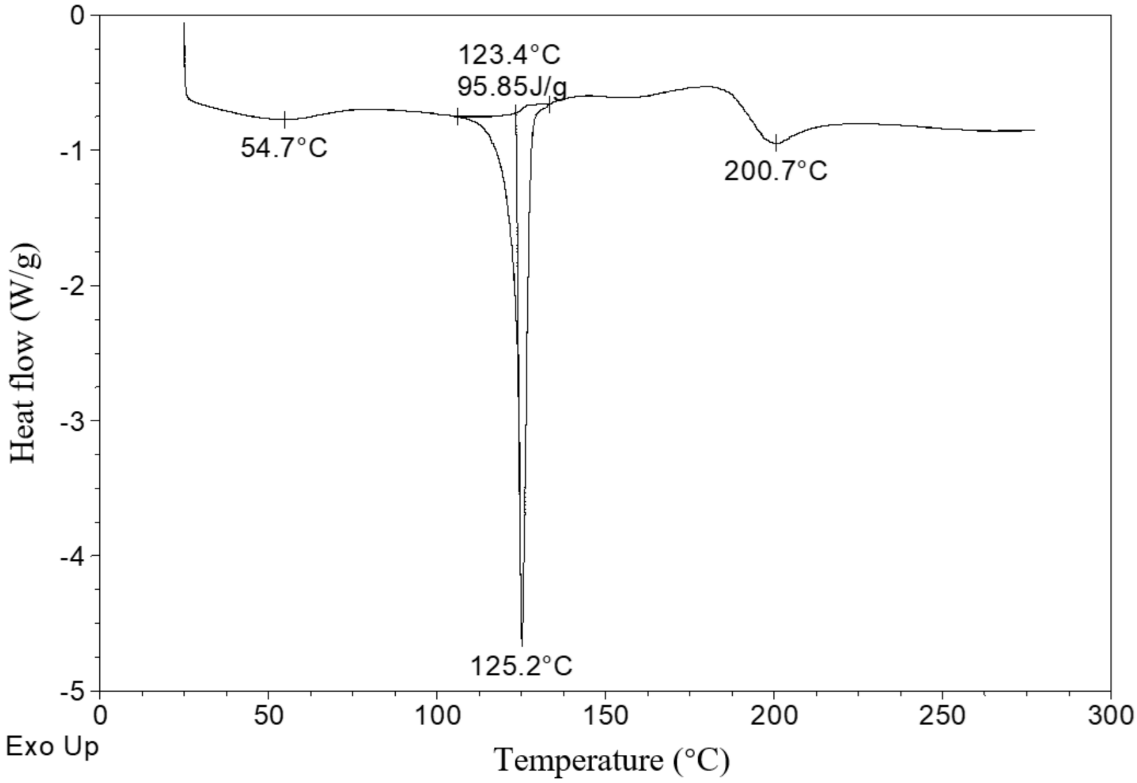
0
-1
-2
-3
-4
-5
Heat flow (W/g)
123.4°C
95.85J/g
54.7°C
200.7°C
125.2°C
0
50
100
150
200
250
300
Exo Up
Temperature (°C)

FIG. 27

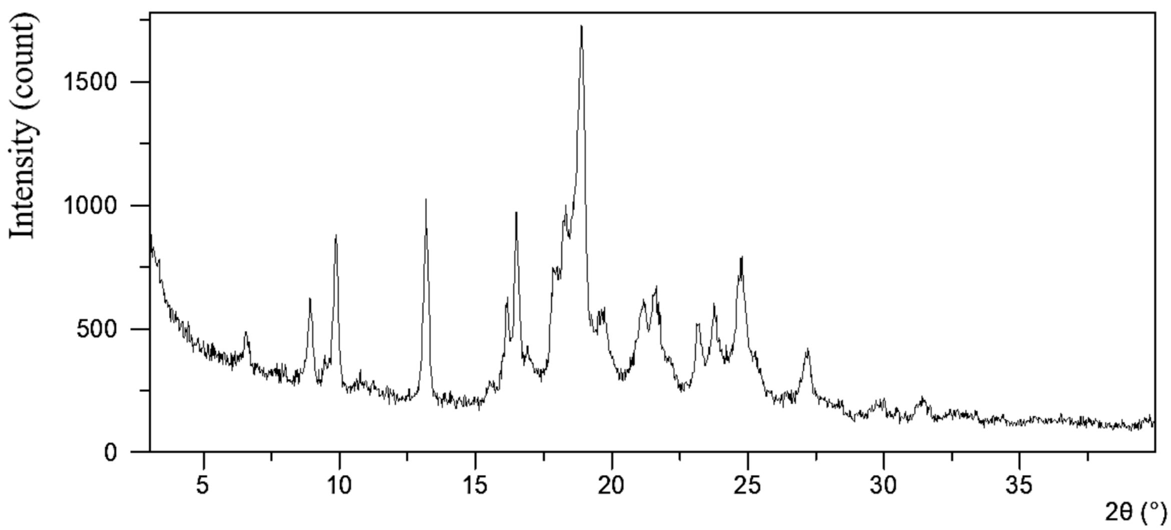
Intensity (count)
1500
1000
500
0
5
10
15
20
25
30
35
2θ (°)

FIG. 28

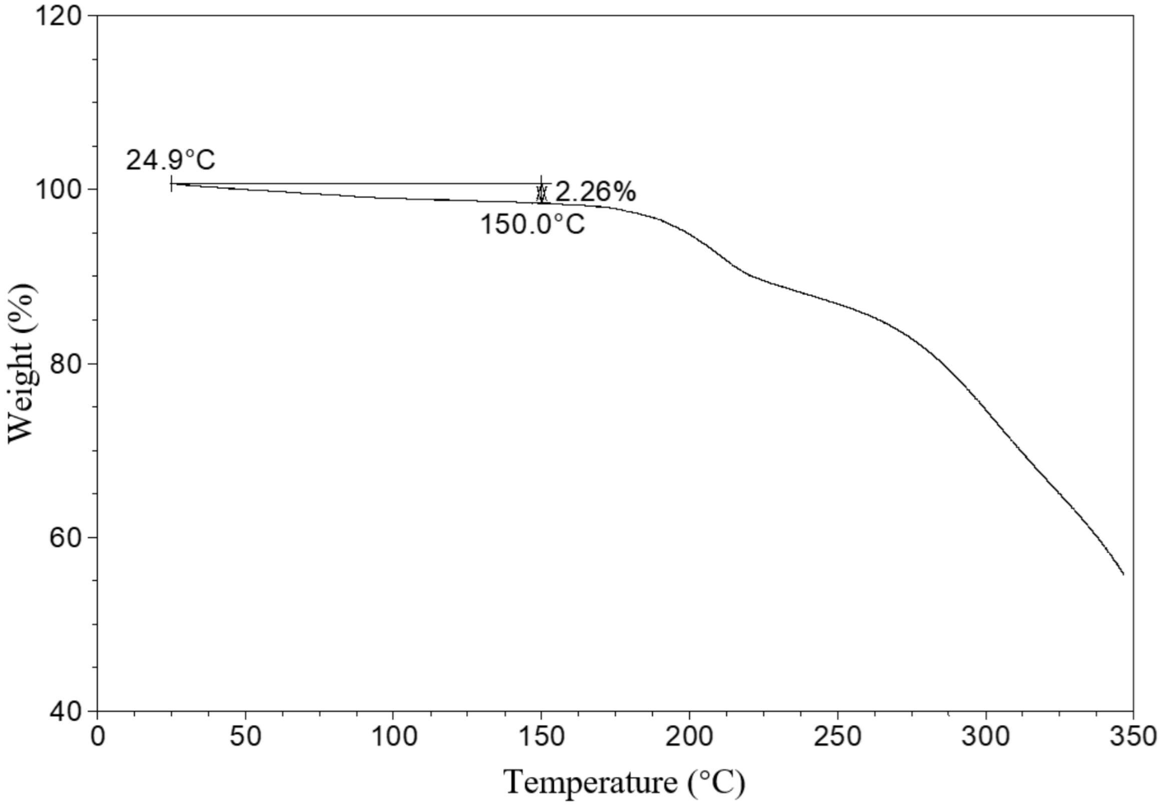
120
100
80
60
40
Weight (%)
24.9°C
2.26%
150.0°C
0
50
100
150
200
250
300
350
Temperature (°C)

FIG. 29

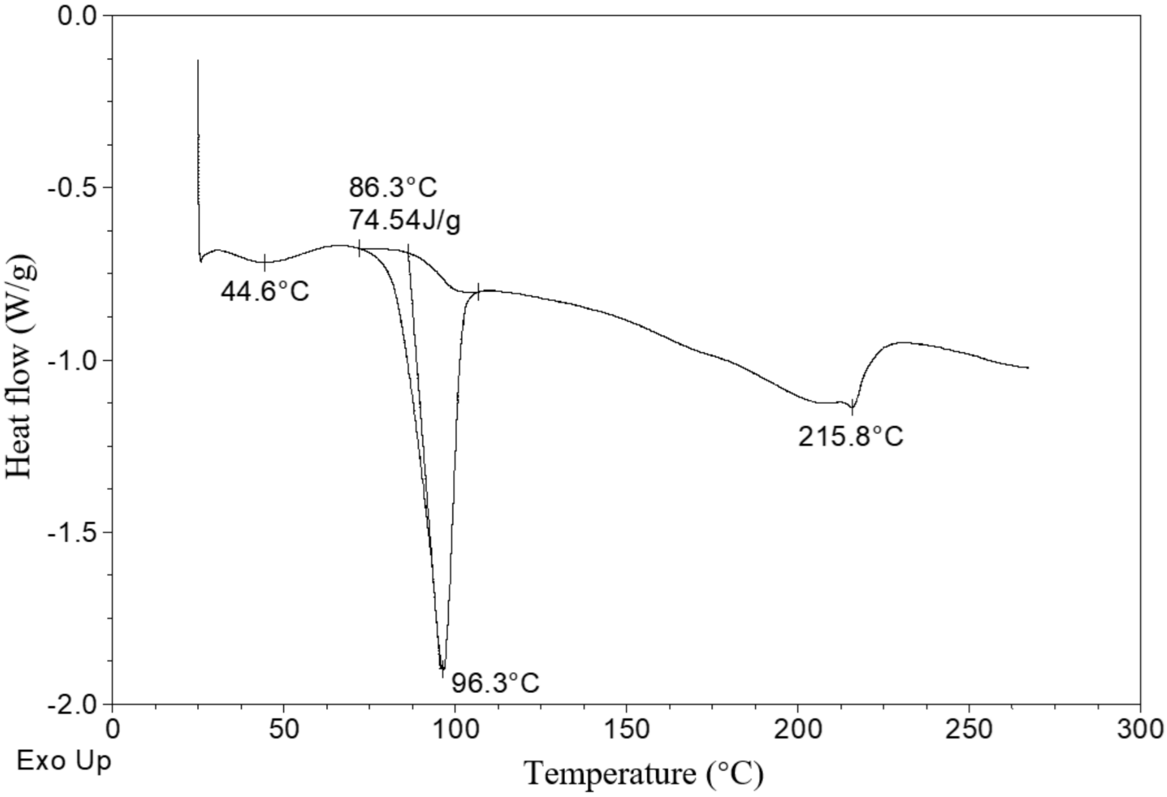
0.0
-0.5
-1.0
-1.5
-2.0
Heat flow (W/g)
86.3°C
74.54J/g
44.6°C
215.8°C
96.3°C
0
50
100
150
200
250
300
Exo Up
Temperature (°C)

FIG. 30

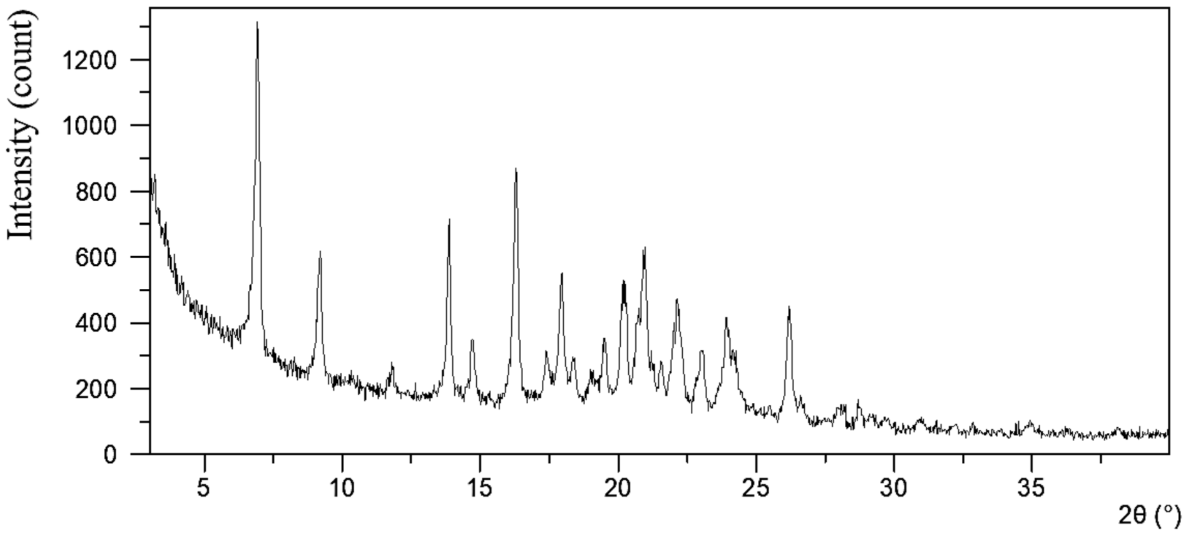
Intensity (count)
1200
1000
800
600
400
200
0
5
10
15
20
25
30
35
2θ (°)

FIG. 31

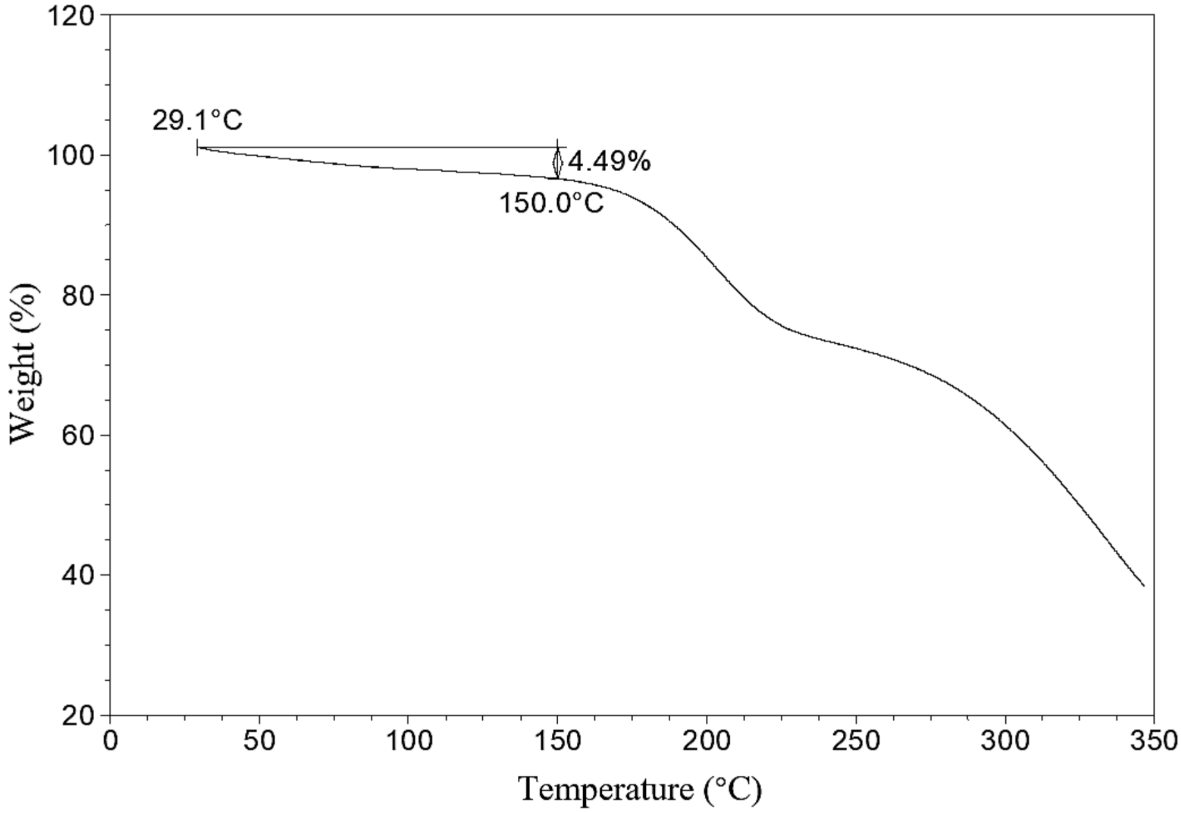
120
100
80
60
40
20
Weight (%)
29.1°C
4.49%
150.0°C
0
50
100
150
200
250
300
350
Temperature (°C)

FIG. 32

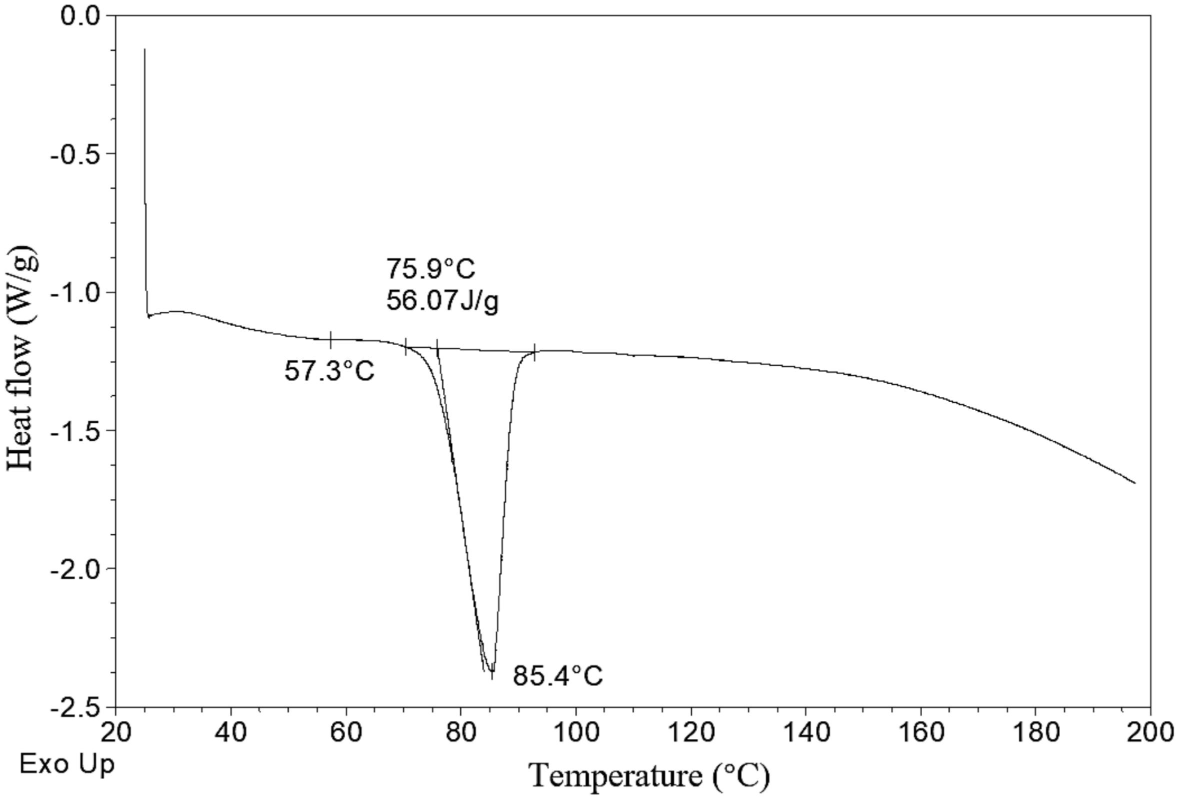
0.0
-0.5
-1.0
-1.5
-2.0
-2.5
Heat flow (W/g)
75.9°C
56.07J/g
57.3°C
85.4°C
20
40
60
80
100
120
140
160
180
200
Exo Up
Temperature (°C)

FIG. 33

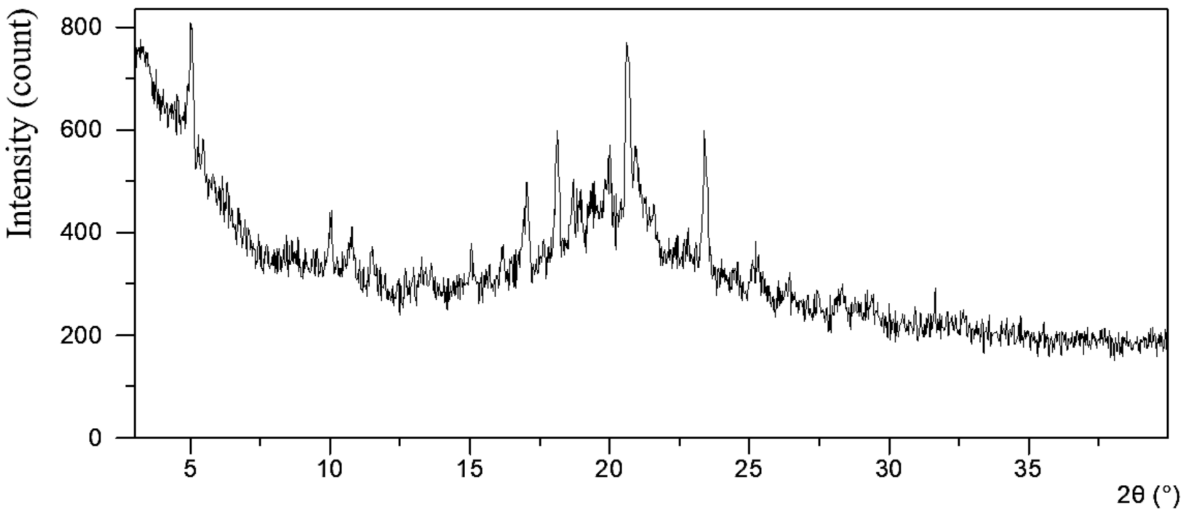
800
600
400
200
0
Intensity (count)
5
10
15
20
25
30
35
2θ (°)

FIG. 34

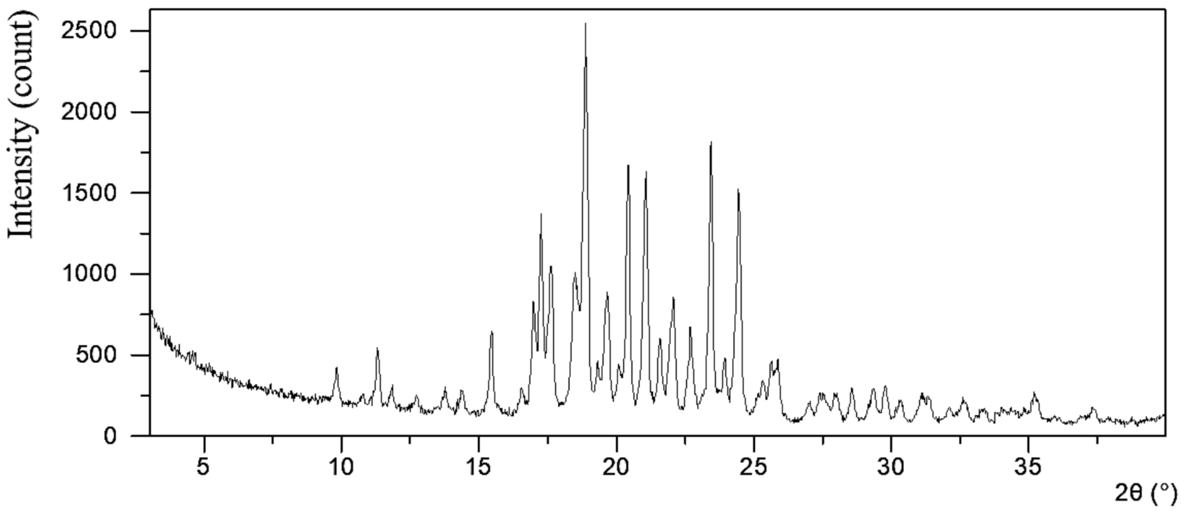
Intensity (count)
2500
2000
1500
1000
500
0
5
10
15
20
25
30
35
2θ (°)

FIG. 35

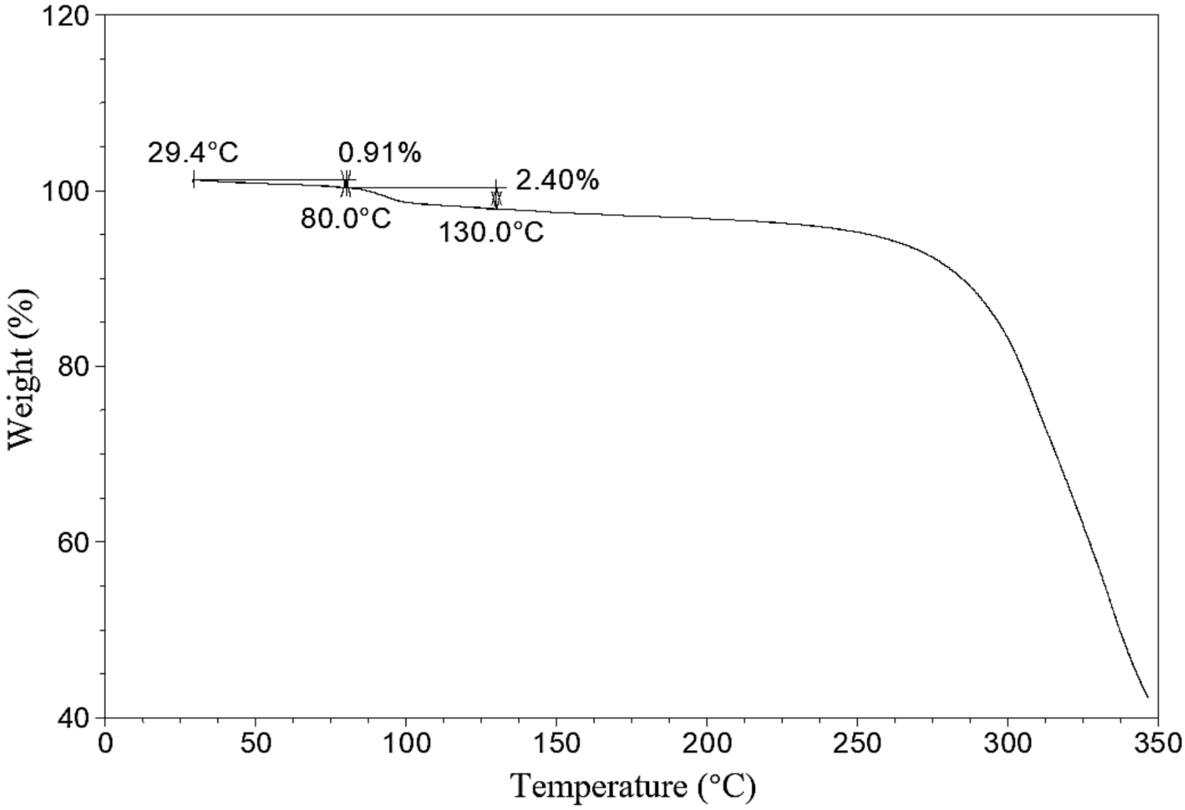
120
100
80
60
40
Weight (%)
29.4°C
0.91%
2.40%
80.0°C
130.0°C
0
50
100
150
200
250
300
350
Temperature (°C)

FIG. 36

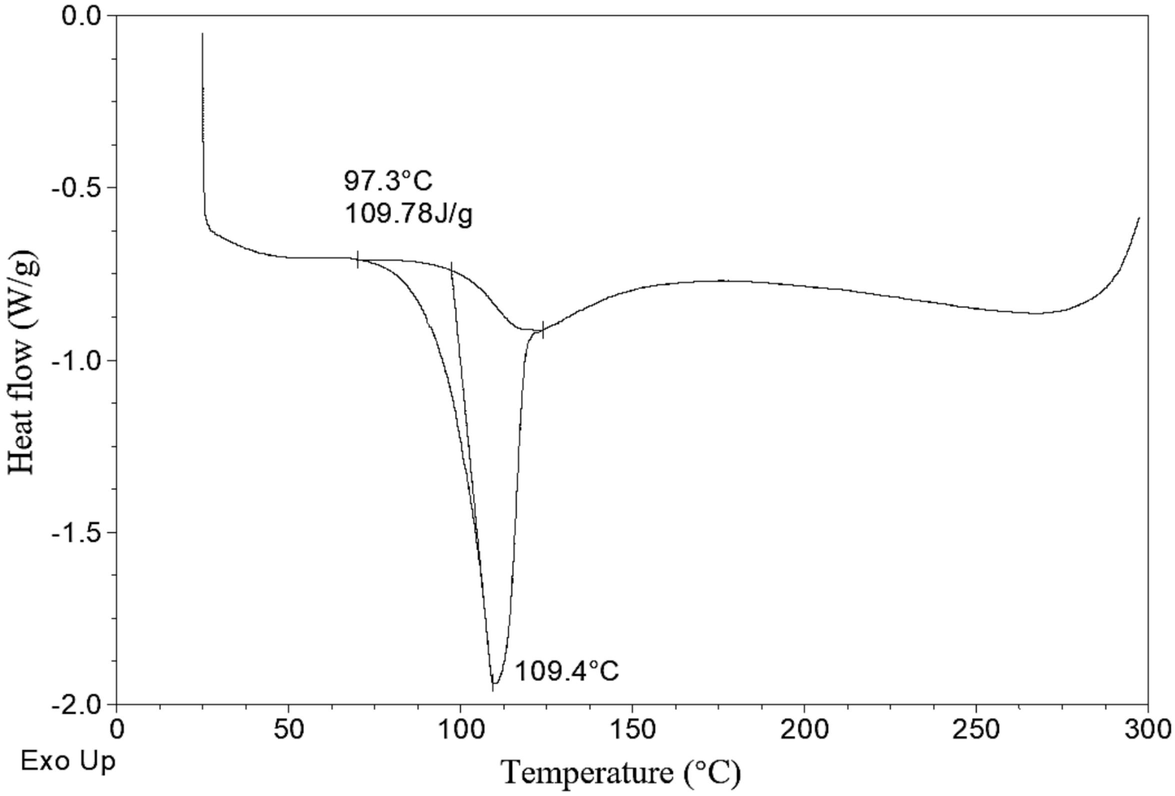
0.0
-0.5
-1.0
-1.5
-2.0
Heat flow (W/g)
97.3°C
109.78J/g
109.4°C
0
50
100
150
200
250
300
Exo Up
Temperature (°C)

FIG. 37

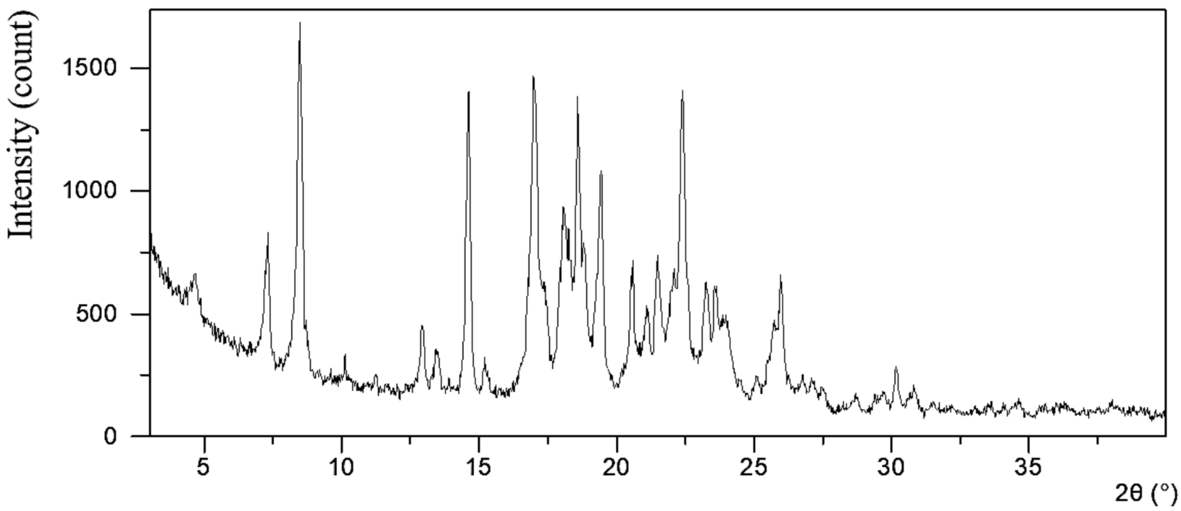
Intensity (count)
0
500
1000
1500
5
10
15
20
25
30
35
2θ (°)

FIG. 38

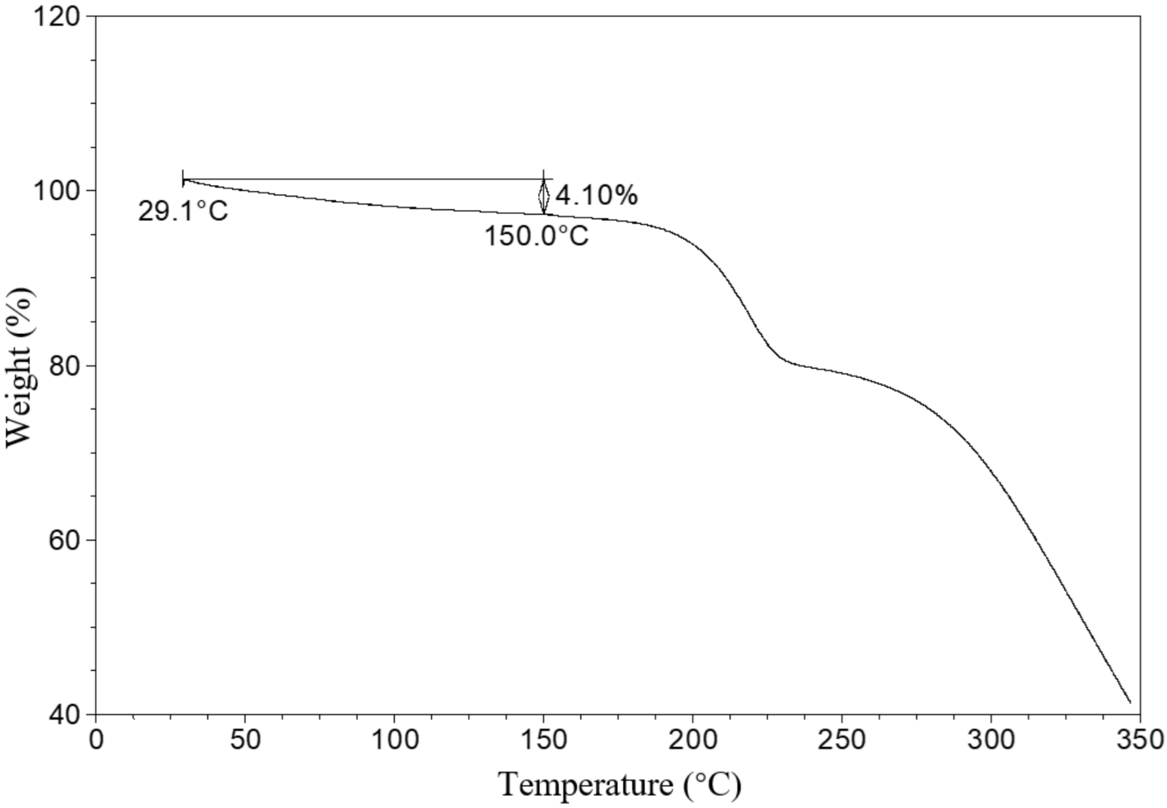
120
100
80
60
40
Weight (%)
29.1°C
4.10%
150.0°C
0
50
100
150
200
250
300
350
Temperature (°C)

FIG. 39

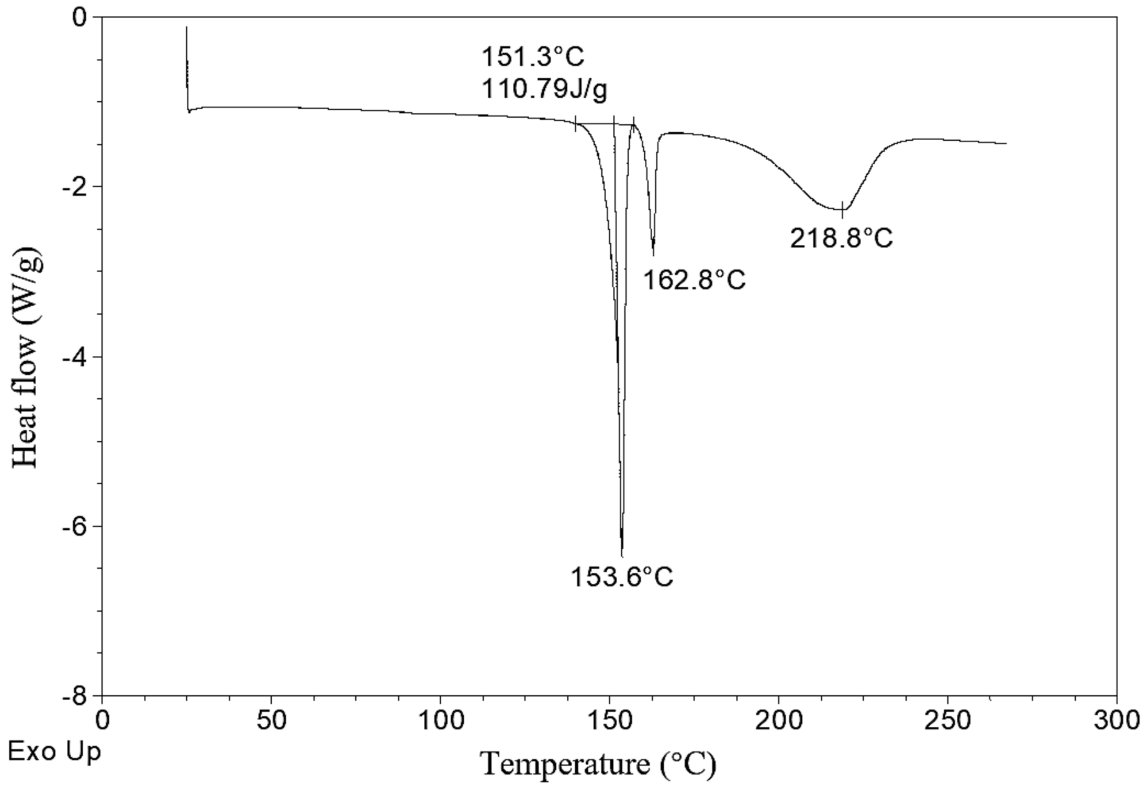
0
-2
-4
-6
-8
Heat flow (W/g)
151.3°C
110.79J/g
162.8°C
218.8°C
153.6°C
0
50
100
150
200
250
300
Exo Up
Temperature (°C)

FIG. 40

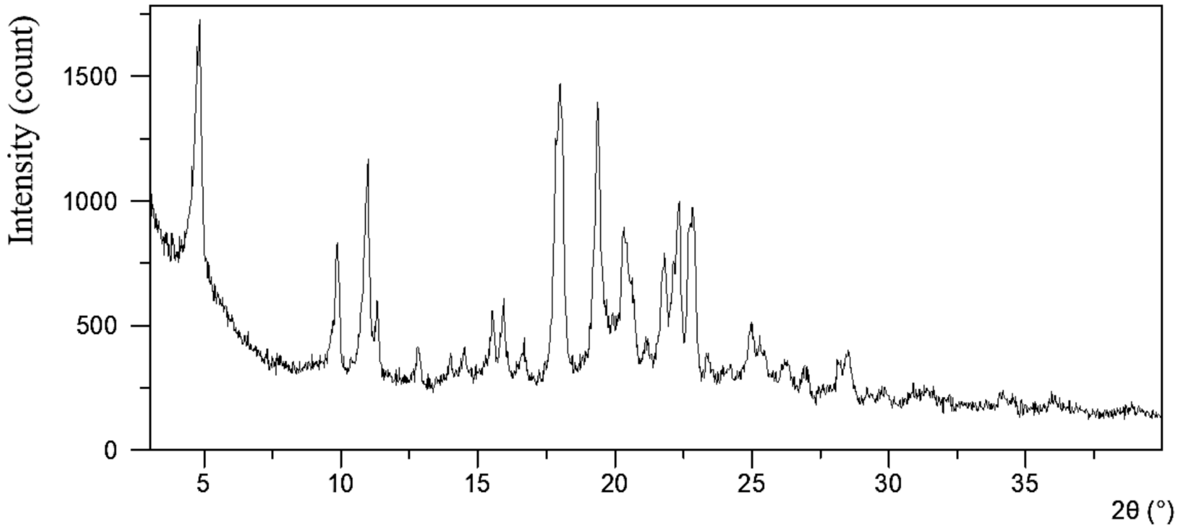
Intensity (count)
1500
1000
500
0
5
10
15
20
25
30
35
2θ (°)

FIG. 41

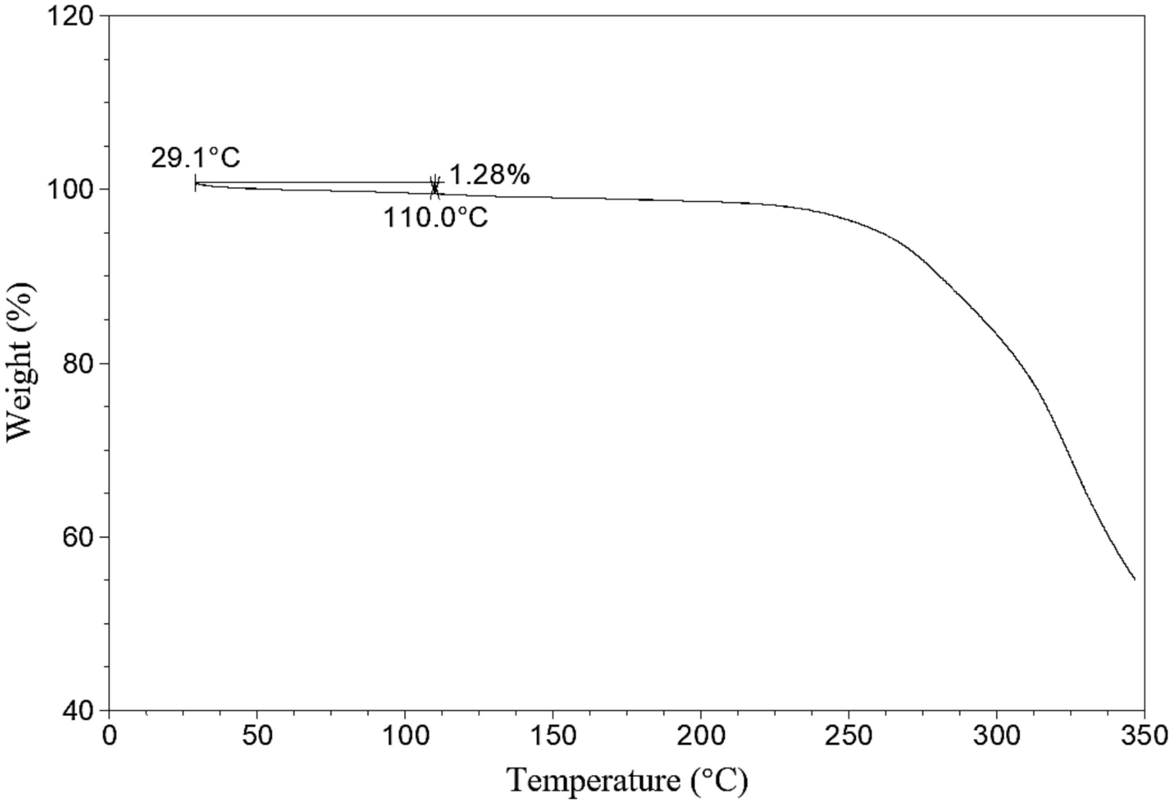
120
100
80
60
40
Weight (%)
29.1°C
1.28%
110.0°C
0
50
100
150
200
250
300
350
Temperature (°C)

FIG. 42

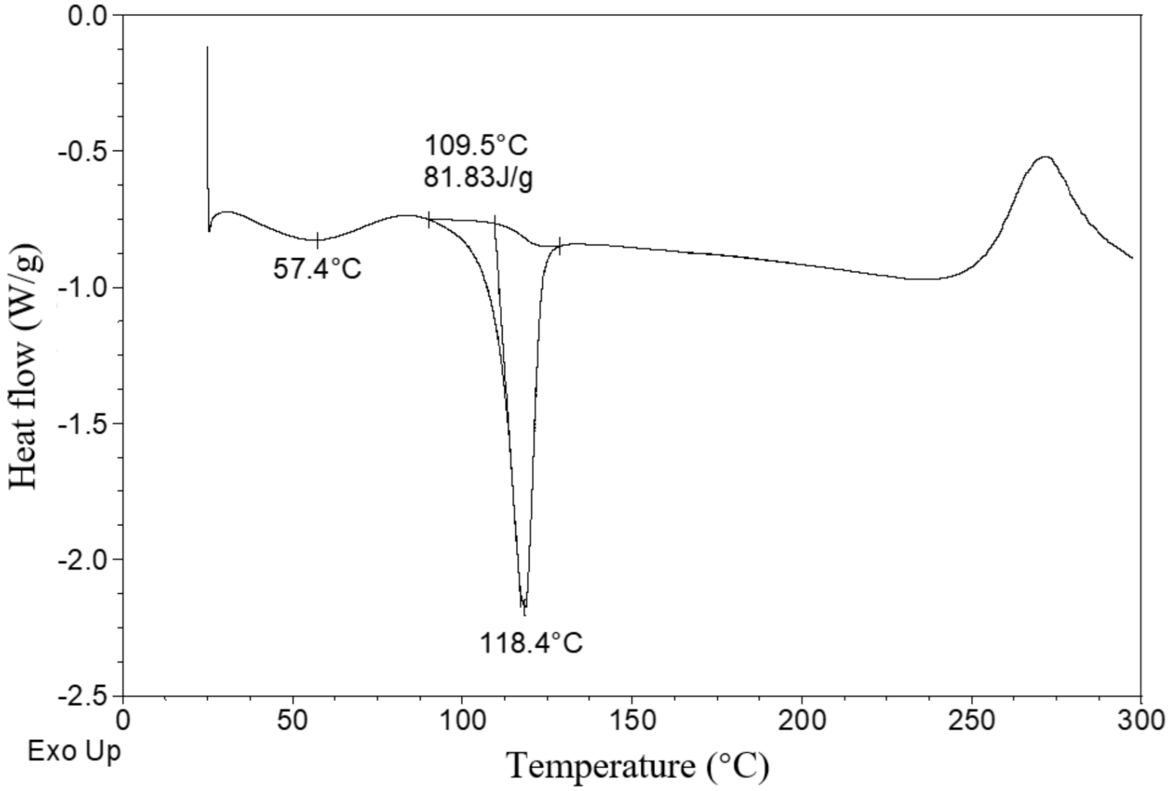
0.0
-0.5
-1.0
-1.5
-2.0
-2.5
Heat flow (W/g)
109.5°C
81.83J/g
57.4°C
118.4°C
0
50
100
150
200
250
300
Exo Up
Temperature (°C)

FIG. 43

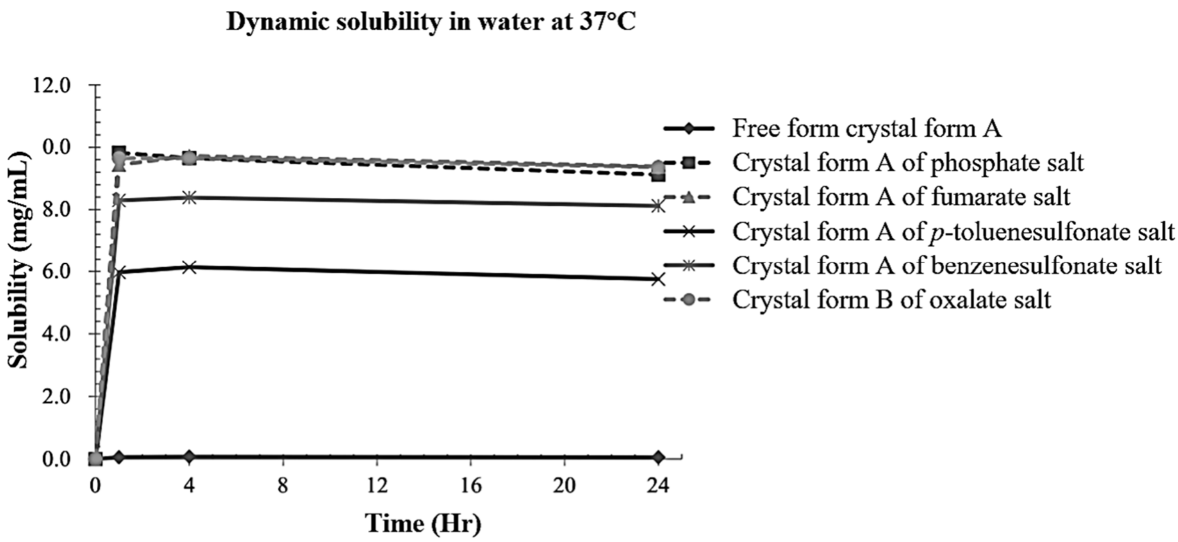
Dynamic solubility in water at 37°C
12.0
0.0
8.0
6.0
4.0
2.0
0.0
Solubility (mg/mL)
0
4
8
12
16
20
24
Time (Hr)
Free form crystal form A
Crystal form A of phosphate salt
Crystal form A of fumarate salt
Crystal form A of p-toluenesulfonate salt
Crystal form A of benzenesulfonate salt
Crystal form B of oxalate salt

FIG. 44

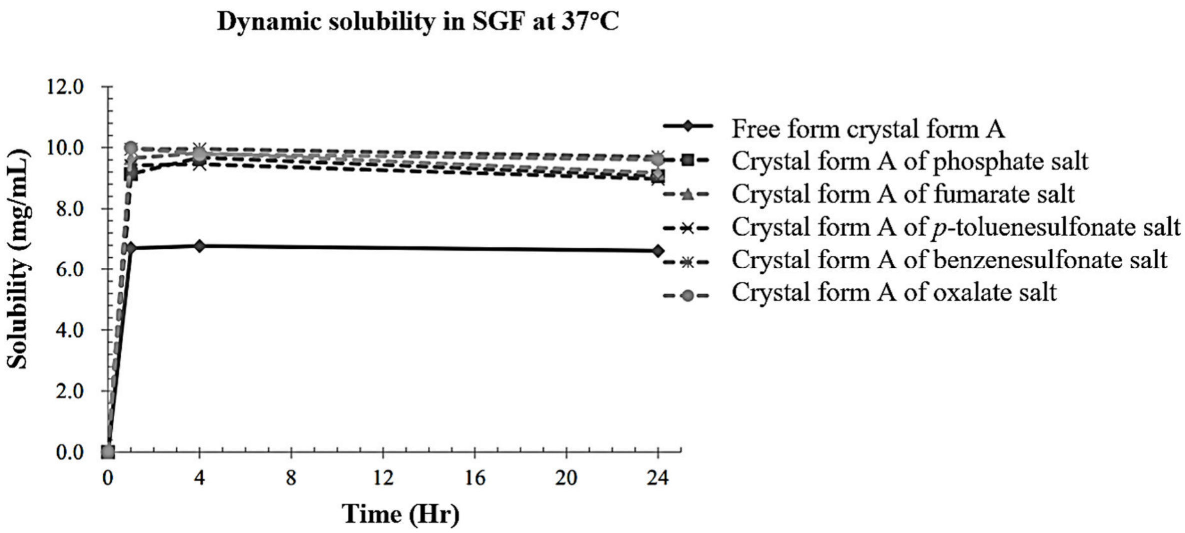
Dynamic solubility in SGF at 37°C
Solubility (mg/mL)
12.0
10.0
8.0
6.0
4.0
2.0
0.0
0
4
8
12
16
20
24
Time (Hr)
Free form crystal form A
Crystal form A of phosphate salt
Crystal form A of fumarate salt
Crystal form A of p-toluenesulfonate salt
Crystal form A of benzenesulfonate salt
Crystal form A of oxalate salt

FIG. 45

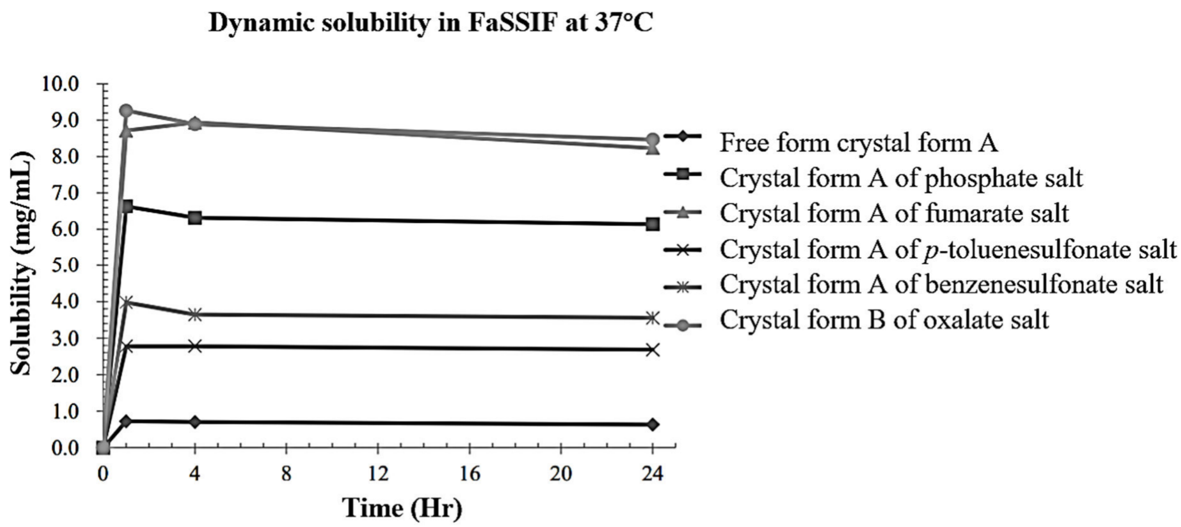
Dynamic solubility in FaSSIF at 37°C
Solubility (mg/mL)
10.0
9.0
8.0
7.0
6.0
5.0
4.0
3.0
2.0
1.0
0.0
0
4
8
12
16
20
24
Time (Hr)
Free form crystal form A
Crystal form A of phosphate salt
Crystal form A of fumarate salt
Crystal form A of p-toluenesulfonate salt
Crystal form A of benzenesulfonate salt
Crystal form B of oxalate salt

FIG. 46

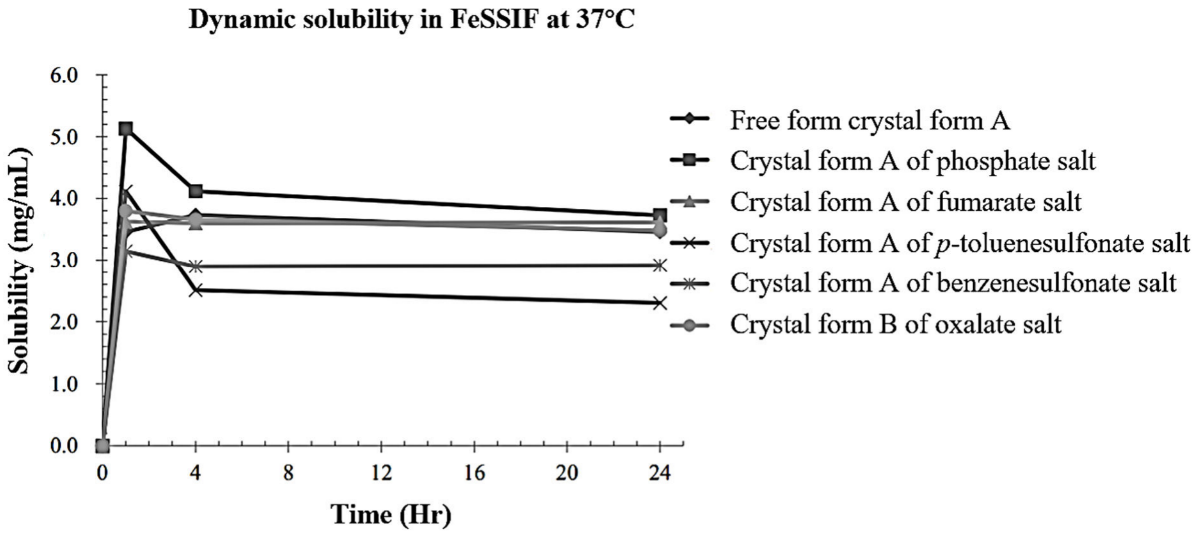
Dynamic solubility in FeSSIF at 37°C
Solubility (mg/mL)
6.0
5.0
4.0
3.0
2.0
1.0
0.0
0
4
8
12
16
20
24
Time (Hr)
Free form crystal form A
Crystal form A of phosphate salt
Crystal form A of fumarate salt
Crystal form A of p-toluenesulfonate salt
Crystal form A of benzenesulfonate salt
Crystal form B of oxalate salt

FIG. 47

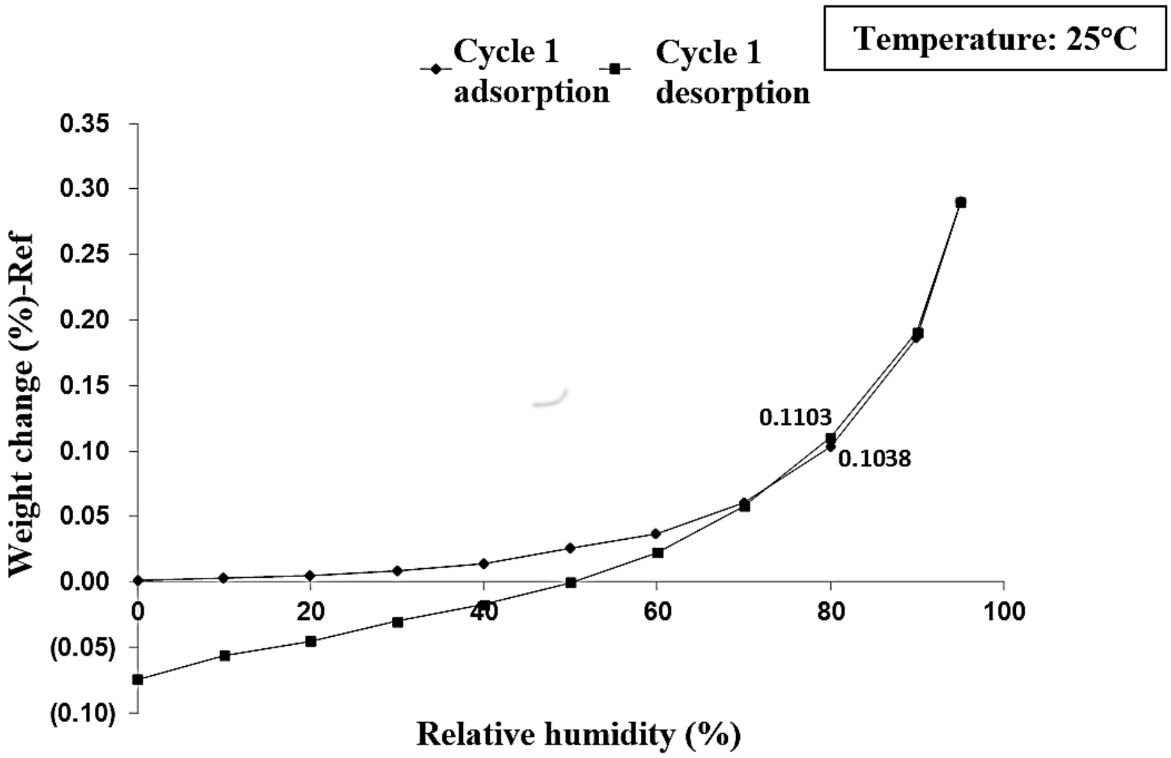
Cycle 1 adsorption
Cycle 1 desorption
Temperature: 25°C
Weight change (%)-Ref
0.35
0.30
0.25
0.20
0.15
0.10
0.05
0.00
(0.05)
(0.10)
0
20
40
60
80
100
0.1103
0.1038
Relative humidity (%)

FIG. 48

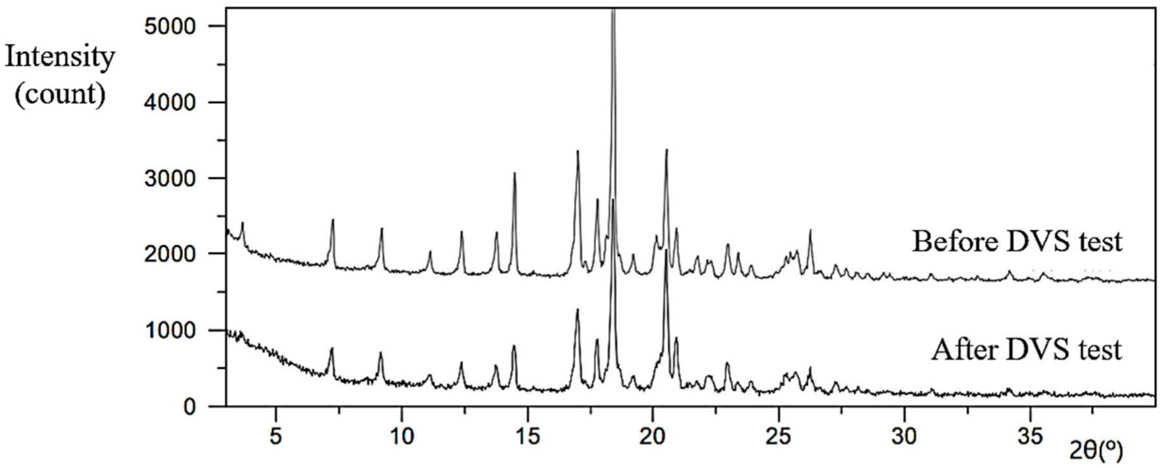
Intensity
(count)
5000
4000
3000
2000
1000
0
5
10
15
20
25
30
35
2θ(°)
Before DVS test
After DVS test

FIG. 49

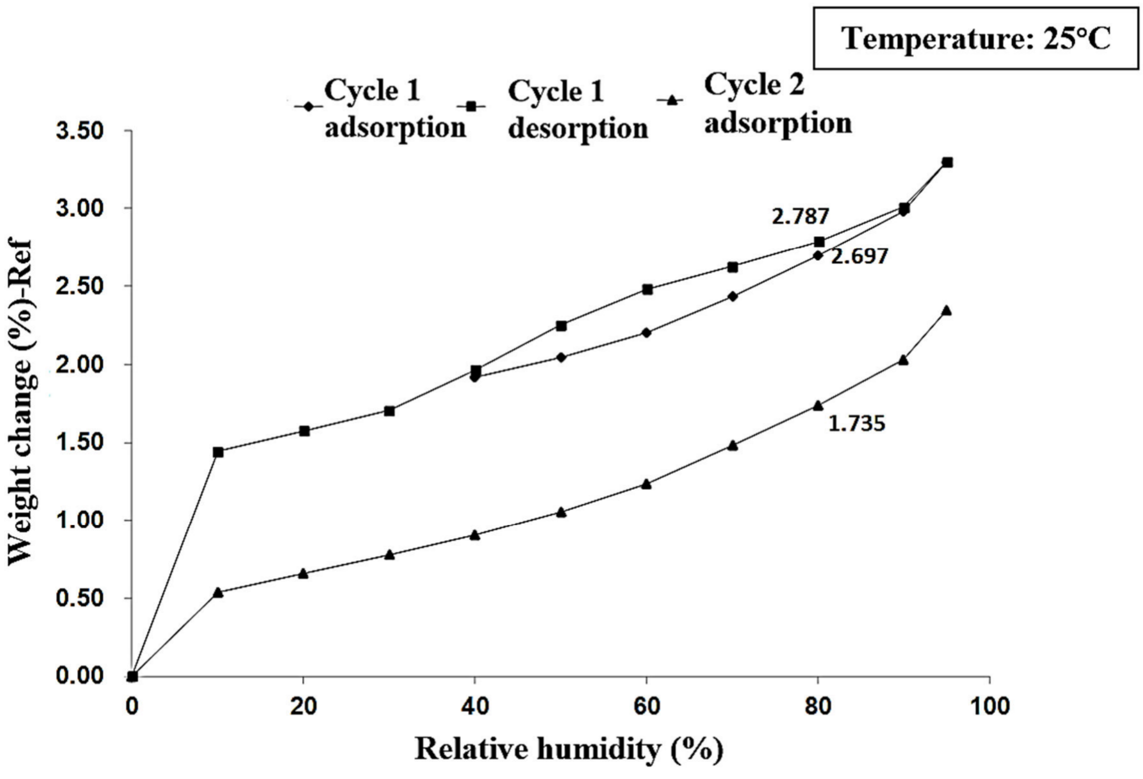
Temperature: 25°C
Cycle 1 adsorption
Cycle 1 desorption
Cycle 2 adsorption
Weight change (%)-Ref
3.50
3.00
2.50
2.00
1.50
1.00
0.50
0.00
2.787
2.697
1.735
0
20
40
60
80
100
Relative humidity (%)

FIG. 50

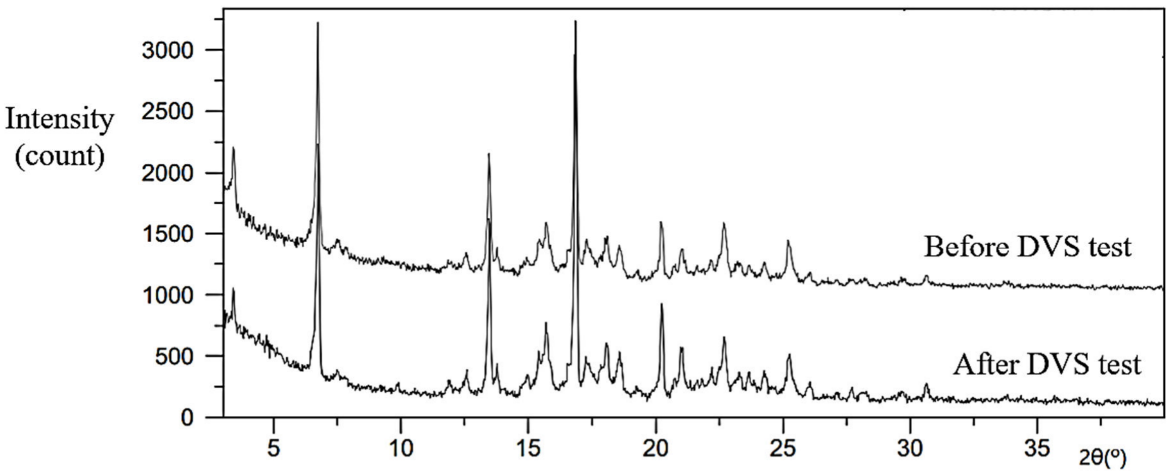
Intensity
(count)
3000
2500
2000
1500
1000
500
0
5
10
15
20
25
30
35
2θ(°)
Before DVS test
After DVS test

FIG. 51

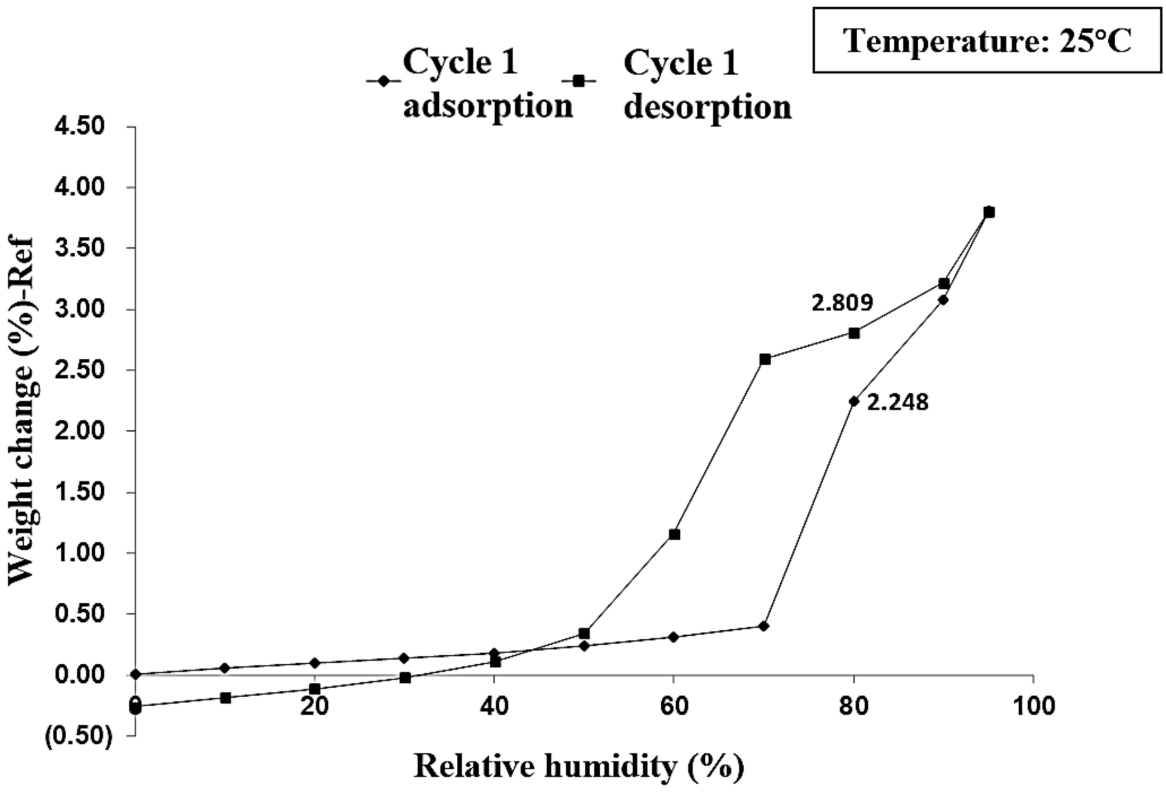
Temperature: 25°C
Cycle 1 adsorption
Cycle 1 desorption
Weight change (%)-Ref
4.50
4.00
3.50
3.00
2.50
2.00
1.50
1.00
0.50
0.00
(0.50)
0
20
40
60
80
100
2.809
2.248
Relative humidity (%)

FIG. 52

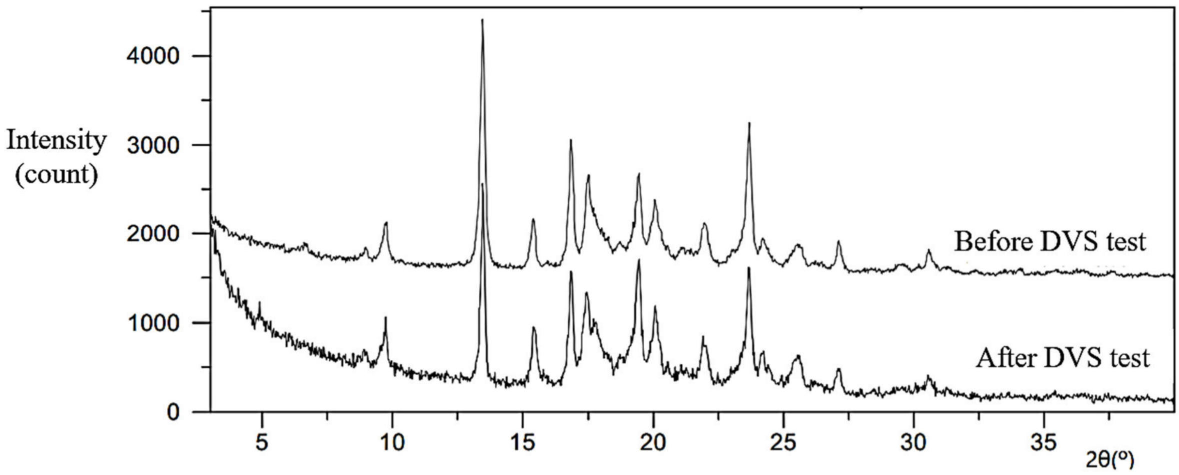
Intensity (count)
4000
3000
2000
1000
0
5
10
15
20
25
30
35
2θ(°)
Before DVS test
After DVS test

FIG. 53

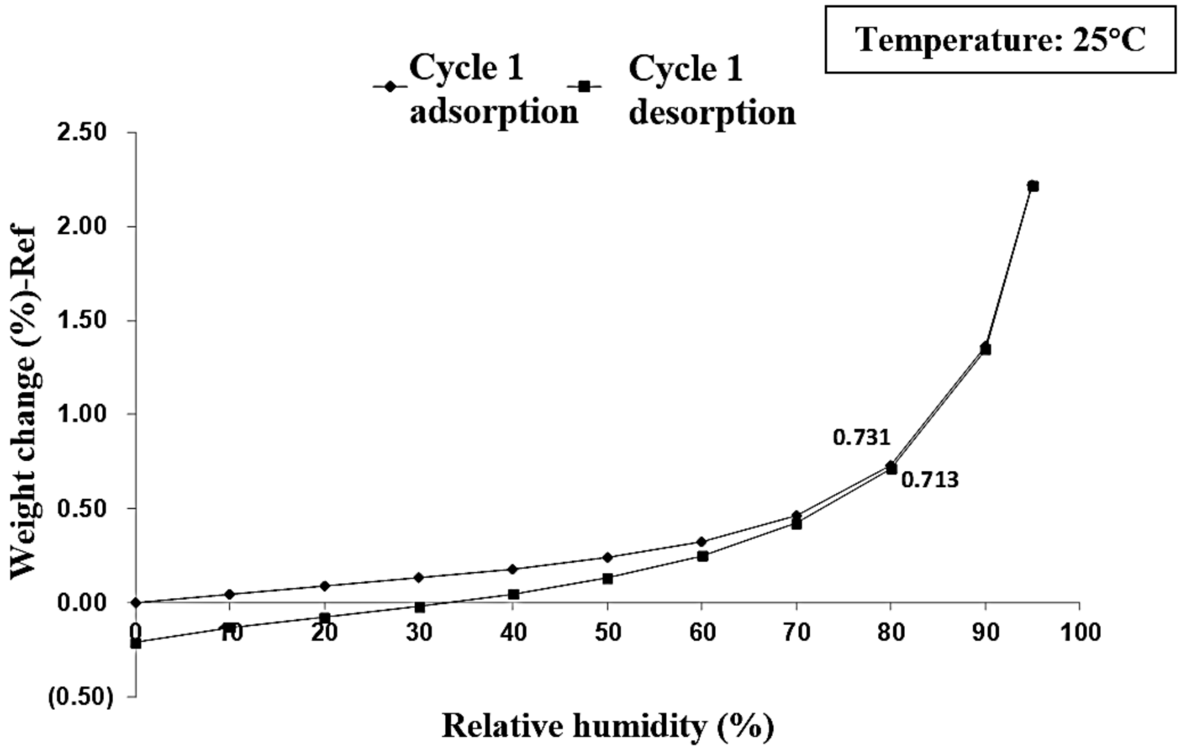
Temperature: 25°C
Cycle 1 adsorption
Cycle 1 desorption
Weight change (%)-Ref
2.50
2.00
1.50
1.00
0.50
0.00
(0.50)
0
10
20
30
40
50
60
70
80
90
100
0.731
0.713
Relative humidity (%)

FIG. 54

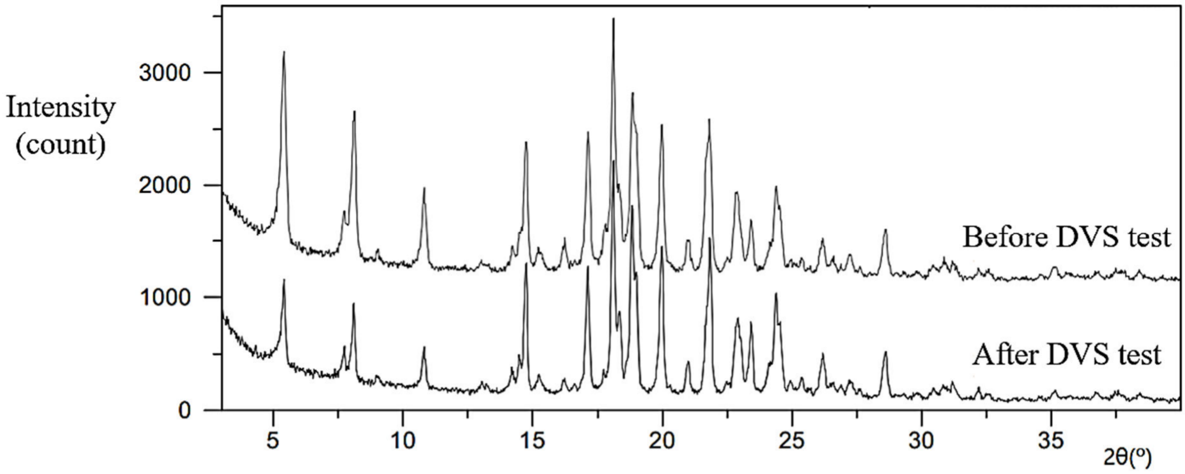
Intensity (count)
3000
2000
1000
0
5
10
15
20
25
30
35
2θ(°)
Before DVS test
After DVS test

FIG. 55

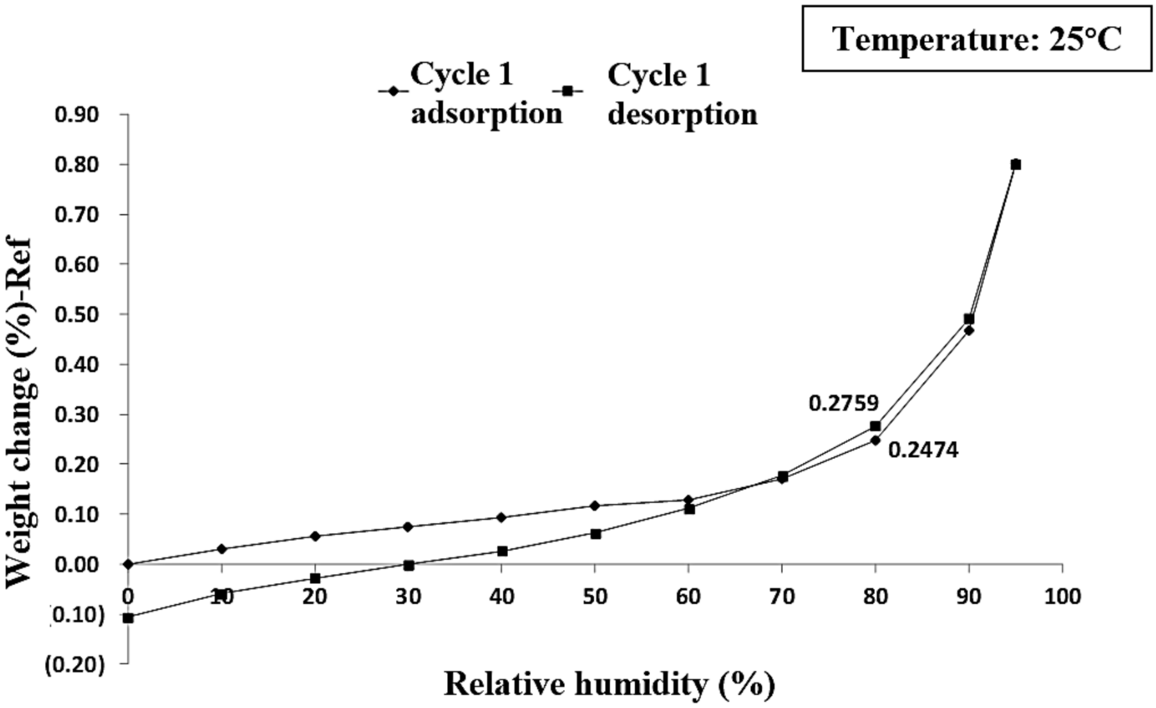
Temperature: 25°C
Cycle 1 adsorption
Cycle 1 desorption
0.90
0.80
0.70
0.60
0.50
0.40
0.30
0.20
0.10
0.00
(0.10)
(0.20)
Weight change (%)-Ref
0
10
20
30
40
50
60
70
80
90
100
0.2759
0.2474
Relative humidity (%)

FIG. 56

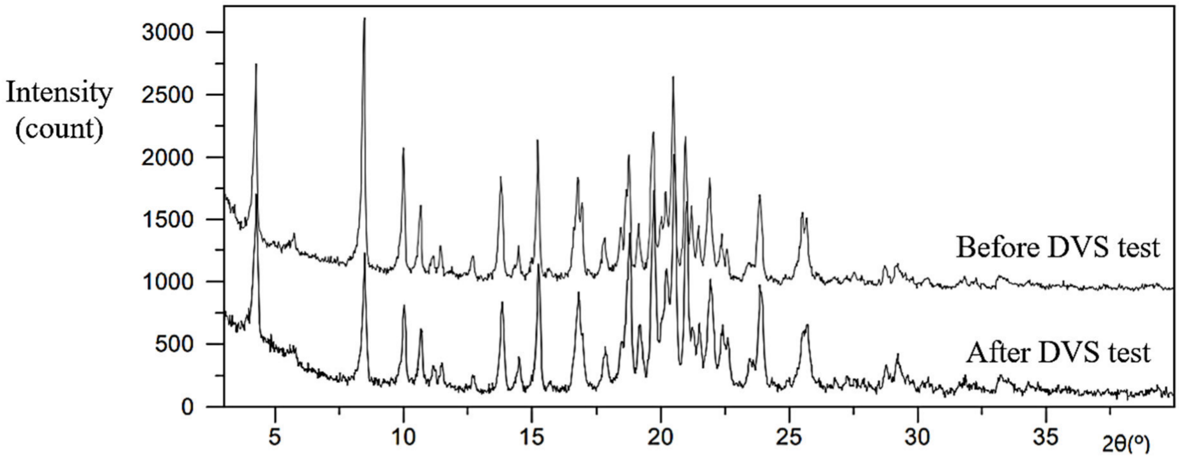
Intensity
(count)
3000
2500
2000
1500
1000
500
0
5
10
15
20
25
30
35
2θ(°)
Before DVS test
After DVS test

FIG. 57

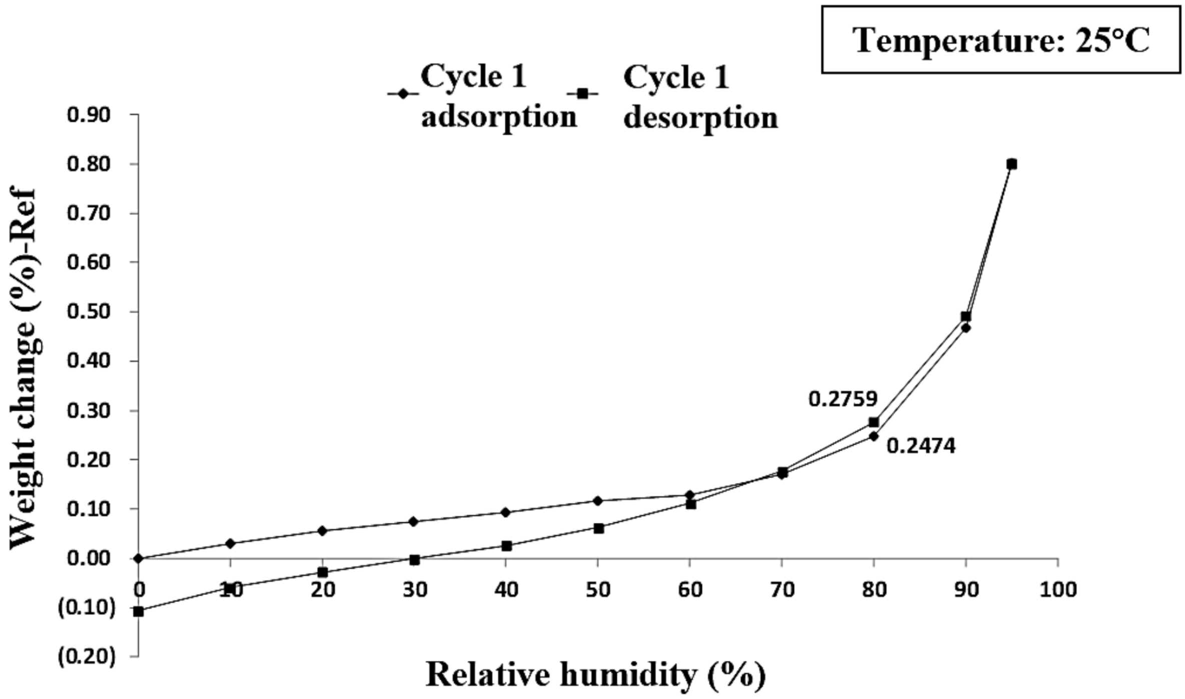
Temperature: 25°C
Cycle 1 adsorption
Cycle 1 desorption
Weight change (%)-Ref
0.90
0.80
0.70
0.60
0.50
0.40
0.30
0.20
0.10
0.00
(0.10)
(0.20)
0
10
20
30
40
50
60
70
80
90
100
0.2759
0.2474
Relative humidity (%)

FIG. 58

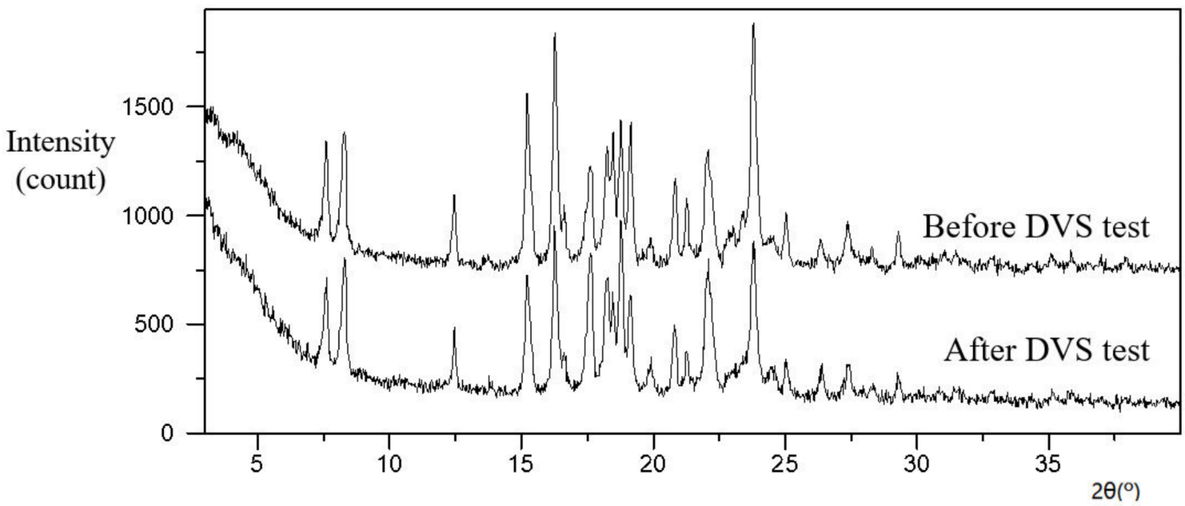


FIG. 59

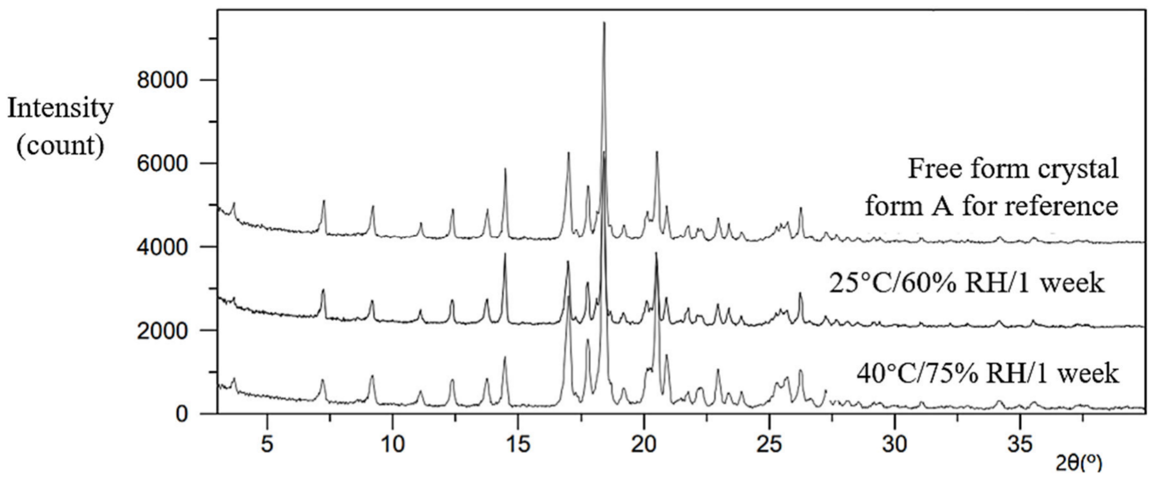


FIG. 60

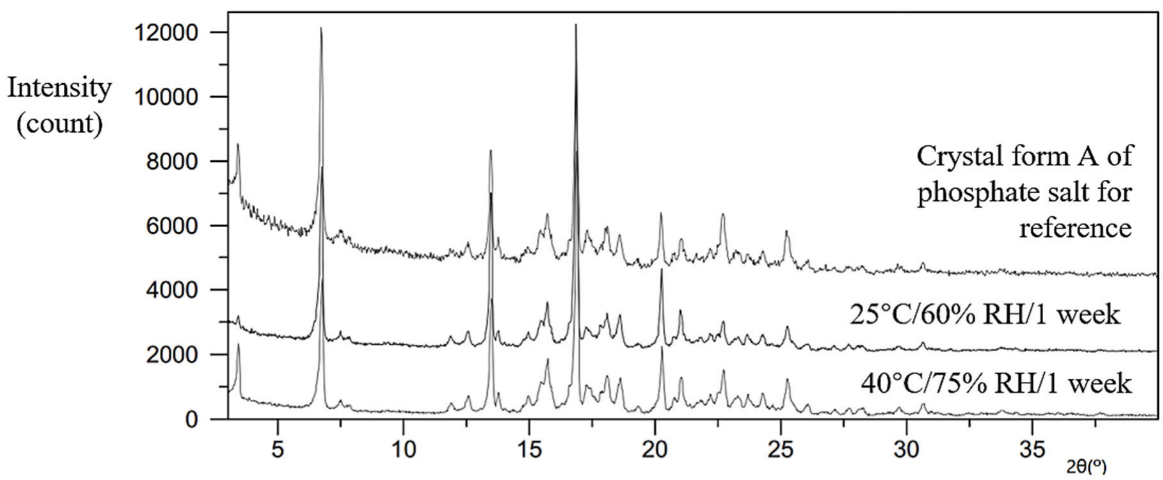


FIG. 61

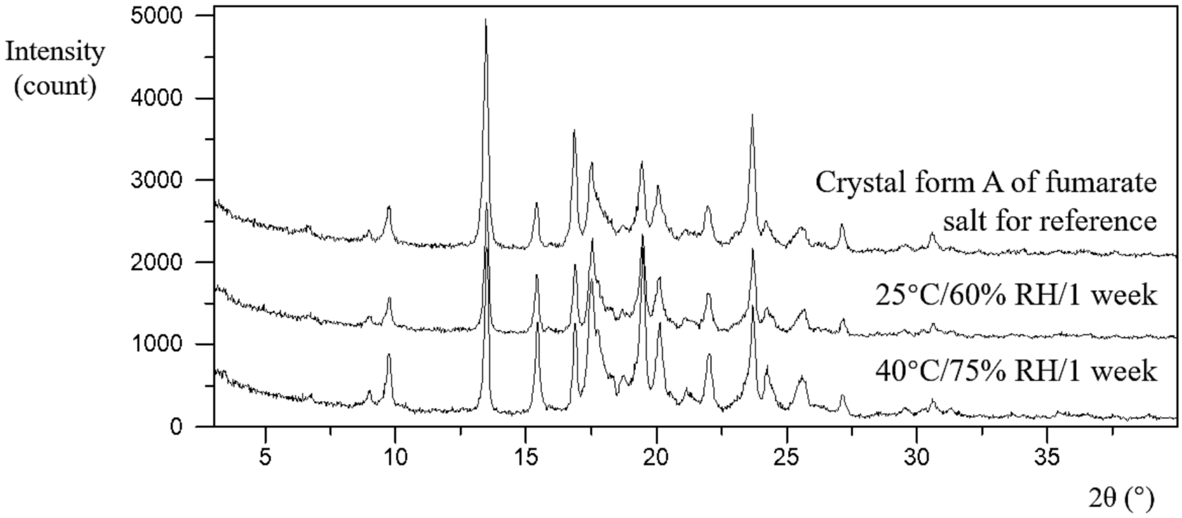


FIG. 62

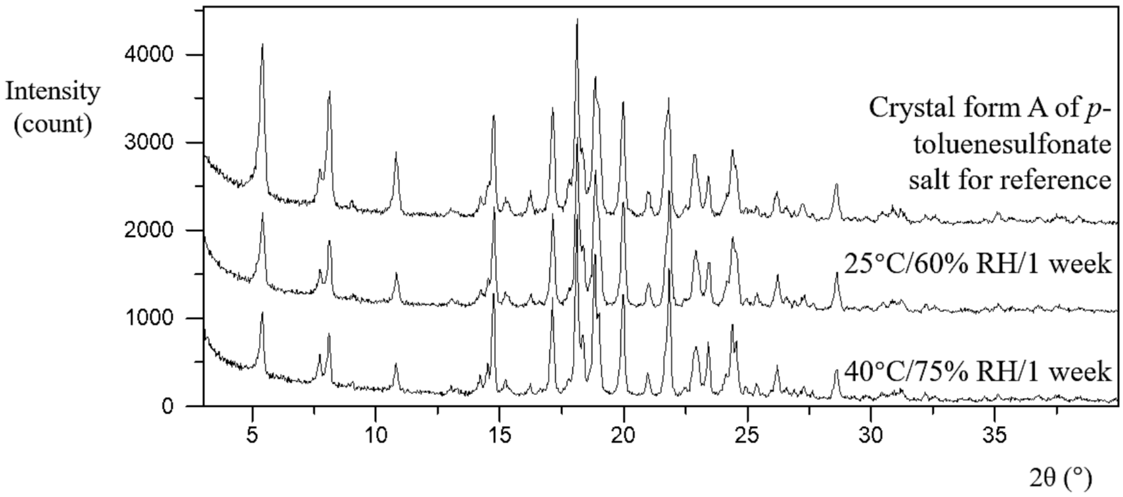


FIG. 63

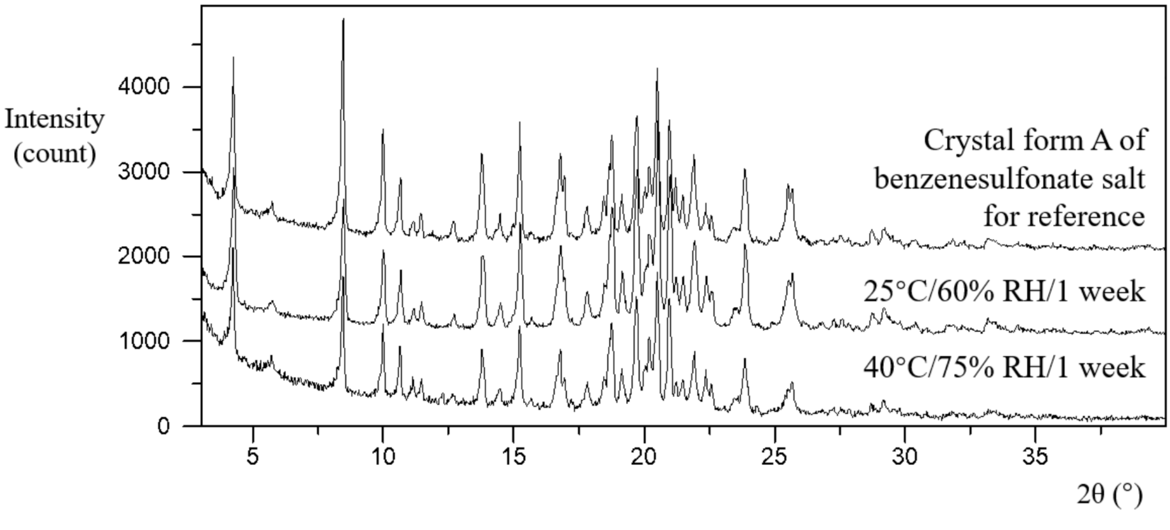


FIG. 64

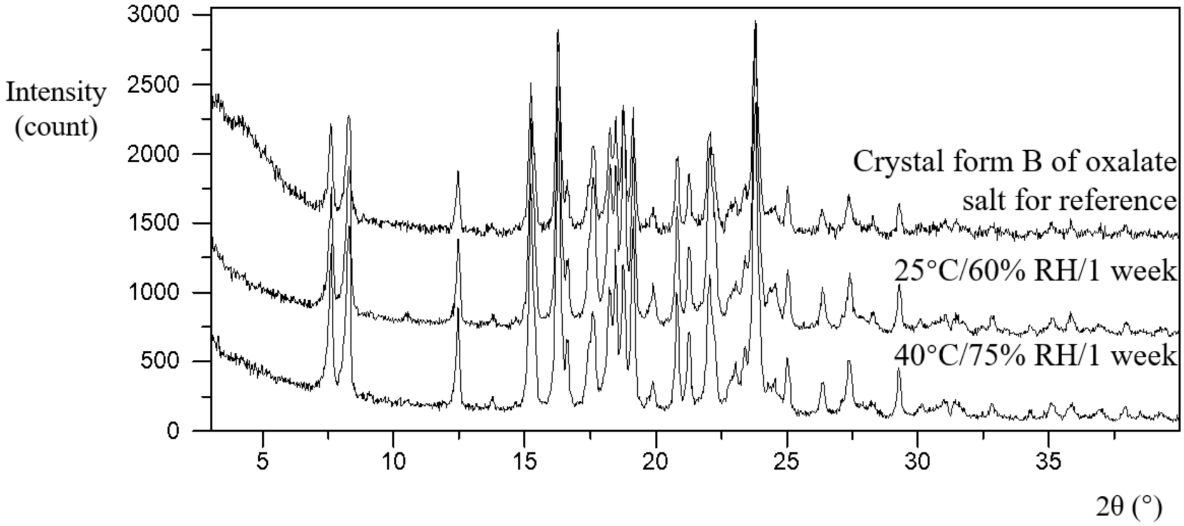

Intensity (count)
3000
2500
2000
1500
1000
500
0
5
10
15
20
25
30
35
2θ (°)
Crystal form B of oxalate salt for reference
25°C/60% RH/1 week
40°C/75% RH/1 week


FIG. 65

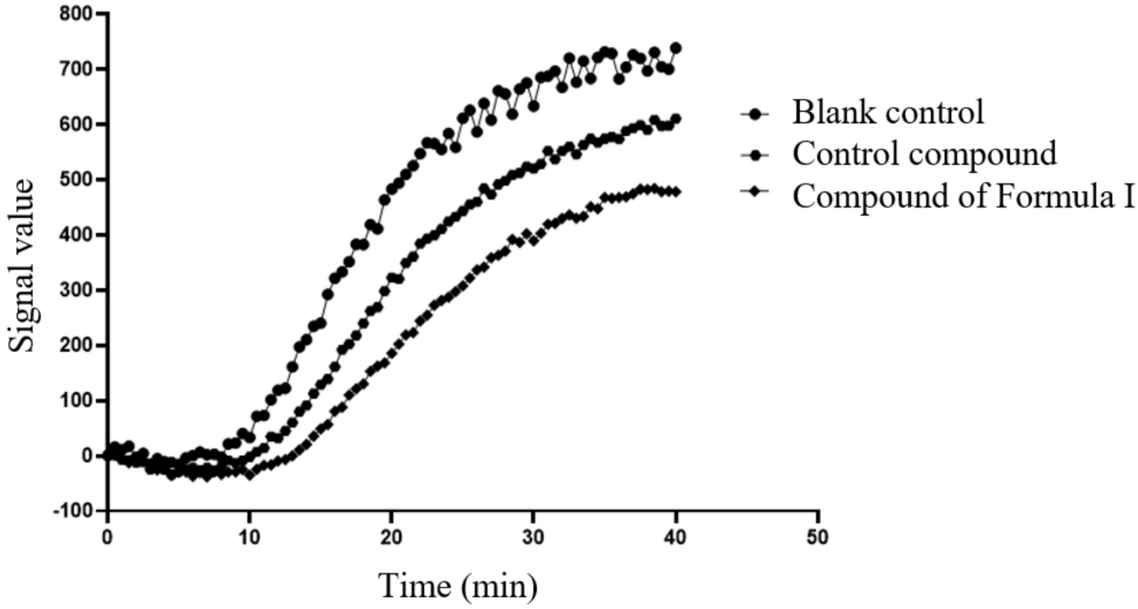

Signal value
800
700
600
500
400
300
200
100
0
-100
0
10
20
30
40
50
Time (min)
Blank control
Control compound
Compound of Formula I


FIG. 66

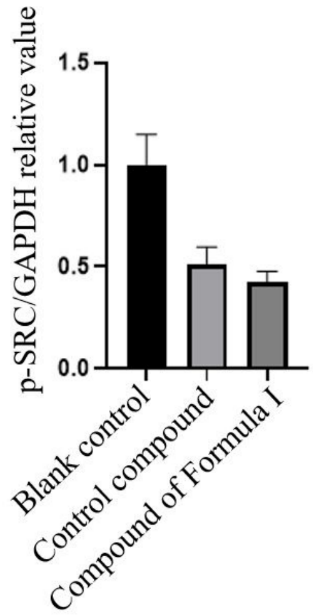
p-SRC/GAPDH relative value
1.5
1.0
0.5
0.0
Blank control
Control compound
Compound of Formula I

FIG. 67

## INTERNATIONAL SEARCH REPORT

International application No.
**PCT/CN2024/105220**

**A. CLASSIFICATION OF SUBJECT MATTER**

C07D401/12(2006.01)i; C07D413/12(2006.01)i; A61K31/4439(2006.01)i; A61K31/5377(2006.01)i; A61K31/137(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT; VEN; WOTXT; EPTXT; USTXT; VEN; STN: 武汉人福创新药物研发中心有限公司, 张学军, 臧杨, 杨辉, 魏文军, 宋泽金, 张辛, 李莉娥, 杨俊, 吡啶, 乙酰胺, 吡咯, 微管蛋白, 抑制剂, 激酶, 肿瘤, 皮肤病, 晶体, 结构式, structural formula, pyridine, pyrroleacetamide, inhibitor, cancer, tumour, tumor, tubulin, src, crystal, skin diseases

**C. DOCUMENTS CONSIDERED TO BE RELEVANT**

| Category* | Citation of document, with indication, where appropriate, of the relevant passages | Relevant to claim No. |
|---|---|---|
| PX | CN 116444427 A (WUHAN HUMANWELL INNOVATION DRUG RESEARCH AND DEVELOPMENT CENTER CO., LTD. et al.) 18 July 2023 (2023-07-18) claims 28 and 34-38 | 1-7, 8 (in part), 9, 10 |
| PX | CN 116444426 A (WUHAN HUMANWELL INNOVATION DRUG RESEARCH AND DEVELOPMENT CENTER CO., LTD. et al.) 18 July 2023 (2023-07-18) claims 1-18 and 20-25 | 1-7, 8 (in part), 9, 10 |
| X | CN 113461665 A (CHENGDU ZENITAR BIOMEDICAL TECHNOLOGY CO., LTD.) 01 October 2021 (2021-10-01) claims 1, 11, 15 and 16 | 1-7, 8 (in part), 9, 10 |
| A | CN 102905700 A (KINEX PHARMACEUTICALS, LLC.) 30 January 2013 (2013-01-30) entire document | 1-7, 8 (in part), 9, 10 |
| A | CN 112805007 A (ATHENEX HK INNOVATIVE LTD.) 14 May 2021 (2021-05-14) entire document | 1-7, 8 (in part), 9, 10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 September 2024** | **23 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/105220**

**C. DOCUMENTS CONSIDERED TO BE RELEVANT**

| Category* | Citation of document, with indication, where appropriate, of the relevant passages | Relevant to claim No. |
|---|---|---|
| A | CN 115477608 A (CHENGDU BEINUO KECHENG BIOLOGICAL TECHNOLOGY CO., LTD.) 16 December 2022 (2022-12-16)<br>entire document | 1-7, 8 (in part), 9, 10 |
| A | WANG, Lun et al. "Design, Synthesis, and Bioactivity Evaluation of Dual-Target Inhibitors of Tubulin and Src Kinase Guided by Crystal Structure"<br>*J. Med. Chem.*, Vol. vol. 64, 03 June 2021 (2021-06-03), 8127-8141<br>ISSN: 1520-4804,<br>page 8129 | 1-7, 8 (in part), 9, 10 |

**INTERNATIONAL SEARCH REPORT**

International application No.
**PCT/CN2024/105220**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8 (in part)**
because they relate to subject matter not required to be searched by this Authority, namely:

Claim 8 sets forth the use of a substance M, wherein the substance M is a crystal form and a salt form of the compound as shown in formula I of any one of claims 1-6 or the pharmaceutical composition of claim 7, and the use comprises: 1) inhibiting tubulin polymerization and/or Src kinase; and 2) preventing and/or treating diseases related to tubulin polymerization and/or Src kinase, which falls within the scope of methods for treatment of a human body, that is, falls within the cases set out in PCT Rule 39.1(iv) for which no international search is required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/105220**

| Patent document cited in search report | Publication date (day/month/year) | Patent family member(s) | Publication date (day/month/year) |
|---|---|---|---|
| CN 116444427 A | 18 July 2023 | TW 202329956 A | 01 August 2023 |
| | | WO 2023134752 A1 | 20 July 2023 |
| CN 116444426 A | 18 July 2023 | WO 2023134751 A1 | 20 July 2023 |
| CN 113461665 A | 01 October 2021 | CN 113461665 B | 19 May 2023 |
| CN 102905700 A | 30 January 2013 | EP 2558085 A2 | 20 February 2013 |
| | | EP 2558085 A4 | 09 October 2013 |
| | | EP 2558085 B1 | 30 August 2017 |
| | | US 2011281872 A1 | 17 November 2011 |
| | | US 8748423 B2 | 10 June 2014 |
| | | ES 2645367 T3 | 05 December 2017 |
| | | CA 2796419 A1 | 20 October 2011 |
| | | CA 2796419 C | 06 November 2018 |
| | | WO 2011129936 A2 | 20 October 2011 |
| | | WO 2011129936 A3 | 19 January 2012 |
| | | WO 2011129936 A9 | 08 March 2012 |
| | | WO 2011129936 A4 | 19 April 2012 |
| | | HK 1181312 A0 | 08 November 2013 |
| | | CN 107441097 A | 08 December 2017 |
| | | HK 1181312 A1 | 11 May 2018 |
| CN 112805007 A | 14 May 2021 | TW 202017572 A | 16 May 2020 |
| | | US 2021038608 A1 | 11 February 2021 |
| | | US 11752156 B2 | 12 September 2023 |
| | | CA 3092939 A1 | 12 September 2019 |
| | | WO 2019173533 A1 | 12 September 2019 |
| | | EP 3761988 A1 | 13 January 2021 |
| | | AU 2019231706 A1 | 24 September 2020 |
| | | CN 112805007 B | 15 December 2023 |
| | | CN 117599189 A | 27 February 2024 |
| CN 115477608 A | 16 December 2022 | CN 115477608 B | 12 December 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023108740075
- CN 202211139978
- WO 2008002676 A2